(19) Europäisches Patentamt
European Patent Office
Office européen des brevets

(11) EP 3 960 872 A1

(12) EUROPEAN PATENT APPLICATION

(43) Date of publication:
**02.03.2022 Bulletin 2022/09**

(21) Application number: **20193227.4**

(22) Date of filing: **27.08.2020**

(51) International Patent Classification (IPC):
***C12Q 1/6876*** *(2018.01)* ***G16B 40/00*** *(2019.01)*

(52) Cooperative Patent Classification (CPC):
**C12Q 1/6876; G16B 25/10; G16B 40/20;**
C12Q 2600/124; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Moreira Borralho Relógio, Angela**
**10439 Berlin (DE)**

(72) Inventors:
- **Moreira Borralho Relógio, Angela**
  **10439 Berlin (DE)**
- **Hesse, Janina**
  **13629 Berlin (DE)**
- **Akhondzadeh Basti, Alireza**
  **14167 Berlin (DE)**

(74) Representative: **Kilger, Ute**
**Boehmert & Boehmert**
**Anwaltspartnerschaft mbB**
**Pettenkoferstrasse 22**
**80336 München (DE)**

# (54) METHOD OF ASSESSING THE CIRCADIAN RHYTHM OF A SUBJECT AND/OR ASSESSING AND PREDICTING THE ATHLETIC PERFORMANCE OF SAID SUBJECT

(57) Subject matter of the present invention is a method of assessing the circadian rhythm of a subject and/or assessing and predicting the athletic performance of said subject, wherein said method comprises the steps of providing at least three samples of saliva, more preferably four samples of saliva, from said subject, wherein said samples have been taken at different time points over the day and determining gene expression of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2,, in particular of BMAL1 and PER2, in each of said samples. The method further comprises the step of assessing and predicting by means of a computational step based on said expression levels of BMAL1 and PER2 over the day the circadian rhythm of said subject and/or the individual diurnal athletic performance times.

EP 3 960 872 A1

SEQUENCE LISTING

<110> Moreira Borralho Relógio Angela

<120> Method of assessing the circadian rhythm of a subject and/or assessing and predicting the athletic performance of said subject

<130> C75977EP

<160> 20

<170> PatentIn version 3.5

<210> 1
<211> 1878
<212> DNA
<213> Human

<400> 1
```
atggcagacc agagaatgga catttcttca accatcagtg atttcatgtc cccgggcccc      60

accgacctgc tttccagctc tcttggtacc agtggtgtgg attgcaaccg caaacggaaa     120

ggcagctcca ctgactacca agaaagcatg gacacagaca aagatgaccc tcatggaagg     180

ttagaatata cagaacacca aggaaggata aaaaatgcaa gggaagctca cagtcagatt     240

gaaaagcggc gtcgggataa aatgaacagt tttatagatg aattggcttc tttggtacca     300

acatgcaacg caatgtccag gaaattagat aaacttactg tgctaaggat ggctgttcag     360

cacatgaaaa cattaagagg tgccaccaat ccatacacag aagcaaacta caaaccaact     420

tttctatcag acgatgaatt gaaacacctc attctcaggg cagcagatgg atttttgttt     480

gtcgtaggat gtgaccgagg gaagatactc tttgtctcag agtctgtctt caagatcctc     540

aactacagcc agaatgatct gattggtcag agtttgtttg actacctgca tcctaaagat     600

attgccaaag tcaaggagca gctctcctcc tctgacaccg caccccggga gcggctcata     660

gatgcaaaaa ctggacttcc agttaaaaca gatataaccc ctgggccatc tcgattatgt     720

tctggagcac gacgttcttt cttctgtagg atgaagtgta acaggccttc agtaaaggtt     780

gaagacaagg acttcccctc tacctgctca aagaaaaaag atcgaaaaag cttctgcaca     840

atccacagca caggctattt gaaaagctgg ccacccacaa agatggggct ggatgaagac     900

aacgaaccag acaatgaggg gtgtaacctc agctgcctcg tcgcaattgg acgactgcat     960

tctcatgtag ttccacaacc agtgaacggg gaaatcaggg tgaaatctat ggaatatgtt    1020

tctcggcacg cgatagatgg aaagtttgtt tttgtagacc agagggcaac agctattttg    1080

gcatatttac cacaagaact tctaggcaca tcgtgttatg aatattttca ccaagatgac    1140

ataggacatc ttgcagaatg tcataggcaa gttttacaga cgagagaaaa aattacaact    1200

aattgctata aatttaaaat caaagatggt tcttttatca cactacggag tcgatggttc    1260

agtttcatga acccttggac caaggaagta gaatatattg tctcaactaa cactgttgtt    1320
```

ttagccaacg tcctggaagg cggggaccca accttcccac agctcacagc atcccccac 1380

agcatggaca gcatgctgcc ctctggagaa ggtggcccaa agaggaccca ccccactgtt 1440

ccagggattc cagggggaac ccgggctggg gcaggaaaaa taggccgaat gattgctgag 1500

gaaatcatgg aaatccacag gataagaggg tcatcgcctt ctagctgtgg ctccagccca 1560

ttgaacatca cgagtacgcc tccccctgat gcctcttctc caggaggcaa gaagatttta 1620

aatggaggga ctccagacat tccttccagt ggcctactat caggccaggc tcaggagaac 1680

ccaggttatc catattctga tagttcttct attcttggtg agaaccccca cataggtata 1740

gacatgattg acaacgacca aggatcaagt agtcccagta atgatgaggc agcaatggct 1800

gtcatcatga gcctcttgga agcagatgct ggactgggtg gccctgttga ctttagtgac 1860

ttgccatggc cgctgtaa 1878

<210> 2
<211> 1911
<212> DNA
<213> Human

<400> 2
atggcggcgg aagaggaggc tgcggcggga ggtaaagtgt tgagagagga gaaccagtgc 60

attgctcctg tggtttccag ccgcgtgagt ccagggacaa gaccaacagc tatggggtct 120

ttcagctcac acatgacaga gtttccacga aaacgcaaag gaagtgattc agacccatcc 180

cagtcaggaa tcatgacaga aaaagtggtg gaaaagcttt ctcagaatcc ccttacctat 240

cttctttcaa caaggataga aatatcagcc tccagtggca gcagagtgga agatggtgaa 300

caccaagtta aaatgaaggc cttcagagaa gctcatagcc aaactgaaaa gcggaggaga 360

gataaaatga ataacctgat tgaagaactg tctgcaatga tccctcagtg caacccccatg 420

gcgcgtaaac tggacaaact tacagtttta agaatggctg ttcaacactt gagatcttta 480

aaaggcttga caaattctta tgtgggaagt aattatagac catcatttct tcaggataat 540

gagctcagac atttaatcct taagactgca gaaggcttct tatttgtggt tggatgtgaa 600

agaggaaaaa ttctcttcgt ttctaagtca gtctccaaaa tacttaatta tgatcaggct 660

agtttgactg gacaaagctt atttgacttc ttacatccaa aagatgttgc caaagtaaag 720

gaacaacttt cttcttttga tatttcacca agagaaaagc taatagatgc caaaactggt 780

ttgcaagttc acagtaatct ccacgctgga aggacacgtg tgtattctgg ctcaagacga 840

tctttttct gtcggataaa gagttgtaaa atctctgtca aagaagagca tggatgctta 900

cccaactcaa agaagaaaga gcacagaaaa ttctatacta tccattgcac tggttacttg 960

agaagctggc ctccaaatat tgttggaatg gaagaagaaa ggaacagtaa gaaagacaac 1020

agtaatttta cctgccttgt ggccattgga agattacagc catatattgt tccacagaac 1080

agtggagaga ttaatgtgaa accaactgaa tttataaccc ggtttgcagt gaatggaaaa 1140

tttgtctatg tagatcaaag ggcaacagcg attttaggat atctgcctca ggaacttttg 1200

ggaacttctt gttatgaata ttttcatcaa gatgaccaca ataatttgac tgacaagcac 1260

aaagcagttc tacagagtaa ggagaaaata cttacagatt cctacaaatt cagagcaaaa 1320

gatggctctt ttgtaacttt aaaaagccaa tggtttagtt tcacaaatcc ttggacaaaa 1380

gaactggaat atattgtatc tgtcaacact ttagttttgg gacatagtga gcctggagaa 1440

gcatcatttt taccttgtag ctctcaatca tcagaagaat cctctagaca gtcctgtatg 1500

agtgtacctg gaatgtctac tggaacagta cttggtgctg gtagtattgg aacagatatt 1560

gcaaatgaaa ttctggattt acagaggtta cagtcttctt catacettga tgattcgagt 1620

ccaacaggtt taatgaaaga tactcatact gtaaactgca ggagtatgtc aaataaggag 1680

ttgtttccac caagtccttc tgaaatgggg gagctagagg ctaccaggca aaaccagagt 1740

actgttgctg tccacagcca tgagccactc ctcagtgatg gtgcacagtt ggatttcgat 1800

gccctatgtg acaatgatga cacagccatg gctgcattta tgaattactt agaagcagag 1860

gggggcctgg gagaccctgg ggacttcagt gacatccagt ggaccctcta g 1911

<210> 3
<211> 3873
<212> DNA
<213> Human

<400> 3
atgagtggcc ccctagaagg ggctgatggg ggaggggacc ccaggcctgg ggaatcattt 60

tgtcctgggg gcgtcccatc ccctgggccc ccacagcacc ggccttgccc aggccccagc 120

ctggccgatg acaccgatgc caacagcaat ggttcaagtg gcaatgagtc caacgggcat 180

gagtctagag gcgcatctca gcggagctca cacagctcct cctcaggcaa cggcaaggac 240

tcagccctgc tggagaccac tgagagcagc aagagcacaa actctcagag cccatcccca 300

cccagcagtt ccattgccta cagcctcctg agtgccagct cagagcagga caacccgtcc 360

accagtggct gcagcagtga acagtcagcc cgggcaagga ctcagaagga actcatgaca 420

gcacttcgag agctcaagct tcgactgccg ccagagcgcc ggggcaaggg ccgctctggg 480

accctggcca cgctgcagta cgcactggcc tgtgtcaagc aggtgcaggc caaccaggaa 540

tactaccagc agtggagcct ggaggagggc gagccttgct ccatggacat gtccacctat 600

accctggagg agctggagca catcacgtct gagtacacac ttcagaacca ggataccttc 660

tcagtggctg tctccttcct gacgggccga atcgtctaca tttcggagca ggcagccgtc 720

ctgctgcgtt gcaagcggga cgtgttccgg ggtacccgct tctctgagct cctggctccc 780

caggatgtgg gagtcttcta tggttccact gctccatctc gcctgcccac ctggggcaca 840

gggcctcag caggttcagg cctcagggac tttacccagg agaagtccgt cttctgccgt 900
atcagaggag gtcctgaccg ggatccaggg cctcggtacc agccattccg cctaaccccg 960
tatgtgacca agatccgggt ctcagatggg gcccctgcac agccgtgctg cctgctgatt 1020
gcagagcgca tccattcggg ttacgaagct ccccggatac cccctgacaa gaggattttc 1080
actacgcggc acacacccag ctgcctcttc caggatgtgg atgaaagggc tgcccccctg 1140
ctgggctacc tgccccagga cctcctgggg gccccagtgc tcctgttcct gcatcctgag 1200
gaccgacccc tcatgctggc tatccacaag aagattctgc agttggcggg ccagcccttt 1260
gaccactccc ctatccgctt ctgtgcccgc aacggggagt atgtcaccat ggacaccagc 1320
tgggctggct ttgtgcaccc ctggagccgc aaggtagcct tcgtgttggg ccgccacaaa 1380
gtacgcacgg ccccccctgaa tgaggacgtg ttcactcccc cggcccccag cccagctccc 1440
tccctggaca ctgatatcca ggagctgtca gagcagatcc accggctgct gctgcagccc 1500
gtccacagcc ccagccccac gggactctgt ggagtcggcg ccgtgacatc cccaggccct 1560
ctccacagcc ctgggtcctc cagtgatagc aacgggggtg atgcagaggg gcctgggcct 1620
cctgcgccag tgactttcca gcagatctgt aaggatgtgc atctggtgaa gcaccagggc 1680
cagcagcttt ttattgagtc tcgggcccgg cctcagtccc ggccccgcct ccctgctaca 1740
ggcacgttca aggccaaggc ccttccctgc caatccccag acccagagct ggaggcgggt 1800
tctgctcccg tccaggcccc actagccttg gtccctgagg aggccgagag gaaagaagcc 1860
tccagctgct cctaccagca gatcaactgc ctggacagca tcctcaggta cctggagagc 1920
tgcaacctcc ccagcaccac taagcgtaaa tgtgcctcct cctcctccta taccacctcc 1980
tcagcctctg acgacgacag gcagaggaca ggtccagtct ctgtggggac caagaaagat 2040
ccgccgtcag cagcgctgtc tggggagggg gccaccccac ggaaggagcc agtggtggga 2100
ggcaccctga gcccgctcgc cctggccaat aaggcggaga gtgtggtgtc cgtcaccagt 2160
cagtgtagct tcagctccac catcgtccat gtgggagaca agaagccccc ggagtcggac 2220
atcatcatga tggaggacct gcctggccta gccccaggcc cagcccccag cccagccccc 2280
agccccacag tagcccctga cccagcccca gacgcctacc gtccagtggg gctgaccaag 2340
gccgtgctgt ccctgcacac acagaaggaa gagcaagcct tcctcagccg cttccgagac 2400
ctgggcaggc tgcgtggact cgacagctct tccacagctc cctcagccct tggcgagcga 2460
ggctgccacc acggccccgc acccccaagc cgccgacacc actgccgatc caaagccaag 2520
cgctcacgcc accaccagaa ccctcgggct gaagcgccct gctatgtctc acacccctca 2580
cccgtgccac cctccacccc ctggcccacc ccaccagcca ctaccccctt cccagcggtt 2640
gtccagccct accctctccc agtgttctct cctcgaggag gcccccagcc tcttcccccct 2700
gctcccacat ctgtgccccc agctgctttc cccgcccctt tggtgacccc aatggtggcc 2760

ttggtgctcc ctaactatct gttcccaacc ccatccagct atccttatgg ggcactccag 2820

acccctgctg aagggcctcc cactcctgcc tcgcactccc cttctccatc cttgcccgcc 2880

ctcgccccga gtcctcctca ccgcccggac tctccactgt tcaactcgag atgcagctct 2940

ccactccagc tcaatctgct gcagctggag gagctccccc gtgctgaggg ggctgctgtt 3000

gcaggaggcc ctgggagcag tgccgggccc ccacctccca gtgcggaggc tgctgagcca 3060

gaggccagac tggcggaggt cactgagtcc tccaatcagg acgcactttc cggctccagt 3120

gacctgctcg aacttctgct gcaagaggac tcgcgctccg gcacaggctc cgcagcctcg 3180

ggctccttgg gctctggctt gggctctggg tctggttcag gctcccatga agggggcagc 3240

acctcagcca gcatcactcg cagcagccag agcagccaca caagcaaata ctttggcagc 3300

atcgactctt ccgaggctga ggctggggct gctcggggcg ggggctgagcc tggggaccag 3360

gtgattaagt acgtgctcca ggatcccatt tggctgctca tggccaatgc tgaccagcgc 3420

gtcatgatga cctaccaggt gccctccagg gacatgacct ctgtgctgaa gcaggatcgg 3480

gagcggctcc gagccatgca gaagcagcag cctcggtttt ctgaggacca gcggcgggaa 3540

ctgggtgctg tgcactcctg ggtccggaag ggccaactgc ctcgggctct tgatgtgatg 3600

gcctgtgtgg actgtgggag cagcacccaa gatcctggtc accctgatga cccactcttc 3660

tcagagctgg atggactggg gctggagccc atggaagagg gtggaggcga gcagggcagc 3720

agcggtggcg gcagtggtga gggagagggc tgcgaggagg cccaaggcgg ggccaaggct 3780

tcaagctctc aggacttggc tatggaggag gaggaagaag gcaggagctc atccagtcca 3840

gccttaccta cagcaggaaa ctgcaccagc tag 3873

<210> 4
<211> 3768
<212> DNA
<213> Human

<400> 4
atgaatggat acgcggaatt tccgcccagc cccagtaacc ccaccaagga gcccgtggag 60

ccccagccca gccaggtccc actgcaggaa gatgtggaca tgagcagtgg ctccagtgga 120

catgagacca acgaaaactg ctccacgggg cgggactcgc agggcagtga ctgtgacgac 180

agtgggaagg agctggggat gctggtggag ccaccggatg cccgccagag tccagatacc 240

tttagcctga tgatggcaaa atctgaacac aacccatcta caagtggctg cagtagcgac 300

cagtcttcga aagtggacac acacaaagaa ctgataaaaa cactaaagga gctgaaggtc 360

cacctccctg cagacaagaa ggccaagggc aaggccagta cgctggccac cttgaagtac 420

gccctcagga gcgtgaagca ggtgaaagcc aatgaagagt attaccagct gctgatgtcc 480

agcgagggtc acccctgtgg agcagacgtg ccctcctaca ccgtggagga gatggagagc 540

gttacctctg agcacattgt gaagaatgcc gatatgtttg cggtggccgt gtccctggtg 600

tctgggaaga tcctgtacat ctctgaccag gttgcatcca tatttcactg taaaagagat 660

gccttcagcg atgccaagtt tgtggagttc ctggcgcctc acgatgtggg cgtgttccac 720

agtttcacct ccccgtacaa gcttcccttg tggagcatgt gcagtggagc agattctttt 780

actcaagaat gcatggagga gaaatctttc ttttgccgtg tcagtgtccg gaaaagccac 840

gagaatgaaa tccgctacca ccccttccgc atgacgccct acctggtcaa ggtgcgggac 900

caacaaggtg ctgagagtca gctttgctgc cttctgctgg cagagagagt gcactctggt 960

tatgaagccc ctagaattcc tcctgaaaag agaattttta caaccaccca tacaccaaat 1020

tgtttgttcc aggatgtgga tgaaagggcg gtccctctcc tgggctacct acctcaggac 1080

ctgattgaaa ccccagtgct cgtgcagctc caccctagtg acaggccctt gatgctggcc 1140

atccacaaaa agatcctgca gtcaggcggg cagcctttcg actattctcc cattcggttt 1200

cgcgcccgga acggagagta catcacgttg gacaccagct ggtccagctt catcaaccca 1260

tggagcagga aaatctcctt catcattggg aggcacaaag tcagggtggg ccctttgaat 1320

gaggacgtgt ttgcagccca cccctgcaca gaggagaagg ccctgcaccc cagcattcag 1380

gagctcacag agcagatcca ccggctcctg ctgcagcccg tcccccacag cggctccagt 1440

ggctacggga gtctgggcag caacgggtcc cacgagcacc ttatgagcca gacctcctcc 1500

agcgacagca acggccatga ggactcacgc cggaggagag ccgaaatttg taaaaatggt 1560

aacaagacca aaaatagaag tcattattct catgaatctg gagaacaaaa gaaaaaatcc 1620

gttacagaaa tgcaaactaa tcccccagct gagaagaaag ctgtccctgc catggaaaag 1680

gacagcctgg gggtcagctt ccccgaggag ttggcctgca agaaccagcc cacctgctcc 1740

taccagcaga tcagctgctt ggacagcgtc atcaggtact tggagagctg caatgaggct 1800

gccaccctga agaggaaatg cgagttccca gcaaacgtcc cagcgctaag gtccagtgat 1860

aagcggaagg ccacagtcag cccagggcca cacgctggag aggcagagcc gccctccagg 1920

gtgaacagcc gcacgggagt aggtacgcac ctgacctcgc tggcactgcc gggcaaggca 1980

gagagtgtgg cgtcgctcac cagccagtgc agctacagca gcaccatcgt ccatgtggga 2040

gacaagaagc cgcagccgga gttagagatg gtggaagatg ctgcgagtgg gccagaatcc 2100

ctggactgcc tggcgggccc tgccctggcc tgtggtctca gccaagagaa ggagcccttc 2160

aagaagctgg gcctcaccaa ggaggtactc gctgcacaca cacagaagga ggagcagagc 2220

ttcctgcaga agttcaaaga aataagaaaa ctcagcattt tccagtccca ctgccattac 2280

tacttgcaag aaagatccaa ggggcagcca agtgaacgaa ctgcccctgg actaagaaat 2340

acttccggaa tagattcacc ttggaaaaaa acaggaaaga acagaaaatt gaagtccaag 2400

cgggtcaaac ctcgagactc atctgagagc accggatctg gggggcccgt gtccgcccgg 2460

cccccgctgg tgggcttgaa cgccacagcc tggtcaccct cagacacgtc ccagtccagc 2520

tgcccagccg tgccctttcc cgccccagtg ccagcagctt attcactgcc cgtgtttcca 2580

gcgccaggga ctgtggcagc acccccggca cctccccacg ccagcttcac agtgcctgct 2640

gtgcccgtgg acctccagca ccagtttgca gtccagcccc cacctttccc tgcccctttg 2700

gcgcctgtca tggcattcat gctacccagt tattccttcc cctcggggac cccaaacctg 2760

ccccaggcct tcttccccag ccagcctcag tttccgagcc accccacact cacatccgag 2820

atggcctctg cctcacagcc tgagttcccc agccggacct cgatccccag acagccatgt 2880

gcttgtccag ccacccgggc caccccacca tcggccatgg gtagggcctc cccaccgctc 2940

tttcagtccc gcagcagctc gcccctgcag ctcaacctgc tgcagctgga ggaagcccct 3000

gagggtggca ctggagccat ggggaccaca ggggccacag agacagcagc tgtaggggcg 3060

gactgcaaac ctggcacttc tcgggaccag cagccgaagg cgcctctgac ccgtgatgaa 3120

ccctcagaca cacagaacag tgacgccctt tccacgtcaa gcggcctcct aaacctcctg 3180

ctgaatgagg acctctgctc agcctcgggc tctgctgctt cggagtctct gggctccggc 3240

tcactgggct gcgacgcctc cccgagtggg gcaggcagta gtgacacaag tcataccagc 3300

aaatattttg gaagcattga ctcctcagag aataatcaca aagcaaaaat gaacactggt 3360

atggaagaaa gtgagcattt cattaagtgc gtcctgcagg atcccatctg gctgctgatg 3420

gcagatgcgg acagcagcgt catgatgacg taccagctgc cttcccgaaa tttagaagcg 3480

gttttgaagg aggacagaga gaagctgaag ctcctacaga aactccagcc caggttcacg 3540

gagagtcaga agcaggagct gcgcgaggtc caccagtgga tgcagacggg cggcctgccc 3600

gcagccatcg acgtggcaga atgtgtttac tgtgaaaaca aggaaaaagg taatatttgc 3660

ataccatatg aggaagatat tccttctctg ggactcagcg aagtgtcgga caccaaagaa 3720

gacgaaaatg gatcccccctt gaatcacagg atcgaagagc agacgtaa 3768

<210> 5
<211> 3633
<212> DNA
<213> Human

<400> 5
atgccccgcg gggaagctcc tggccccggg agacgggggg ctaaggacga ggccctgggc 60

gaagaatcgg gggagcggtg gagccccgag ttccatctgc agaggaaatt ggcggacagc 120

agccacagtg aacagcaaga tcgaaacaga gtttctgaag aacttatcat ggttgtccaa 180

gaaatgaaaa aatacttccc ctcggagaga cgcaataaac caagcactct agatgccctc 240

aactatgctc tccgctgtgt ccacagcgtt caagcaaaca gtgagttttt ccagattctc 300

agtcagaatg gagcacctca ggcagatgtg agcatgtaca gtcttgagga gctggccact 360
atcgcttcag aacacacttc caaaaacaca gatacctttg tggcagtatt ttcatttctg 420
tctggaaggt tagtgcacat ttctgaacag gctgctttga tcctgaatcg taagaaagat 480
gtcctggcgt cttctcactt tgttgacctg cttgcacctc aagacatgag ggtattctac 540
gcgcacactg ccagagctca gcttcctttc tggaacaact ggacccaaag agcagctgca 600
cggtatgaat gtgctccggt gaaacctttt ttctgcagga tccgtggagg tgaagacaga 660
aagcaagaga agtgtcactc cccattccgg atcatcccct atctgattca tgtacatcac 720
cctgcccagc cagaattgga atcggaacct tgctgtctca ctgtggttga aaagattcac 780
tctggttatg aagctcctcg gatcccagtg aataaaagaa tcttcaccac cacacacacc 840
ccagggtgtg tttttcttga agtagatgaa aaagcagtgc ctttgctggg ttacctacct 900
caggacctga ttggaacatc gatcctaagc tacctgcacc ctgaagatcg ttctctgatg 960
gttgccatac accaaaaagt tttgaagtat gcagggcatc ctccctttga acattctccc 1020
attcgatttt gtactcaaaa cggagactac atcatactgg attccagttg gtccagcttt 1080
gtgaatccct ggagccggaa gatttctttc atcattggtc ggcataaagt tcgaacgagc 1140
ccactaaatg aggatgtttt tgctaccaaa attaaaaaga tgaacgataa tgacaaagac 1200
ataacagaat tacaagaaca aatttacaaa cttctcttac agccagttca cgtgagcgtg 1260
tccagcggct acgggagcct ggggagcagc gggtcgcagg agcagcttgt cagcatcgcc 1320
tcctccagtg aggccagtgg gcaccgtgtg gaggagacga aggcggagca gatgaccttg 1380
cagcaggtct atgccagtgt gaacaaaatt aaaaatctgg gtcagcagct ctacattgag 1440
tcaatgacca aatcatcatt caagccagtg acggggacac gcacagaacc gaatggtggt 1500
ggtgagtcag cgaatggtgg tggtgaatgt aagaccttta cttccttcca ccaaacactg 1560
aaaaacaata gtgtgtacac tgagccctgt gaggatttga ggaacgatga gcacagccca 1620
tcctatcaac agatcaactg tatcgacagt gtcatcagat acctgaagag ctacaacatt 1680
ccagctttga aaagaaagtg tatctcctgt acaaatacaa cttcttcctc ctcagaagaa 1740
gacaaacaga accacaaggc agatgatgtc caagccttac aagctggttt gcaaatccca 1800
gccataccta aatcagaaat gccaacaaat ggacggtcca tagacacagg aggaggagct 1860
ccacagatcc tgtccacggc gatgctgagc ttggggtcgg gcataagcca atgcggttac 1920
agcagcacca ttgtccatgt cccacccccca gagacagcca gggatgctac cctcttctgt 1980
gagccctgga ccctgaacat gcagccagcc cctttgacct cggaagaatt taaacacgtg 2040
gggctcacag cggctgttct gtcagcgcac acccagaagg aagagcagaa ttatgttgat 2100
aaattccgag aaaagatcct gtcatcaccc tacagctcct atcttcagca agaaagcagg 2160
agcaaagcta aatattcata ttttcaagga gattctactt ccaagcagac gcggtcggcc 2220

ggctgcagga aagggaagca caagcggaag aagctgccgg agccgccaga cagcagcagc 2280

tcgaacaccg gctctggtcc ccgcagggga gcgcatcaga acgcacagcc ctgctgcccc 2340

tccgcggcct cctctccgca cacctcgagc ccgaccttcc cacctgccgc catggtgccc 2400

agccaggccc cttacctcgt cccagctttt cccctcccag ccgcgacctc acccggaaga 2460

gaatacgcag cccccggaac tgcaccggaa ggcctgcatg ggctgccctt gtccgagggc 2520

ttgcagcctt acccagcttt ccctttttcct tacttggata cttttatgac cgttttcctg 2580

cctgaccccc ctgtctgtcc tctgttgtcg ccatcgtttt tgccatgtcc attcctgggg 2640

gcgacagcct cttctgcgat atcaccctca atgtcgtcag caatgagtcc aactctggac 2700

ccaccccctt cagtcaccag ccaaaggaga gaggaggaaa agtgggaggc acaaagcgag 2760

gggcacccgt tcattacttc gagaagcagc tcacccttgc agttaaactt acttcaggaa 2820

gagatgccca gaccctctga atctccagat cagatgagaa ggaacacgtg cccacaaact 2880

gagtatcagt gtgttacagg caacaatggc agtgagagca gtcctgctac taccggtgca 2940

ctgtccacgg ggtcacctcc cagggagaat ccatcccatc ctactgccag cgctctgtcc 3000

acaggatcgc ctcccatgaa gaatccatcc catcctactg ccagcgctct gtccacagga 3060

tcgcctccca tgaagaatcc atcccatcct actgccagca cactgtccat gggattgcct 3120

cccagcagga ctccatccca tcctactgcc actgttctgt ccacggggtc acctcccagc 3180

gaatccccat ccagaactgg ttcagcagca tcaggaagca gcgacagcag tatatacctt 3240

actagtagtg tttattcttc taaaatctcc caaaatgggc agcaatctca ggacgtacag 3300

aaaaaagaaa catttcctaa tgtcgccgaa gagcccatct ggagaatgat acggcagaca 3360

cctgagcgca ttctcatgac ataccaggta cctgagaggg ttaaagaagt tgtactaaaa 3420

gaagacctgg aaaagctaga aagtatgagg cagcagcagc cccagttttc tcatgggcaa 3480

aaggaggagc tggctaaggt gtataattgg attcaaagcc agactgtcac tcaagaaatc 3540

gacattcaag cctgtgtcac ttgtgaaaat gaagattcag ctgatggtgc ggccacatcc 3600

tgtggtcagg ttctggtaga agacagctgt tga 3633

<210> 6
<211> 2541
<212> DNA
<213> Human

<400> 6
atgttgttta ccgtaagctg tagtaaaatg agctcgattg ttgacagaga tgacagtagt 60

atttttgatg ggttggtgga agaagatgac aaggacaaag cgaaaagagt atctagaaac 120

aaatctgaaa agaaacgtag agatcaattt aatgttctca ttaaagaact gggatccatg 180

cttcctggta atgctagaaa gatggacaaa tctactgttc tgcagaaaag cattgatttt 240

ttacgaaaac ataaagaaat cactgcacag tcagatgcta gtgaaattcg acaggactgg 300
aaacctacat tccttagtaa tgaagagttt acacaattaa tgttagaggc tcttgatggt 360
tttttttag caatcatgac agatggaagc ataatatatg tgtctgagag tgtaacttca 420
ttacttgaac atttaccatc tgatcttgtg gatcaaagta tatttaattt tatcccagaa 480
ggggaacatt cagaggttta taaaatactc tctactcatc tgctggaaag tgattcatta 540
accccagaat atttaaaatc aaaaaatcag ttagaattct gttgtcacat gctgcgagga 600
acaatagacc caaaggagcc atctacctat gaatatgtaa aatttatagg aaatttcaaa 660
tctttaaaca gtgtatcctc ttcagcacac aatggttttg aaggaactat acaacgcaca 720
cataggccat cttatgaaga tagagtttgt tttgtagcta ctgtcaggtt agctacacct 780
cagttcatca aggaaatgtg cactgttgaa gaacccaatg aagagtttac atctagacat 840
agtttagaat ggaagtttct gtttctagat cacagggcac cacccataat agggtatttg 900
ccatttgaag ttctgggaac atcaggctat gattactatc atgtggatga cctagaaaat 960
ttggcaaaat gtcatgagca cttaatgcaa tatgggaaag gcaaatcatg ttattatagg 1020
ttcctgacta aggggcaaca gtggatttgg cttcagactc attattatat cacttaccat 1080
cagtggaatt caaggccaga gtttattgtt tgtactcaca ctgtagtaag ttatgcagaa 1140
gttagggctg aaagacgacg agaacttggc attgaagagt ctcttcctga gacagctgct 1200
gacaaaagcc aagattctgg gtcagataat cgtataaaca cagtcagtct caaggaagca 1260
ttggaaaggt ttgatcacag cccaacccct tctgcctctt ctcggagttc aagaaaatca 1320
tctcacacgg ccgtctcaga cccttcctca acaccaacca agatcccgac ggatacgagc 1380
actccaccca ggcagcattt accagctcat gagaagatgg tgcaaagaag gtcatcattt 1440
agtagtcagt ccataaattc ccagtctgtt ggttcatcat taacacagcc agtgatgtct 1500
caagctacaa atttaccaat tccacaaggc atgtcccagt ttcagttttc agctcaatta 1560
ggagccatgc aacatctgaa agaccaattg gaacaacgga cacgcatgat agaagcaaat 1620
attcatcggc aacaagaaga actaagaaaa attcaagaac aacttcagat ggtccatggt 1680
caggggctgc agatgttttt gcaacaatca aatcctgggt tgaattttgg ttccgttcaa 1740
ctttcttctg gaaattcatc taatatccag caacttgcac ctataaatat gcaaggccaa 1800
gttgttccta ctaaccagat tcaaagtgga atgaatactg gacacattgg cacaactcag 1860
cacatgatac aacaacagac tttacagagt acatcaactc agagtcaaca aaatgtactg 1920
agtgggcaca gtcagcaaac atctctaccc agtcagacac agagcactct tacagcccca 1980
ctgtataaca ctatggtgat ttctcagcct gcagccggaa gcatggtcca gattccatct 2040
agtatgccac aaaacagcac ccagagtgct gcagtaacta cattcactca ggacaggcag 2100

```
ataagatttt ctcaaggtca acaacttgtg accaaattag tgactgctcc tgtagcttgt    2160
ggggcagtca tggtacctag tactatgctt atgggccagg tggtgactgc atatcctact    2220
tttgctacac aacagcaaca gtcacagaca ttgtcagtaa cgcagcagca gcagcagcag    2280
agctcccagg agcagcagct cacttcagtt cagcaaccat ctcaggctca gctgacccag    2340
ccaccgcaac aatttttaca gacttctagg ttgctccatg ggaatccctc aactcaactc    2400
attctctctg ctgcatttcc tctacaacag agcaccttcc ctcagtcaca tcaccagcaa    2460
catcagtctc agcaacagca gcaactcagc cggcacagga ctgacagctt gcccgaccct    2520
tccaaggttc aaccacagta g                                              2541
```

<210> 7
<211> 2475
<212> DNA
<213> Human

<400> 7

```
atggatgaag atgagaaaga cagagccaag agagcttctc gaaacaagtc tgagaagaag      60
cgtcgggacc agttcaatgt tctcatcaaa gagctcagtt ccatgctccc tggcaacacg     120
cggaaaatgg acaaaaccac cgtgttggaa aaggtcatcg gatttttgca gaaacacaat     180
gaagtctcag cgcaaacgga aatctgtgac attcagcaag actggaagcc ttcattcctc     240
agtaatgaag aattcaccca gctgatgttg gaggcattag atggcttcat tatcgcagtg     300
acaacagacg gcagcatcat ctatgtctct gacagtatca cgcctctcct tgggcattta     360
ccgtcggatg tcatggatca gaatttgtta aatttcctcc cagaacaaga acattcagaa     420
gtttataaaa tcctttcttc ccatatgctt gtgacggatt ccccctcccc agaatactta     480
aaatctgaca gcgatttaga gttttattgc catcttctca gaggcagctt gaacccaaag     540
gaatttccaa cttatgaata cataaaattt gtaggaaatt ttcgctctta caacaatgtg     600
cctagcccct cctgtaatgg ttttgacaac accctttcaa gaccttgccg ggtgccacta     660
ggaaaggagg tttgcttcat tgccaccgtt cgtctggcaa caccacaatt cttaaaggaa     720
atgtgcatag ttgacgaacc tttagaggaa ttcacttcaa ggcatagctt ggaatggaaa     780
tttttatttc tggatcacag agcacctcca atcataggat acctgccttt tgaagtgctg     840
ggaacctcag gctatgacta ctaccacatt gatgacctgg agctcctggc caggtgtcac     900
cagcacctga tgcagtttgg caaagggaag tcgtgttgct accggtttct gaccaaaggt     960
cagcagtgga tctggctgca gactcactac tacatcacct accatcagtg gaactccaag    1020
cccgagttca tcgtgtgcac acactcggtg gtcagttacg cagatgtccg ggtggaaagg    1080
aggcaggagc tggctctgga agacccgcca tccgaggccc tccactcctc agcactaaag    1140
gacaagggct caagcctgga acctcggcag cactttaaca cactcgacgt gggtgcctcg    1200
```

```
ggccttaata ccagtcattc gccatcggcg tcctcaagaa gttcccacaa atcctcgcac      1260
acagccatgt cagaacccac ctccactccc accaagctga tggcagaggc cagcaccccg      1320
gctttgccaa gatcagccac cctgccccaa gagttacctg tccccgggct cagccaggca      1380
gccaccatgc cggcccctct gccttcccca tcgtcctgcg acctcacaca gcagctcctg      1440
cctcagaccg ttctgcagag cacgcccgct cccatggcac agttttcggc acagttcagc      1500
atgttccaga ccatcaaaga ccagctagag cagcggacgc ggatcctgca ggccaatatc      1560
cggtggcaac aggaagagct ccacaagatc caggagcagc tctgcctggt ccaggactcc      1620
aacgtccaga tgttcctgca gcagccagct gtatccctga gcttcagcag cacccagcga      1680
cctgaggctc agcagcagct acagcaaagg tcagctgcag tgactcagcc ccagctcggg      1740
gcgggccccc aacttccagg gcagatctcc tctgcccagg tcacaagcca gcacctgctc      1800
agagaatcaa gtgtgatatc aacccagggt ccaaagccaa tgagaagctc acagctaatg      1860
cagagcagcg gccgctctgg aagcagccta gtgtccccgt tcagcagcgc cacagctgcg      1920
ctcccgccaa gtctgaatct gaccacacct gcttccacct cccaggatgc cagccagtgc      1980
cagcccagcc cagacttcag ccatgatcgg cagctcaggc tgttgctgag ccagcccatc      2040
cagcccatga tgcccgggtc ctgtgacgca aggcagccct cggaagtcag caggacggga      2100
cggcaagtca agtacgccca gagccagacc gtgtttcaaa atccagacgc acaccccgcc      2160
aacagcagca gcgccccgat gcccgtcctg ctgatggggc aggcggtgct ccaccccagc      2220
ttccctgcct cccaaccatc gcccctgcag cctgcacagg cccggcagca gccaccgcag      2280
cactacctgc aggtacaggc accaacctct ttgcacagtg agcagcagga ctcgctactt      2340
ctctccacct actcacaaca gccagggacc ctgggctacc cccaaccacc cccagcacag      2400
ccccagcccc tacgtcctcc ccgaagggtc agcagtctgt ctgagtcgtc aggcctccag      2460
cagccgcccc gataa                                                       2475
```

<210> 8
<211> 1761
<212> DNA
<213> Human

<400> 8

```
atgggggtga acgccgtgca ctggttccga aaggggctcc ggctccacga caaccccgcc        60
ctgaaggagt gcattcaggg cgccgacacc atccgctgcg tctacatcct ggacccctgg       120
ttcgccggct cctccaatgt gggcatcaac aggtggcgat ttttgcttca gtgtcttgag       180
gatcttgatg ccaatctacg aaaattaaac tcccgtctgt ttgtgattcg tggacaacca       240
gcagatgtgt ttcccaggct tttcaaggaa tggaacatta ctaaactttc aattgagtat       300
gattctgagc cctttggaaa ggaacgagac gcagctatta agaaactggc aactgaagct       360
```

```
ggagtagaag tcattgtaag aatttcacat acattatatg acctagacaa gatcatagaa      420

ctcaatggtg gacaaccgcc tctaacttat aaaagattcc agactctcat cagcaaaatg      480

gaaccactag agataccagt agagacaatt acttcagaag tgatagaaaa gtgcacaact      540

cctctgtctg atgaccatga tgagaaatat ggagtccctt cactggaaga gctaggtttt      600

gatacagatg gcttatcctc tgcagtgtgg ccaggtggag aaactgaagc acttactcgt      660

ttggaaaggc atttggaaag aaaagcttgg gtggcaaatt ttgaaagacc tcgaatgaat      720

gcgaattctc tgcttgcaag ccctactgga cttagtcctt atctccgatt tggttgtttg      780

tcatgtcgac tgttttactt caaactaaca gatctctaca aaaaggtaaa gaagaacagt      840

tcccctcccc tttcccttta tgggcaactg ttatggcgtg aatttttcta tacagcagca      900

acaaataatc cacgctttga taaaatggaa ggaaacccta tctgtgttca gattccttgg      960

gataaaaatc ctgaggcttt agccaaatgg gcggaaggcc ggacaggctt tccatggatt     1020

gatgccatca tgacacagct tcgtcaggag ggttggattc atcatctagc caggcatgca     1080

gttgcttgct tcctgacacg aggggacctg tggattagtt gggaagaagg aatgaaggta     1140

tttgaagaat tattgcttga tgcagattgg agcataaatg ctggaagttg gatgtggctg     1200

tcttgtagtt cctttttca acagtttttt cactgctatt gccctgttgg ttttggtagg     1260

agaacagatc ccaatggaga ctatatcagg cgttatttgc ctgtcctaag aggcttccct     1320

gcaaaatata tctatgatcc ctggaatgca ccagaaggta tccaaaaggt agccaaatgt     1380

ttgataggag ttaattatcc taaaccaatg gtgaaccatg ctgaggcaag ccgtttgaat     1440

atcgaaagga tgaaacagat ctatcagcag ctttcacgat atagaggact aggtcttctg     1500

gcatcagtac cttctaatcc taatgggaat ggaggcttca tgggatattc tgcagaaaat     1560

atcccaggtt gtagcagcag tggaagttgc tctcaaggga gtggtatttt acactatgct     1620

catggcgaca gtcagcaaac tcacctgttg aagcaaggaa gaagctccat gggcactggt     1680

ctcagtggtg ggaaacgtcc tagtcaggaa gaggacacac agagtattgg tcctaaagtc     1740

cagagacaga gcactaatta g                                               1761
```

```
<210>  9
<211>  1845
<212>  DNA
<213>  Human

<400>  9
atgggcgggg tccacgtcgc ctaccggggc ggagcggggg tggctggagc agtctggaca       60

gtcatggcgg cgactgtggc gacggcggca gctgtggccc cggcgccagc gcccggcacg      120

gacagcgcct cttcggtgca ctggttccgc aaagggctgc gactccacga caacccggcg      180

ttgctggcgg ccgtgcgcgg ggcgcgctgc gtgcgctgcg tttacattct cgacccgtgg      240
```

ttcgcggcct cctcctcagt cgggatcaac cgatggaggt tcctacttca gtctctggaa 300

gatttggaca caagtttaag gaaactgaac tcccgcctgt ttgtagtccg gggacagcca 360

gccgacgtgt tcccaaggct gttcaaggaa tggggagtga cccgcttgac ctttgaatat 420

gactctgaac cctttgggaa agaacgggat gcagccatca tgaagatggc caaggaggct 480

ggtgtggaag tagtgacgga gaattctcat accctctatg acctggacag gatcattgag 540

ctgaatgggc agaagccacc ccttacatac aagcgctttc aggccatcat cagccgcatg 600

gagctgccca agaagccagt gggcttggtg accagccagc agatggagag ctgcagggcc 660

gagatccagg agaaccacga cgagacctac ggcgtgccct ccctggagga gctggggttc 720

cccactgaag gacttggtcc agctgtctgg cagggaggag agacagaagc tctggcccgc 780

ctggataagc acttggaacg gaaggcctgg gttgccaact atgagagacc ccgaatgaac 840

gccaactccc tcctggccag ccccacaggc ctcagcccct acctgcgctt tggttgtctc 900

tcctgccgcc tcttctacta ccgcctgtgg gacctgtata aaaaggtgaa gcggaacagc 960

acacctcccc tctccctatt tgggcaactc ctatggcgag agttcttcta cacggcagct 1020

accaacaacc ccaggtttga ccgcatggag gggaacccca tctgcatcca gatcccctgg 1080

gaccgcaatc ctgaggccct ggccaagtgg gctgagggca agacaggctt cccttggatt 1140

gatgccatca tgacccaact gaggcaggag ggctggatcc accacctggc ccggcatgcc 1200

gtggcctgct tcctgacccg cggggacctc tgggtcagct gggagagcgg ggtccgggta 1260

tttgatgagc tgctcctgga tgcagatttc agcgtgaacg caggcagctg gatgtggctg 1320

tcctgcagtg ctttcttcca gcagttcttc cactgctact gccctgtggg ctttggccgt 1380

cgcacggacc ccagtgggga ctacatcagg cgatacctgc ccaaattgaa agcgttcccc 1440

tctcgataca tctatgagcc ctggaatgcc ccagagtcaa ttcagaaggc agccaagtgc 1500

atcattggtg tggactaccc acggcccatc gtcaaccatg ccgagaccag ccggcttaac 1560

attgaacgaa tgaagcagat ttaccagcag ctttcgcgct accggggact ctgtctactg 1620

gcatctgtcc cttcctgtgt ggaagacctc agtcaccctg tggcagagcc cagctcgagc 1680

caggctggca gcatgagcag tgcaggccca agaccactac ccagtggccc agcatccccc 1740

aaacgcaagc tggaagcagc cgaggaacca cctggtgaag aactcagcaa acgggcccgg 1800

gtggcagagt tgccaacccc agagctgccg agcaaggatg cctga 1845

<210> 10
<211> 1845
<212> DNA
<213> Human

<400> 10
atgacgaccc tggactccaa caacaacaca ggtggcgtca tcacctacat tggctccagt 60

ggctcctccc caagccgcac cagccctgaa tccctctata gtgacaactc caatggcagc 120
ttccagtccc tgacccaagg ctgtcccacc tacttcccac catcccccac tggctccctc 180
acccaagacc cggctcgctc ctttgggagc attccaccca gcctgagtga tgacggctcc 240
ccttcttcct catcttcctc gtcgtcatcc tcctcctcct tctataatgg gagcccccct 300
gggagtctac aagtggccat ggaggacagc agccgagtgt cccccagcaa gagcaccagc 360
aacatcacca agctgaatgg catggtgtta ctgtgtaaag tgtgtgggga cgttgcctcg 420
ggcttccact acggtgtgca cgcctgcgag ggctgcaagg gctttttccg tcggagcatc 480
cagcagaaca tccagtacaa aaggtgtctg aagaatgaga attgctccat cgtccgcatc 540
aatcgcaacc gctgccagca atgtcgcttc aagaagtgtc tctctgtggg catgtctcga 600
gacgctgtgc gttttgggcg catccccaaa cgagagaagc agcggatgct tgctgagatg 660
cagagtgcca tgaacctggc caacaaccag ttgagcagcc agtgcccgct ggagacttca 720
cccacccagc accccacccc aggccccatg ggcccctcgc caccccctgc tccggtcccc 780
tcacccctgg tgggcttctc ccagtttcca caacagctga cgcctcccag atccccaagc 840
cctgagccca cagtggagga tgtgatatcc caggtggccc gggcccatcg agagatcttc 900
acctacgccc atgacaagct gggcagctca cctggcaact tcaatgccaa ccatgcatca 960
ggtagccctc cagccaccac cccacatcgc tgggaaaatc agggctgccc acctgccccc 1020
aatgacaaca acaccttggc tgcccagcgt cataacgagg ccctaaatgg tctgcgccag 1080
gctccctcct cctaccctcc cacctggcct cctggccctg cacaccacag ctgccaccag 1140
tccaacagca acgggcaccg tctatgcccc acccacgtgt atgcagcccc agaaggcaag 1200
gcacctgcca acagtccccg gcagggcaac tcaaagaatg ttctgctggc atgtcctatg 1260
aacatgtacc cgcatggacg cagtgggcga acggtgcagg agatctggga ggatttctcc 1320
atgagcttca cgcccgctgt gcgggaggtg gtagagtttg ccaaacacat cccgggcttc 1380
cgtgaccttt ctcagcatga ccaagtcacc ctgcttaagg ctggcacctt tgaggtgctg 1440
atggtgcgct ttgcttcgtt gttcaacgtg aaggaccaga cagtgatgtt cctaagccgc 1500
accacctaca gcctgcagga gcttggtgcc atgggcatgg gagacctgct cagtgccatg 1560
ttcgacttca gcgagaagct caactccctg gcgcttaccg aggaggagct gggcctcttc 1620
accgcggtgg tgcttgtctc tgcagaccgc tcgggcatgg agaattccgc ttcggtggag 1680
cagctccagg agacgctgct gcgggctctt cgggctctgg tgctgaagaa ccggcccttg 1740
gagacttccc gcttcaccaa gctgctgctc aagctgccgg acctgcggac cctgaacaac 1800
atgcattccg agaagctgct gtccttccgg gtggacgccc agtga 1845

<210> 11
<211> 1740

<212> DNA
<213> Human

<400> 11

```
atggaggtga atgcaggagg tgtgattgcc tatatcagtt cttccagctc agcctcaagc      60
cctgcctctt gtcacagtga gggttctgag aatagtttcc agtcctcctc ctcttctgtt     120
ccatcttctc caaatagctc taattctgat accaatggta atcccaagaa tggtgatctc     180
gccaatattg aaggcatctt gaagaatgat cgaatagatt gttctatgaa aacaagcaaa     240
tcgagtgcac ctgggatgac aaaaagtcat agtggtgtga caaaatttag tggcatggtt     300
ctactgtgta aagtctgtgg ggatgtggcg tcaggattcc actatggagt tcatgcttgc     360
gaaggctgta agggtttctt tcggagaagt attcaacaaa acatccagta caagaagtgc     420
ctgaagaatg aaaactgttc tataatgaga atgaatagga acagatgtca gcaatgtcgc     480
ttcaaaaagt gtctgtctgt tggaatgtca agagatgctg ttcggtttgg tcgtattcct     540
aagcgtgaaa aacagaggat gctaattgaa atgcaaagtg caatgaagac catgatgaac     600
agccagttca gtggtcactt gcaaaatgac acattagtag aacatcatga acagacagcc     660
ttgccagccc aggaacagct gcgacccaag ccccaactgg agcaagaaaa catcaaaagc     720
tcttctcctc catcttctga ttttgcaaag gaagaagtga ttggcatggt gaccagagct     780
cacaaggata cctttatgta taatcaagag cagcaagaaa actcagctga gagcatgcag     840
ccccagagag gagaacggat tcccaagaac atggagcaat ataatttaaa tcatgatcat     900
tgcggcaatg ggcttagcag ccattttccc tgtagtgaga gccagcagca tctcaatgga     960
cagttcaaag ggaggaatat aatgcattac ccaaatggtc atgccatttg tattgcaaat    1020
ggacattgta tgaacttctc caatgcttat actcaaagag tatgtgatag agttccgata    1080
gatggatttt ctcagaatga gaacaagaat agttacctgt gcaacactgg aggaagaatg    1140
catctggttt gtccaatgag taagtctcca tatgtggatc ctcataaatc aggacatgaa    1200
atctgggaag aattttcgat gagcttcact ccagcagtga aagaagtggt ggaatttgca    1260
aagcgtattc ctgggttcag agatctctct cagcatgacc aggtcaacct tttaaaggct    1320
gggacttttg aggttttaat ggtacggttc gcatcattat ttgatgcaaa ggaacgtact    1380
gtcacctttt taagtggaaa gaaatatagt gtggatgatt tacactcaat gggagcaggg    1440
gatctgctaa actctatgtt tgaatttagt gagaagctaa atgccctcca acttagtgat    1500
gaagagatga gtttgtttac agctgttgtc ctggtatctg cagatcgatc tggaatagaa    1560
aacgtcaact ctgtggaggc tttgcaggaa actctcattc gtgcactaag gaccttaata    1620
atgaaaaacc atccaaatga ggcctctatt tttacaaaac tgcttctaaa gttgccagat    1680
cttcgatctt taaacaacat gcactctgag gagctcttgg cctttaaagt tcacccttaa    1740
```

<210> 12
<211> 1572
<212> DNA
<213> Human

<400> 12
atggagtcag ctccggcagc ccccgacccc gccgccagcg agccaggcag cagcggcgcg 60

gacgcggccg ccggctccag ggagaccccg ctgaaccagg aatccgcccg caagagcgag 120

ccgcctgccc cggtgcgcag acagagctat tccagcacca gcagaggtat ctcagtaacg 180

aagaagacac atacatctca aattgaaatt attccatgca agatctgtgg agacaaatca 240

tcaggaatcc attatggtgt cattacatgt gaaggctgca agggcttttt caggagaagt 300

cagcaaagca atgccaccta ctcctgtcct cgtcagaaga actgtttgat tgatcgaacc 360

agtagaaacc gctgccaaca ctgtcgatta cagaaatgcc ttgccgtagg gatgtctcga 420

gatgctgtaa aatttggccg aatgtcaaaa aagcagagag acagcttgta tgcagaagta 480

cagaaacacc ggatgcagca gcagcagcgc gaccaccagc agcagcctgg agaggctgag 540

ccgctgacgc ccacctacaa catctcggcc aacgggctga cggaacttca cgacgacctc 600

agtaactaca ttgacgggca cacccctgag gggagtaagg cagactccgc cgtcagcagc 660

ttctacctgg acatacagcc ttccccagac cagtcaggtc ttgatatcaa tggaatcaaa 720

ccagaaccaa tatgtgacta cacaccagca tcaggcttct ttccctactg ttcgttcacc 780

aacggcgaga cttccccaac tgtgtccatg gcagaattag aacaccttgc acagaatata 840

tctaaatcgc atctggaaac ctgccaatac ttgagagaag agctccagca gataacgtgg 900

cagacctttt tacaggaaga aattgagaac tatcaaaaca agcagcggga ggtgatgtgg 960

caattgtgtg ccatcaaaat tacagaagct atacagtatg tggtggagtt tgccaaacgc 1020

attgatggat ttatggaact gtgtcaaaat gatcaaattg tgcttctaaa agcaggttct 1080

ctagaggtgg tgtttatcag aatgtgccgt gcctttgact ctcagaacaa caccgtgtac 1140

tttgatggga agtatgccag ccccgacgtc ttcaaatcct taggttgtga agactttatt 1200

agctttgtgt ttgaatttgg aaagagttta tgttctatgc acctgactga agatgaaatt 1260

gcattatttt ctgcatttgt actgatgtca gcagatcgct catggctgca agaaaaggta 1320

aaaattgaaa aactgcaaca gaaaattcag ctagctcttc aacacgtcct acagaagaat 1380

caccgagaag atggaatact aacaaagtta atatgcaagg tgtctacctt aagagcctta 1440

tgtggacgac atacagaaaa gctaatggca tttaaagcaa tatacccaga cattgtgcga 1500

cttcattttc ctccattata caaggagttg ttcacttcag aatttgagcc agcaatgcaa 1560

attgatgggt aa 1572

<210> 13
<211> 1380

<212> DNA
<213> Human

<400> 13
atgcgagcac aaattgaagt gataccatgc aaaatttgtg gcgataagtc ctctgggatc 60

cactacggag tcatcacatg tgaaggctgc aagggattct ttaggaggag ccagcagaac 120

aatgcttctt attcctgccc aaggcagaga aactgtttaa ttgacagaac gaacagaaac 180

cgttgccaac actgccgact gcagaagtgt cttgccctag gaatgtcaag agatgctgtg 240

aagtttggga ggatgtccaa gaagcaaagg gacagcctgt atgctgaggt gcagaagcac 300

cagcagcggc tgcaggaaca gcggcagcag cagagtgggg aggcagaagc ccttgccagg 360

gtgtacagca gcagcattag caacggcctg agcaacctga acaacgagac cagcggcact 420

tatgccaacg ggcacgtcat tgacctgccc aagtctgagg gttattacaa cgtcgattcc 480

ggtcagccgt cccctgatca gtcaggactt gacatgactg gaatcaaaca gataaagcaa 540

gaacctatct atgacctcac atccgtaccc aacttgttta cctatagctc tttcaacaat 600

gggcagttag caccagggat aaccatgact gaaatcgacc gaattgcaca gaacatcatt 660

aagtcccatt tggagacatg tcaatacacc atggaagagc tgcaccagct ggcgtggcag 720

acccacacct atgaagaaat taaagcatat caaagcaagt ccagggaagc actgtggcaa 780

caatgtgcca tccagatcac tcacgccatc caatacgtgg tggagtttgc aaagcggata 840

acaggcttca tggagctctg tcaaaatgat caaattctac ttctgaagtc aggttgcttg 900

gaagtggttt tagtgagaat gtgccgtgcc ttcaacccat taaacaacac tgttctgttt 960

gaaggaaaat atggaggaat gcaaatgttc aaagccttag gttctgatga cctagtgaat 1020

gaagcatttg actttgcaaa gaatttgtgt tccttgcagc tgaccgagga ggagatcgct 1080

ttgttctcat ctgctgttct gatatctcca gaccgagcct ggcttataga accaaggaaa 1140

gtccagaagc ttcaggaaaa aatttatttt gcacttcaac atgtgattca gaagaatcac 1200

ctggatgatg agaccttggc aaagttaata gccaagatac caaccatcac ggcagtttgc 1260

aacttgcacg gggagaagct gcaggtattt aagcaatctc atccagagat agtgaataca 1320

ctgtttcctc cgttatacaa ggagctcttt aatcctgact gtgccaccgg ctgcaaatga 1380

<210> 14
<211> 1557
<212> DNA
<213> Human

<400> 14
atggacaggg ccccacagag acagcaccga gcctcacggg agctgctggc tgcaaagaag 60

acccacacct cacaaattga agtgatccct tgcaaaatct gtggggacaa gtcgtctggg 120

atccactacg gggttatcac ctgtgagggg tgcaagggct tcttccgccg gagccagcgc 180

tgtaacgcgg cctactcctg cacccgtcag cagaactgcc ccatcgaccg caccagccga 240

aaccgatgcc agcactgccg cctgcagaaa tgcctggcgc tgggcatgtc ccgagatgct 300

gtcaagttcg gccgcatgtc caagaagcag agggacagcc tgcatgcaga agtgcagaaa 360

cagctgcagc agcggcaaca gcagcaacag gaaccagtgg tcaagacccc tccagcaggg 420

gcccaaggag cagataccct cacctacacc ttggggctcc cagacgggca gctgcccctg 480

ggctcctcgc ctgacctgcc tgaggcttct gcctgtcccc ctggcctcct gaaagcctca 540

ggctctgggc cctcatattc caacaacttg gccaaggcag ggctcaatgg ggcctcatgc 600

caccttgaat acagccctga gcggggcaag gctgagggca gagagagctt ctatagcaca 660

ggcagccagc tgacccctga ccgatgtgga cttcgttttg aggaacacag gcatcctggg 720

cttggggaac tgggacaggg cccagacagc tacggcagcc ccagtttccg cagcacaccg 780

gaggcaccct atgcctccct gacagagata gagcacctgg tgcagagcgt ctgcaagtcc 840

tacagggaga catgccagct gcggctggag gacctgctgc ggcagcgctc caacatcttc 900

tcccgggagg aagtgactgg ctaccagagg aagtccatgt gggagatgtg ggaacggtgt 960

gcccaccacc tcaccgaggc cattcagtac gtggtggagt tcgccaagag gctctcaggc 1020

tttatggagc tctgccagaa tgaccagatt gtgcttctca aagcaggagc aatggaagtg 1080

gtgctggtta ggatgtgccg ggcctacaat gctgacaacc gcacggtctt ttttgaaggc 1140

aaatacggtg gcatggagct gttccgagcc ttgggctgca gcgagctcat cagctccatc 1200

tttgacttct cccactccct aagtgccttg cacttttccg aggatgagat tgccctctac 1260

acagcccttg ttctcatcaa tgcccatcgg ccagggctcc aagagaaaag gaaagtagaa 1320

cagctgcagt acaatctgga gctggccttt catcatcatc tctgcaagac tcatcgccaa 1380

agcatcctgg caaagctgcc acccaagggg aagcttcgga gcctgtgtag ccagcatgtg 1440

gaaaggctgc agatcttcca gcacctccac cccatcgtgg tccaagccgc tttccctcca 1500

ctctacaagg agctcttcag cactgaaacc gagtcacctg tggggctgtc caagtga 1557

<210> 15
<211> 1443
<212> DNA
<213> Human

<400> 15
atgagcgacg tggctattgt gaaggagggt tggctgcaca aacgagggga gtacatcaag 60

acctggcggc cacgctactt cctcctcaag aatgatggca ccttcattgg ctacaaggag 120

cggccgcagg atgtggacca acgtgaggct cccctcaaca acttctctgt ggcgcagtgc 180

cagctgatga agacggagcg gccccggccc aacaccttca tcatccgctg cctgcagtgg 240

accactgtca tcgaacgcac cttccatgtg gagactcctg aggagcggga ggagtggaca 300

accgccatcc agactgtggc tgacggcctc aagaagcagg aggaggagga gatggacttc 360

cggtcgggct cacccagtga caactcaggg gctgaagaga tggaggtgtc cctggccaag 420

cccaagcacc gcgtgaccat gaacgagttt gagtacctga agctgctggg caagggcact 480

ttcggcaagg tgatcctggt gaaggagaag gccacaggcc gctactacgc catgaagatc 540

ctcaagaagg aagtcatcgt ggccaaggac gaggtggccc acacactcac cgagaaccgc 600

gtcctgcaga actccaggca ccccttcctc acagccctga agtactcttt ccagacccac 660

gaccgcctct gctttgtcat ggagtacgcc aacgggggcg agctgttctt ccacctgtcc 720

cgggagcgtg tgttctccga ggaccgggcc cgcttctatg gcgctgagat tgtgtcagcc 780

ctggactacc tgcactcgga gaagaacgtg gtgtaccggg acctcaagct ggagaacctc 840

atgctggaca aggacgggca cattaagatc acagacttcg ggctgtgcaa ggaggggatc 900

aaggacggtg ccaccatgaa gacctttgc ggcacacctg agtacctggc ccccgaggtg 960

ctggaggaca atgactacgg ccgtgcagtg gactggtggg ggctgggcgt ggtcatgtac 1020

gagatgatgt gcggtcgcct gcccttctac aaccaggacc atgagaagct tttgagctc 1080

atcctcatgg aggagatccg cttcccgcgc acgcttggtc ccgaggccaa gtccttgctt 1140

tcagggctgc tcaagaagga ccccaagcag aggcttggcg gggctccga ggacgccaag 1200

gagatcatgc agcatcgctt ctttgccggt atcgtgtggc agcacgtgta cgagaagaag 1260

ctcagcccac ccttcaagcc ccaggtcacg tcggagactg acaccaggta ttttgatgag 1320

gagttcacgg cccagatgat caccatcaca ccacctgacc aagatgacag catggagtgt 1380

gtggacagcg agcgcaggcc ccacttcccc cagttctcct actcggccag cggcacggcc 1440

tga 1443

<210> 16
<211> 963
<212> DNA
<213> Human

<400> 16
atggagctac tgtcgccacc gctccgcgac gtagacctga cggcccccga cggctctctc 60

tgctcctttg ccacaacgga cgacttctat gacgacccgt gtttcgactc cccggacctg 120

cgcttcttcg aagacctgga cccgcgcctg atgcacgtgg gcgcgctcct gaaacccgaa 180

gagcactcgc acttccccgc ggcggtgcac ccggccccgg gcgcacgtga ggacgagcat 240

gtgcgcgcgc ccagcgggca ccaccaggcg ggccgctgcc tactgtgggc ctgcaaggcg 300

tgcaagcgca agaccaccaa cgccgaccgc cgcaaggccg ccaccatgcg cgagcggcgc 360

cgcctgagca aagtaaatga ggcctttgag acactcaagc gctgcacgtc gagcaatcca 420

aaccagcggt tgcccaaggt ggagatcctg cgcaacgcca tccgctatat cgagggcctg 480

caggctctgc tgcgcgacca ggacgccgcg ccccctggcg ccgcagccgc cttctatgcg 540

ccgggcccgc tgcccccggg ccgcggcggc gagcactaca gcggcgactc cgacgcgtcc 600

agcccgcgct ccaactgctc cgacggcatg atggactaca gcggcccccc gagcggcgcc 660

cggcggcgga actgctacga aggcgcctac tacaacgagg cgcccagcga acccaggccc 720

gggaagagtg cggcggtgtc gagcctagac tgcctgtcca gcatcgtgga gcgcatctcc 780

accgagagcc ctgcggcgcc cgccctcctg ctggcggacg tgccttctga gtcgcctccg 840

cgcaggcaag aggctgccgc ccccagcgag ggagagagca gcggcgaccc cacccagtca 900

ccggacgccg ccccgcagtg ccctgcgggt gcgaacccca acccgatata ccaggtgctc 960

tga 963

<210> 17
<211> 3921
<212> DNA
<213> Human

<400> 17
atgggggccg cctcgggccg ccgggggccg gggctgctgc tgccgctgcc gctgctgttg 60

ctgctgccgc cgcagcccgc cctggcgttg gaccccgggc tgcagcccgg caacttttct 120

gctgacgagg ccggggcgca gctcttcgcg cagagctaca actccagcgc cgaacaggtg 180

ctgttccaga gcgtggccgc cagctgggcg cacgacacca acatcaccgc ggagaatgca 240

aggcgccagg aggaagcagc cctgctcagc caggagtttg cggaggcctg gggccagaag 300

gccaaggagc tgtatgaacc gatctggcag aacttcacgg acccgcagct gcgcaggatc 360

atcggagctg tgcgcaccct gggctctgcc aacctgcccc tggctaagcg gcagcagtac 420

aacgccctgc taagcaacat gagcaggatc tactccaccg ccaaggtctg cctccccaac 480

aagactgcca cctgctggtc cctggaccca gatctcacca acatcctggc ttcctcgcga 540

agctacgcca tgctcctgtt tgcctgggag ggctggcaca acgctgcggg catcccgctg 600

aaaccgctgt acgaggattt cactgccctc agcaatgaag cctacaagca ggacggcttc 660

acagacacgg gggcctactg gcgctcctgg tacaactccc ccaccttcga ggacgatctg 720

gaacacctct accaacagct agagcccctc tacctgaacc tccatgcctt cgtccgccgc 780

gcactgcatc gccgatacgg agacagatac atcaacctca ggggacccat ccctgctcat 840

ctgctgggag acatgtgggc ccagagctgg gaaaacatct acgacatggt ggtgcctttc 900

ccagacaagc ccaacctcga tgtcaccagt actatgctgc agcagggctg gaacgccacg 960

cacatgttcc gggtggcaga ggagttcttc acctccctgg agctctcccc catgcctccc 1020

gagttctggg aagggtcgat gctggagaag ccggccgacg ggcgggaagt ggtgtgccac 1080

gcctcggctt gggacttcta caacaggaaa gacttcagga tcaagcagtg cacacgggtc 1140

acgatggacc agctctccac agtgcaccat gagatgggcc atatacagta ctacctgcag 1200

tacaaggatc tgcccgtctc cctgcgtcgg ggggccaacc ccggcttcca tgaggccatt 1260

ggggacgtgc tggcgctctc ggtctccact cctgaacatc tgcacaaaat cggcctgctg 1320

gaccgtgtca ccaatgacac ggaaagtgac atcaattact tgctaaaaat ggcactggaa 1380

aaaattgcct tcctgccctt tggctacttg gtggaccagt ggcgctgggg ggtctttagt 1440

gggcgtaccc ccccttcccg ctacaacttc gactggtggt atcttcgaac caagtatcag 1500

gggatctgtc ctcctgttac ccgaaacgaa acccactttg atgctggagc taagtttcat 1560

gttccaaatg tgacaccata catcaggtac tttgtgagtt ttgtcctgca gttccagttc 1620

catgaagccc tgtgcaagga ggcaggctat gagggcccac tgcaccagtg tgacatctac 1680

cggtccacca aggcaggggc caagctccgg aaggtgctgc aggctggctc ctccaggccc 1740

tggcaggagg tgctgaagga catggtcggc ttagatgccc tggatgccca gccgctgctc 1800

aagtacttcc agccagtcac ccagtggctg caggagcaga accagcagaa cggcgaggtc 1860

ctgggctggc ccgagtacca gtggcacccg ccgttgcctg acaactaccc ggagggcata 1920

gacctggtga ctgatgaggc tgaggccagc aagtttgtgg aggaatatga ccggacatcc 1980

caggtggtgt ggaacgagta tgccgaggcc aactggaact acaacaccaa catcaccaca 2040

gagaccagca agattctgct gcagaagaac atgcaaatag ccaaccacac cctgaagtac 2100

ggcacccagg ccaggaagtt tgatgtgaac cagttgcaga acaccactat caagcggatc 2160

ataaagaagg ttcaggacct agaacgggca gcactgcctg cccaggagct ggaggagtac 2220

aacaagatcc tgttggatat ggaaaccacc tacagcgtgg ccactgtgtg ccacccgaat 2280

ggcagctgcc tgcagctcga gccagatctg acgaatgtga tggccacgtc ccggaaatat 2340

gaagacctgt tatgggcatg ggagggctgg cgagacaagg cggggagagc catcctccag 2400

ttttacccga aatacgtgga actcatcaac caggctgccc ggctcaatgg ctatgtagat 2460

gcaggggact cgtggaggtc tatgtacgag acaccatccc tggagcaaga cctggagcgg 2520

ctcttccagg agctgcagcc actctacctc aacctgcatg cctacgtgcg ccgggccctg 2580

caccgtcact acgggcccca gcacatcaac ctggaggggc ccattcctgc tcacctgctg 2640

gggaacatgt gggcgcagac ctggtccaac atctatgact tggtggtgcc cttcccttca 2700

gcccccctcga tggacaccac agaggctatg ctaaagcagg gctggacgcc caggaggatg 2760

tttaaggagg ctgatgattt cttcacctcc ctggggctgc tgcccgtgcc tcctgagttc 2820

tggaacaagt cgatgctgga gaagccaacc gacgggcggg aggtggtctg ccacgcctcg 2880

gcctgggact tctacaacgg caaggacttc cggatcaagc agtgcaccac cgtgaacttg 2940

gaggacctgg tggtggccca ccacgaaatg ggccacatcc agtatttcat gcagtacaaa 3000

gacttacctg tggccttgag ggagggtgcc aaccccggct tccatgaggc cattggggac 3060

gtgctagccc tctcagtgtc tacgcccaag cacctgcaca gtctcaacct gctgagcagt 3120

gagggtggca gcgacgagca tgacatcaac tttctgatga agatggccct tgacaagatc 3180

gcctttatcc ccttcagcta cctcgtcgat cagtggcgct ggagggtatt tgatggaagc 3240

atcaccaagg agaactataa ccaggagtgg tggagcctca ggctgaagta ccagggcctc 3300

tgccccccag tgcccaggac tcaaggtgac tttgacccag gggccaagtt ccacattcct 3360

tctagcgtgc cttacatcag gtactttgtc agcttcatca tccagttcca gttccacgag 3420

gcactgtgcc aggcagctgg ccacacgggc cccctgcaca agtgtgacat ctaccagtcc 3480

aaggaggccg ggcagcgcct ggcgaccgcc atgaagctgg gcttcagtag gccgtggccg 3540

gaagccatgc agctgatcac gggccagccc aacatgagcg cctcggccat gttgagctac 3600

ttcaagccgc tgctggactg gctccgcacg gagaacgagc tgcatgggga gaagctgggc 3660

tggccgcagt acaactggac gccgaactcc gctcgctcag aagggcccct cccagacagc 3720

ggccgcgtca gcttcctggg cctggacctg gatgcgcagc aggcccgcgt gggccagtgg 3780

ctgctgctct tcctgggcat cgccctgctg gtagccaccc tgggcctcag ccagcggctc 3840

ttcagcatcc gccaccgcag cctccaccgg cactcccacg ggccccagtt cggctccgag 3900

gtggagctga gacactcctg a 3921

<210> 18
<211> 2397
<212> DNA
<213> Human

<400> 18
atggcgtggg acatgtgcaa ccaggactct gagtctgtat ggagtgacat cgagtgtgct 60

gctctggttg gtgaagacca gcctctttgc ccagatcttc ctgaacttga tctttctgaa 120

ctagatgtga acgacttgga tacagacagc tttctgggtg gactcaagtg gtgcagtgac 180

caatcagaaa taatatccaa tcagtacaac aatgagcctt caaacatatt tgagaagata 240

gatgaagaga atgaggcaaa cttgctagca gtcctcacag agacactaga cagtctccct 300

gtggatgaag acggattgcc ctcatttgat gcgctgacag atggagacgt gaccactgac 360

aatgaggcta gtccttcctc catgcctgac ggcacccctc caccccagga ggcagaagag 420

ccgtctctac ttaagaagct cttactggca ccagccaaca ctcagctaag ttataatgaa 480

tgcagtggtc tcagtaccca gaaccatgca aatcacaatc acaggatcag aacaaaccct 540

gcaattgtta agactgagaa ttcatggagc aataaagcga agagtatttg tcaacagcaa 600

aagccacaaa gacgtccctg ctcggagctt ctcaaatatc tgaccacaaa cgatgaccct 660

cctcacacca aacccacaga gaacagaaac agcagcagag acaaatgcac ctccaaaaag 720

aagtcccaca cacagtcgca gtcacaacac ttacaagcca aaccaacaac tttatctctt 780

cctctgaccc cagagtcacc aaatgacccc aagggttccc catttgagaa caagactatt 840
gaacgcacct taagtgtgga actctctgga actgcaggcc taactccacc caccactcct 900
cctcataaag ccaaccaaga taacccttt agggcttctc caaagctgaa gtcctcttgc 960
aagactgtgg tgccaccacc atcaaagaag cccaggtaca gtgagtcttc tggtacacaa 1020
ggcaataact ccaccaagaa agggccggag caatccgagt tgtatgcaca actcagcaag 1080
tcctcagtcc tcactggtgg acacgaggaa aggaagacca agcggcccag tctgcggctg 1140
tttggtgacc atgactattg ccagtcaatt aattccaaaa cagaaatact cattaatata 1200
tcacaggagc tccaagactc tagacaacta gaaaataaag atgtctcctc tgattggcag 1260
gggcagattt gttcttccac agattcagac cagtgctacc tgagagagac tttggaggca 1320
agcaagcagg tctctccttg cagcacaaga aaacagctcc aagaccagga aatccgagcc 1380
gagctgaaca agcacttcgg tcatcccagt caagctgttt ttgacgacga agcagacaag 1440
accggtgaac tgagggacag tgatttcagt aatgaacaat tctccaaact acctatgttt 1500
ataaattcag gactagccat ggatggcctg tttgatgaca gcgaagatga aagtgataaa 1560
ctgagctacc cttgggatgg cacgcaatcc tattcattgt tcaatgtgtc tccttcttgt 1620
tcttctttta actctccatg tagagattct gtgtcaccac ccaaatcctt attttctcaa 1680
agaccccaaa ggatgcgctc tcgttcaagg tccttttctc gacacaggtc gtgttcccga 1740
tcaccatatt ccaggtcaag atcaaggtct ccaggcagta gatcctcttc aagatcctgc 1800
tattactatg agtcaagcca ctacagacac cgcacgcacc gaaattctcc cttgtatgtg 1860
agatcacgtt caagatcgcc ctacagccgt cggcccaggt atgacagcta cgaggaatat 1920
cagcacgaga ggctgaagag ggaagaatat cgcagagagt atgagaagcg agagtctgag 1980
agggccaagc aaagggagag gcagaggcag aaggcaattg aagagcgccg tgtgatttat 2040
gtcggtaaaa tcagacctga cacaacacgg acagaactga gggaccgttt tgaagttttt 2100
ggtgaaattg aggagtgcac agtaaatctg cgggatgatg gagacagcta tggtttcatt 2160
acctaccgtt atacctgtga tgcttttgct gctcttgaaa atggatacac tttgcgcagg 2220
tcaaacgaaa ctgactttga gctgtacttt tgtggacgca agcaattttt caagtctaac 2280
tatgcagacc tagattcaaa ctcagatgac tttgaccctg cttccaccaa gagcaagtat 2340
gactctctgg attttgatag tttactgaaa gaagctcaga gaagcttgcg caggtaa 2397

<210> 19
<211> 900
<212> DNA
<213> Human

<400> 19
atggaacatt ttgatgcatc acttagtacc tatttcaagg cattgctagg ccctcgagat 60

actagagtaa aaggatggtt tcttctggac aattatatac ccacatttat ctgctctgtc 120

atatatttac taattgtatg gctgggacca aaatacatga ggaataaaca gccattctct 180

tgccgggggga ttttagtggt gtataacctt ggactcacac tgctgtctct gtatatgttc 240

tgtgagttag taacaggagt atgggaaggc aaatacaact tcttctgtca gggcacacgc 300

accgcaggag aatcagatat gaagattatc cgtgtcctct ggtggtacta cttctccaaa 360

ctcatagaat ttatggacac tttcttcttc atcctgcgca agaacaacca ccagatcacg 420

gtcctgcacg tctaccacca tgcctcgatg ctgaacatct ggtggtttgt gatgaactgg 480

gtcccctgcg gccactctta ttttggtgcc acacttaata gcttcatcca cgtcctcatg 540

tactcttact atggtttgtc gtcagtccct tccatgcgtc catacctctg gtggaagaag 600

tacatcactc aggggcagct gcttcagttt gtgctgacaa tcatccagac cagctgcggg 660

gtcatctggc cgtgcacatt ccctcttggt tggttgtatt tccagattgg atacatgatt 720

tccctgattg ctctcttcac aaacttctac attcagacct acaacaagaa aggggcctcc 780

cgaaggaaag accacctgaa ggaccaccag aatgggtcca tggctgctgt gaatggacac 840

accaacagct tttcacccct ggaaaacaat gtgaagccaa ggaagctgcg gaaggattga 900

<210> 20
<211> 1530
<212> DNA
<213> Human

<400> 20
atgccgtcgg gcttccaaca gataggctcc gaagatgggg aacccccctca gcagcgagtg 60

actgggaccc tggtccttgc tgtgttctct gcggtgcttg gctccctgca gtttgggtac 120

aacattgggg tcatcaatgc ccctcagaag gtgattgaac agagctacaa tgagacgtgg 180

ctggggaggc aggggcctga gggacccagc tccatccctc caggcaccct caccaccctc 240

tgggccctct ccgtggccat cttttccgtg ggcggcatga tttcctcctt cctcattggt 300

atcatctctc agtggcttgg aaggaaaagg gccatgctgg tcaacaatgt cctggcggtg 360

ctggggggca gcctcatggg cctggccaat gctgctgcct cctatgaaat gctcatcctt 420

ggacgattcc tcattggcgc ctactcaggg ctgacatcag ggctggtgcc catgtacgtg 480

ggggagattg ctcccactca cctgcggggc gccctgggga cgctcaacca actggccatt 540

gttatcggca ttctgatcgc ccaggtgctg ggcttggagt ccctcctggg cactgccagc 600

ctgtggccac tgctcctggg cctcacagtg ctacctgccc tcctgcagct ggtcctgctg 660

cccttctgtc ccgagagccc ccgctacctc tacatcatcc agaatctcga ggggcctgcc 720

agaaagagtc tgaagcgcct gacaggctgg gccgatgttt ctggagtgct ggctgagctg 780

aaggatgaga agcggaagct ggagcgtgag cggccactgt ccctgctcca gctcctgggc 840

```
agccgtaccc accggcagcc cctgatcatt gcggtcgtgc tgcagctgag ccagcagctc      900
tctggcatca atgctgtttt ctattattcg accagcatct tcgagacagc aggggtaggc      960
cagcctgcct atgccaccat aggagctggt gtggtcaaca cagtcttcac cttggtctcg     1020
gtgttgttgg tggagcgggc ggggcgccgg acgctccatc tcctgggcct ggcgggcatg     1080
tgtggctgtg ccatcctgat gactgtggct ctgctcctgc tggagcgagt tccagccatg     1140
agctacgtct ccattgtggc catctttggc ttcgtggcat tttttgagat tggccctggc     1200
cccattcctt ggttcatcgt ggccgagctc ttcagccagg gaccccgccc ggcagccatg     1260
gctgtggctg gtttctccaa ctggacgagc aacttcatca ttggcatggg tttccagtat     1320
gttgcggagg ctatggggcc ctacgtcttc cttctatttg cggtcctcct gctgggcttc     1380
ttcatcttca ccttcttaag agtacctgaa actcgaggcc ggacgtttga ccagatctca     1440
gctgccttcc accggacacc ctctctttta gagcaggagg tgaaacccag cacagaactt     1500
gagtatttag ggccagatga gaacgactga                                      1530
```

## Description

Subject matter of the present invention is a method of assessing the circadian rhythm of a subject and/or assessing and predicting the athletic performance of said subject, wherein said method comprises the steps of:

- Providing at least three samples of saliva, more preferably four samples of saliva, from said subject, wherein said samples have been taken at different time points over the day,
- Determining gene expression of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, ROBC, in particular ARNTL (BMAL1) and PER2, in each of said samples, and
- Assessing and predicting by means of a computational step based on said expression levels of ARNTL (BMAL1) and PER2 over the day the circadian rhythm of said subject and/or the individual diurnal athletic performance times.

## State of the art

The biological clock (also known as circadian clock) regulates several aspects of physiology and behavior via cellular and molecular mechanisms and plays a vital role in maintaining proper human health. This is no wonder, since about half of all human genes are rhythmically expressed in at least one tissue. The disruption of circadian rhythms is associated to several diseases including sleep disorders, depression, diabetes, Alzheimer's disease, obesity and cancer. This disruption might result from conflicting external (environmental) or internal (feeding/resting) signals that are not in synchrony with the internal biological time. This not only affects shift workers, but also most people subjected to societal routine (social jet lag). Furthermore, a desynchronized circadian clock was shown to negatively affect an individual's wellbeing, in particular in the context of metabolism, as well as physical and mental (cognitive) performance.

Recent studies have reported a role for clock dysregulation in the pathologies mentioned above, raising increased awareness from scientists, clinicians and the public. Thus, it is crucial to characterize the individual's internal clock and to adjust the external/internal factors, to avoid or to overcome circadian rhythm disruption. Moreover, the time for certain activities, such as sleep, sports or medicine intake, can be optimized based on the individual's internal timing. A proper functioning circadian clock, synchronized with the individual's behavioral habits, will improve fitness and reduce therapy/recovery time in patients.

Yet, the available methods for clock assessment are either not very accurate or mostly invasive and often require medical assistance over a period of several hours (tedious, time-consuming, and cost-intensive). So far, there are the following methods among the molecular approaches to determine the biological clock in humans:

1. Determination of time-point at which endogenous melatonin (or cortisol), reaches a predefined threshold value concentration (dim-light-melatonin-onset, aka DLMO). To do this, several blood or saliva samples (usually every 0.5-1 hour) under dim-light conditions are taken to determine the internal clock.
2. Blood samples (with one or more sampling times) are also used to identify biomarkers using a machine learning approach that could determine the expression phase of "time-indicating" genes and thus the internal clock timing.
3. In addition, the circadian phase (peak time of secretion/expression) in hair samples (hair follicle cells) or urine samples could reflect that of the individual behavioral rhythm. This strategy is more suitable for assessing the human peripheral clock.

As mentioned above, current methods of clock assessment are either invasive, require medical supervision, are time-consuming or do not provide detailed information on the gene expression level. Saliva plays numerous protective roles for oral tissue maintenance in humans. Adequate salivary flow and saliva content are directly related to health status. Previous studies have shown the potential of saliva and salivary transcriptome as a diagnostic tool, which underlines the importance of saliva sampling as a non-invasive diagnostic method. Time-course saliva sampling is also commonly used for estimating the evening dim-light melatonin onset (DLMO) in humans in order to determine their circadian phase (peak time of secretion/expression); a method that requires controlled dim-light conditions during the entire sampling time of 5-6h.

The circadian rhythm was previously modeled with different approaches, starting with models that simply show oscillations such as phase-oscillators, and going up to molecular models, which model (part of) the molecular interactions underlying the circadian clock. In the present disclosure it is focused on molecular models, because these contain biological information that might be useful for predictions. Molecular models with simple feedback loops are often based on Goodwin's oscillator, e.g. (Ruoff and Rensing 1996), but the level of detail may also be extensive (Forger and Peskin 2003).

An objective is to provide a model at an intermediate state of complexity, complex enough to capture a significant part of the genetic network, but if the model is too complex, we cannot fit our data to the model without significant overfitting. Relogio et al. have published a model at this level of complexity, with 19 dynamical variables, which we use in the following (Relógio et al. 2011).

Other studies with mammalian clock models are named below, including respective literal citations from the respective papers.

(Becker-Weimann et al. 2004): "We present a mathematical model that reflects the essential features of the mammalian circadian oscillator to characterize the differential roles of negative and positive feedback loops. The oscillations that are obtained have a 24-h period and are robust toward parameter variations even when the positive feedback is replaced by a constantly expressed activator. This demonstrates the crucial role of the negative feedback for rhythm generation." [7 dynamical variables]

(Forger and Peskin 2003): "Here we develop a detailed distinctly mammalian model by using mass action kinetics. Parameters for our model are found from experimental data by using a coordinate search method. The model accurately predicts the phase of entrainment, amplitude of oscillation, and shape of time profiles of clock mRNAs and proteins and is also robust to parameter changes and mutations." [74 dynamical variables] There also is a stochastic version of this model (Forger and Peskin 2005).

(Leloup and Goldbeter 2003): "We present a computational model for the mammalian circadian clock based on the intertwined positive and negative regulatory loops involving the Per, Cry, Arntl (Bmal1), Clock, and Rev-Erb $\alpha$ genes. In agreement with experimental observations, the model can give rise to sustained circadian oscillations in continuous darkness, characterized by an antiphase relationship between Per/Cry/Rev-Erb$\alpha$ and Arntl (Bmal1) mRNAs. Sustained oscillations correspond to the rhythms autonomously generated by suprachiasmatic nuclei. For other parameter values, damped oscillations can also be obtained in the model. These oscillations, which transform into sustained oscillations when coupled to a periodic signal, correspond to rhythms produced by peripheral tissues." [19 dynamical variables] Bifurcation analysis of this model published in (Leloup and Goldbeter 2004).

(Mirsky et al. 2009): "In this study, we built a mathematical model from the regulatory structure of the intracellular circadian clock in mice and identified its parameters using an iterative evolutionary strategy, with minimum cost achieved through conformance to phase separations seen in cell-autonomous oscillators. The model was evaluated against the experimentally observed cell-autonomous circadian phenotypes of gene knockouts, particularly retention of rhythmicity and changes in expression level of molecular clock components." "Most importantly, our model addresses the overlapping but differential functions of CRY1 and CRY2 in the clock mechanism: They antagonistically regulate period length and differentially control rhythm persistence and amplitude" [21 dynamical variables] This model focuses on the phase relationship between different genes.

(Kim and Forger 2012): "To understand the biochemical mechanisms of this timekeeping, we have developed a detailed mathematical model of the mammalian circadian clock. Our model can accurately predict diverse experimental data including the phenotypes of mutations or knockdown of clock genes as well as the time courses and relative expression of clock transcripts and proteins. Using this model, we show how a universal motif of circadian timekeeping, where repressors tightly bind activators rather than directly binding to DNA, can generate oscillations when activators and repressors are in stoichiometric balance." [Ref: Kim, Jae Kyoung, and Daniel B Forger. 2012. "A Mechanism for Robust Circadian Timekeeping via Stoichiometric Balance." Molecular Systems Biology 8 (December): 630. https://doi.org/10.1038/msb.2012.62.]

(Jolley et al. 2014): Focus on a new mechanism via D-box, and modern parameter estimation.

Besides these models of the mammalian circadian clock, various models have been published for non-mammalian systems, and intensively investigated, e.g. by bifurcation analysis.

## Summary of the invention

The role of the circadian clock in the daily fluctuations of sports performance in healthy individuals has been explored. Molecular (gene expression) and physiological (biomechanical muscle properties) features in humans have been measured, and the athletic performance of healthy individuals at different times of the day was recorded based on strength and endurance tests. The data shows circadian variations in gene expression, sports performance and muscle properties, and a correlation between the sports performance and the molecular and physiological data has been found. Computational/machine learning approaches using accessible human biological material in time series studies have been applied.

Establishing human saliva as the biological source of material, core-clock gene expression (e.g. ARNTL (BMAL1) and PER2) has been analyzed over time and compared to biomechanical muscle properties and athletic performance. In particular, it has been shown that e.g. ARNTL (BMAL1) and PER2 expression display distinctive daily fluctuations in saliva samples, which correlate to the oscillation amplitude and peak time of athletic performance during the day.

In addition to the core-clock genes the expression of the clock- and sports-related gene AKT1, a serine-threonine protein kinase, involved in metabolism and the response to aerobic exercise has been measured and it was shown that fluctuations of AKT1 expression during the day for all participants tested have significant correlation to the temporal profile of ARNTL (BMAL1), which hints towards the circadian regulation of exercise and athletic performance throughout the day.

According to the present invention personalized predictions of athletic performance have been enabled. The inventors' study revealed that the variation in the expression of the core-clock genes ARNTL (BMAL1) and PER2 across the day, their ratio of expression (e.g. ARNTL (BMAL1) over PER2), and their average expression can be used as predictors for individual optimal sports performance time.

In one embodiment, with regard to PER2 the peak time of expression is used for the computational steps. In one embodiment, with regard to ARNTL (BMAL1) the overall difference in expression levels (between participants) is used for the computational steps.

**Detailed Description of the Invention**

Subject matter of the present invention is a method of assessing the circadian rhythm of a subject and/or assessing and predicting the athletic performance of said subject, wherein said method comprises the steps of:

- Providing at least three samples of saliva, more preferably four samples of saliva, from said subject, wherein said samples have been taken at different time points over the day,
- Determining gene expression of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, in particular of ARNTL (BMAL1) and PER2, in each of said samples, and
- Assessing and predicting by means of a computational step based on said expression levels of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, over the day the circadian rhythm of said subject and/or the individual diurnal athletic performance times.

Subject matter of the present invention is a method of assessing the circadian rhythm of a subject and/or assessing and predicting the athletic performance of said subject, wherein said method comprises the steps of:

- Providing at least three samples of saliva, more preferably four samples of saliva, from said subject, wherein said samples have been taken at different time points over the day,
- Determining gene expression of ARNTL (BMAL1) and PER2 in each of said samples, and
- Assessing and predicting by means of a computational step based on said expression levels of ARNTL (BMAL1) and PER2 over the day the circadian rhythm of said subject and/or the individual diurnal athletic performance times.

In one embodiment of the invention gene expression is determined using a method selected from quantitative PCR (RT-qPCR), NanoString, sequencing and microarray. Any other method for determining gene expression may be used.

In one embodiment of the invention gene expression is determined using quantitative PCR (RT-qPCR).

In one embodiment of the invention gene expression is determined using NanoString, see e.g. Geiss G, et al., Direct multiplexed measurement of gene expression with color-coded probe pairs, 26: 317-25 (2008)., Nature Biotechnology, Feb 08, 2008.

BMALlis also known as ARNTL, Aryl hydrocarbon receptor nuclear translocator-like protein 1 (ARNTL) Or Brain and Muscle ARNT-Like 1 (BMAL1)

The sequence of cDNA ARNTL (BMAL1) comprises SEQ ID No. 1:
>ENST00000389707.8 ARNTL-201 cdna:protein_coding

ATGGCAGACCAGAGAATGGACATTTCTTCAACCATCAGTGATTTCATGTCCCCGGGCCCC
ACCGACCTGCTTTCCAGCTCTCTTGGTACCAGTGGTGTGGATTGCAACCGCAAACGGAAA
GGCAGCTCCACTGACTACCAAGAAAGCATGGACACAGACAAAGATGACCCTCATGGAAGG
TTAGAATATACAGAACACCAAGGAAGGATAAAAAATGCAAGGGAAGCTCACAGTCAGATT
GAAAAGCGGCGTCGGGATAAAATGAACAGTTTTATAGATGAATTGGCTTCTTTGGTACCA
ACATGCAACGCAATGTCCAGGAAATTAGATAAACTTACTGTGCTAAGGATGGCTGTTCAG
CACATGAAAACATTAAGAGGTGCCACCAATCCATACACAGAAGCAAACTACAAACCAACT
TTTCTATCAGACGATGAATTGAAACACCTCATTCTCAGGGCAGCAGATGGATTTTTGTTT
GTCGTAGGATGTGACCGAGGGAAGATACTCTTTGTCTCAGAGTCTGTCTTCAAGATCCTC
AACTACAGCCAGAATGATCTGATTGGTCAGAGTTTGTTTGACTACCTGCATCCTAAAGAT
ATTGCCAAAGTCAAGGAGCAGCTCTCCTCCTCTGACACCGCACCCCGGGAGCGGCTCATA
GATGCAAAAACTGGACTTCCAGTTAAAACAGATATAACCCCTGGGCCATCTCGATTATGT
TCTGGAGCACGACGTTCTTTCTTCTGTAGGATGAAGTGTAACAGGCCTTCAGTAAAGGTT
GAAGACAAGGACTTCCCCTCTACCTGCTCAAAGAAAAAAGATCGAAAAAGCTTCTGCACA
ATCCACAGCACAGGCTATTTGAAAAGCTGGCCACCCACAAAGATGGGGCTGGATGAAGAC
AACGAACCAGACAATGAGGGGTGTAACCTCAGCTGCCTCGTCGCAATTGGACGACTGCAT

TCTCATGTAGTTCCACAACCAGTGAACGGGGAAATCAGGGTGAAATCTATGGAATATGTT
TCTCGGCACGCGATAGATGGAAAGTTTGTTTTTGTAGACCAGAGGGCAACAGCTATTTTG
GCATATTTACCACAAGAACTTCTAGGCACATCGTGTTATGAATATTTTCACCAAGATGAC
ATAGGACATCTTGCAGAATGTCATAGGCAAGTTTTACAGACGAGAGAAAAAATTACAACT
AATTGCTATAAATTTAAAATCAAAGATGGTTCTTTTATCACACTACGGAGTCGATGGTTC
AGTTTCATGAACCCTTGGACCAAGGAAGTAGAATATATTGTCTCAACTAACACTGTTGTT
TTAGCCAACGTCCTGGAAGGCGGGGACCCAACCTTCCCACAGCTCACAGCATCCCCCCAC
AGCATGGACAGCATGCTGCCCTCTGGAGAAGGTGGCCCAAAGAGGACCCACCCCACTGTT
CCAGGGATTCCAGGGGGAACCCGGGCTGGGGCAGGAAAAATAGGCCGAATGATTGCTGAG
GAAATCATGGAAATCCACAGGATAAGAGGGTCATCGCCTTCTAGCTGTGGCTCCAGCCCA
TTGAACATCACGAGTACGCCTCCCCCTGATGCCTCTTCTCCAGGAGGCAAGAAGATTTTA
AATGGAGGGACTCCAGACATTCCTTCCAGTGGCCTACTATCAGGCCAGGCTCAGGAGAAC
CCAGGTTATCCATATTCTGATAGTTCTTCTATTCTTGGTGAGAACCCCCACATAGGTATA
GACATGATTGACAACGACCAAGGATCAAGTAGTCCCAGTAATGATGAGGCAGCAATGGCT
GTCATCATGAGCCTCTTGGAAGCAGATGCTGGACTGGGTGGCCCTGTTGACTTTAGTGAC
TTGCCATGGCCGCTGTAA

The sequence of cDNA ARNTL2 comprises SEQ ID No.2
>ENST00000266503.9 ARNTL2-202 cdna:protein_coding

ATGGCGGCGGAAGAGGAGGCTGCGGCGGGAGGTAAAGTGTTGAGAGAGGAGAACCAGTGC
ATTGCTCCTGTGGTTTCCAGCCGCGTGAGTCCAGGGACAAGACCAACAGCTATGGGGTCT
TTCAGCTCACACATGACAGAGTTTCCACGAAAACGCAAAGGAAGTGATTCAGACCCATCC
CAGTCAGGAATCATGACAGAAAAAGTGGTGGAAAAGCTTTCTCAGAATCCCCTTACCTAT
CTTCTTTCAACAAGGATAGAAATATCAGCCTCCAGTGGCAGCAGAGTGGAAGATGGTGAA
CACCAAGTTAAAATGAAGGCCTTCAGAGAAGCTCATAGCCAAACTGAAAAGCGGAGGAGA
GATAAAATGAATAACCTGATTGAAGAACTGTCTGCAATGATCCCTCAGTGCAACCCCATG
GCGCGTAAACTGGACAAACTTACAGTTTTAAGAATGGCTGTTCAACACTTGAGATCTTTA
AAAGGCTTGACAAATTCTTATGTGGGAAGTAATTATAGACCATCATTTCTTCAGGATAAT
GAGCTCAGACATTTAATCCTTAAGACTGCAGAAGGCTTCTTATTTGTGGTTGGATGTGAA
AGAGGAAAAATTCTCTTCGTTTCTAAGTCAGTCTCCAAAATACTTAATTATGATCAGGCT
AGTTTGACTGGACAAAGCTTATTTGACTTCTTACATCCAAAAGATGTTGCCAAAGTAAAG
GAACAACTTTCTTCTTTTGATATTTCACCAAGAGAAAAGCTAATAGATGCCAAAACTGGT
TTGCAAGTTCACAGTAATCTCCACGCTGGAAGGACACGTGTGTATTCTGGCTCAAGACGA
TCTTTTTTCTGTCGGATAAAGAGTTGTAAAATCTCTGTCAAAGAAGAGCATGGATGCTTA
CCCAACTCAAAGAAGAAAGAGCACAGAAAATTCTATACTATCCATTGCACTGGTTACTTG
AGAAGCTGGCCTCCAAATATTGTTGGAATGGAAGAAGAAAGGAACAGTAAGAAAGACAAC
AGTAATTTTACCTGCCTTGTGGCCATTGGAAGATTACAGCCATATATTGTTCCACAGAAC
AGTGGAGAGATTAATGTGAAACCAACTGAATTTATAACCCGGTTTGCAGTGAATGGAAAA
TTTGTCTATGTAGATCAAAGGGCAACAGCGATTTTAGGATATCTGCCTCAGGAACTTTTG
GGAACTTCTTGTTATGAATATTTTCATCAAGATGACCACAATAATTTGACTGACAAGCAC
AAAGCAGTTCTACAGAGTAAGGAGAAAATACTTACAGATTCCTACAAATTCAGAGCAAAA
GATGGCTCTTTTGTAACTTTAAAAAGCCAATGGTTTAGTTTCACAAATCCTTGGACAAAA
GAACTGGAATATATTGTATCTGTCAACACTTTAGTTTTGGGACATAGTGAGCCTGGAGAA

GCATCATTTTTACCTTGTAGCTCTCAATCATCAGAAGAATCCTCTAGACAGTCCTGTATG
AGTGTACCTGGAATGTCTACTGGAACAGTACTTGGTGCTGGTAGTATTGGAACAGATATT
GCAAATGAAATTCTGGATTTACAGAGGTTACAGTCTTCTTCATACCTTGATGATTCGAGT
CCAACAGGTTTAATGAAAGATACTCATACTGTAAACTGCAGGAGTATGTCAAATAAGGAG
TTGTTTCCACCAAGTCCTTCTGAAATGGGGGAGCTAGAGGCTACCAGGCAAAACCAGAGT
ACTGTTGCTGTCCACAGCCATGAGCCACTCCTCAGTGATGGTGCACAGTTGGATTTCGAT
GCCCTATGTGACAATGATGACACAGCCATGGCTGCATTTATGAATTACTTAGAAGCAGAG
GGGGGCCTGGGAGACCCTGGGGACTTCAGTGACATCCAGTGGACCCTCTAG

The sequence of cDNA PER1 comprises SEQ ID No.3
>ENST00000317276.9 PER1-201 cdna:protein_coding

ATGAGTGGCCCCCTAGAAGGGGCTGATGGGGGAGGGGACCCCAGGCCTGGGGAATCATT
TGTCCTGGGGGCGTCCCATCCCCTGGGCCCCCACAGCACCGGCCTTGCCCAGGCCCCAGC
CTGGCCGATGACACCGATGCCAACAGCAATGGTTCAAGTGGCAATGAGTCCAACGGGCAT
GAGTCTAGAGGCGCATCTCAGCGGAGCTCACACAGCTCCTCCTCAGGCAACGGCAAGGAC
TCAGCCCTGCTGGAGACCACTGAGAGCAGCAAGAGCACAAACTCTCAGAGCCCATCCCCA
CCCAGCAGTTCCATTGCCTACAGCCTCCTGAGTGCCAGCTCAGAGCAGGACAACCCGTCC
ACCAGTGGCTGCAGCAGTGAACAGTCAGCCCGGGCAAGGACTCAGAAGGAACTCATGACA
GCACTTCGAGAGCTCAAGCTTCGACTGCCGCCAGAGCGCCGGGGCAAGGGCCGCTCTGGG
ACCCTGGCCACGCTGCAGTACGCACTGGCCTGTGTCAAGCAGGTGCAGGCCAACCAGGAA
TACTACCAGCAGTGGAGCCTGGAGGAGGGCGAGCCTTGCTCCATGGACATGTCCACCTAT
ACCCTGGAGGAGCTGGAGCACATCACGTCTGAGTACACACTTCAGAACCAGGATACCTTC
TCAGTGGCTGTCTCCTTCCTGACGGGCCGAATCGTCTACATTTCGGAGCAGGCAGCCGTC
CTGCTGCGTTGCAAGCGGGACGTGTTCCGGGGTACCCGCTTCTCTGAGCTCCTGGCTCCC
CAGGATGTGGGAGTCTTCTATGGTTCCACTGCTCCATCTCGCCTGCCCACCTGGGGCACA
GGGGCCTCAGCAGGTTCAGGCCTCAGGGACTTTACCCAGGAGAAGTCCGTCTTCTGCCGT
ATCAGAGGAGGTCCTGACCGGGATCCAGGGCCTCGGTACCAGCCATTCCGCCTAACCCCG
TATGTGACCAAGATCCGGGTCTCAGATGGGGCCCCTGCACAGCCGTGCTGCCTGCTGATT
GCAGAGCGCATCCATTCGGGTTACGAAGCTCCCCGGATACCCCCTGACAAGAGGATTTTC
ACTACGCGGCACACACCCAGCTGCCTCTTCCAGGATGTGGATGAAAGGGCTGCCCCCCTG
CTGGGCTACCTGCCCCAGGACCTCCTGGGGGCCCCAGTGCTCCTGTTCCTGCATCCTGAG
GACCGACCCCTCATGCTGGCTATCCACAAGAAGATTCTGCAGTTGGCGGGCCAGCCCTTT
GACCACTCCCCTATCCGCTTCTGTGCCCGCAACGGGGAGTATGTCACCATGGACACCAGC
TGGGCTGGCTTTGTGCACCCCTGGAGCCGCAAGGTAGCCTTCGTGTTGGGCCGCCACAAA
GTACGCACGGCCCCCCTGAATGAGGACGTGTTCACTCCCCCGGCCCCCAGCCCAGCTCCC
TCCCTGGACACTGATATCCAGGAGCTGTCAGAGCAGATCCACCGGCTGCTGCTGCAGCCC
GTCCACAGCCCCAGCCCCACGGGACTCTGTGGAGTCGGCGCCGTGACATCCCCAGGCCCT
CTCCACAGCCCTGGGTCCTCCAGTGATAGCAACGGGGGTGATGCAGAGGGGCCTGGGCCT
CCTGCGCCAGTGACTTTCCAGCAGATCTGTAAGGATGTGCATCTGGTGAAGCACCAGGGC
CAGCAGCTTTTTATTGAGTCTCGGGCCCGGCCTCAGTCCCGGCCCCGCCTCCCTGCTACA
GGCACGTTCAAGGCCAAGGCCCTTCCCTGCCAATCCCCAGACCCAGAGCTGGAGGCGGGT
TCTGCTCCCGTCCAGGCCCCACTAGCCTTGGTCCCTGAGGAGGCCGAGAGGAAAGAAGCC

TCCAGCTGCTCCTACCAGCAGATCAACTGCCTGGACAGCATCCTCAGGTACCTGGAGAGC
TGCAACCTCCCCAGCACCACTAAGCGTAAATGTGCCTCCTCCTCCTCCTATACCACCTCC
TCAGCCTCTGACGACGACAGGCAGAGGACAGGTCCAGTCTCTGTGGGGACCAAGAAAGAT
CCGCCGTCAGCAGCGCTGTCTGGGGAGGGGGCCACCCCACGGAAGGAGCCAGTGGTGGGA
GGCACCCTGAGCCCGCTCGCCCTGGCCAATAAGGCGGAGAGTGTGGTGTCCGTCACCAGT
CAGTGTAGCTTCAGCTCCACCATCGTCCATGTGGGAGACAAGAAGCCCCCGGAGTCGGAC
ATCATCATGATGGAGGACCTGCCTGGCCTAGCCCCAGGCCCAGCCCCCAGCCCAGCCCCC
AGCCCCACAGTAGCCCCTGACCCAGCCCCAGACGCCTACCGTCCAGTGGGGCTGACCAAG
GCCGTGCTGTCCCTGCACACACAGAAGGAAGAGCAAGCCTTCCTCAGCCGCTTCCGAGAC
CTGGGCAGGCTGCGTGGACTCGACAGCTCTTCCACAGCTCCCTCAGCCCTTGGCGAGCGA
GGCTGCCACCACGGCCCCGCACCCCCAAGCCGCCGACACCACTGCCGATCCAAAGCCAAG
CGCTCACGCCACCACCAGAACCCTCGGGCTGAAGCGCCCTGCTATGTCTCACACCCCTCA
CCCGTGCCACCCTCCACCCCCTGGCCCACCCCACCAGCCACTACCCCCTTCCCAGCGGTT
GTCCAGCCCTACCCTCTCCCAGTGTTCTCTCCTCGAGGAGGCCCCCAGCCTCTTCCCCCT
GCTCCCACATCTGTGCCCCCAGCTGCTTTCCCCGCCCCTTTGGTGACCCCAATGGTGGCC
TTGGTGCTCCCTAACTATCTGTTCCCAACCCCATCCAGCTATCCTTATGGGGCACTCCAG
ACCCCTGCTGAAGGGCCTCCCACTCCTGCCTCGCACTCCCCTTCTCCATCCTTGCCCGCC
CTCGCCCCGAGTCCTCCTCACCGCCCGGACTCTCCACTGTTCAACTCGAGATGCAGCTCT
CCACTCCAGCTCAATCTGCTGCAGCTGGAGGAGCTCCCCCGTGCTGAGGGGGCTGCTGTT
GCAGGAGGCCCTGGGAGCAGTGCCGGGCCCCCACCTCCCAGTGCGGAGGCTGCTGAGCCA
GAGGCCAGACTGGCGGAGGTCACTGAGTCCTCCAATCAGGACGCACTTTCCGGCTCCAGT
GACCTGCTCGAACTTCTGCTGCAAGAGGACTCGCGCTCCGGCACAGGCTCCGCAGCCTCG
GGCTCCTTGGGCTCTGGCTTGGGCTCTGGGTCTGGTTCAGGCTCCCATGAAGGGGGCAGC
ACCTCAGCCAGCATCACTCGCAGCAGCCAGAGCAGCCACACAAGCAAATACTTTGGCAGC
ATCGACTCTTCCGAGGCTGAGGCTGGGGCTGCTCGGGGCGGGGCTGAGCCTGGGGACCAG
GTGATTAAGTACGTGCTCCAGGATCCCATTTGGCTGCTCATGGCCAATGCTGACCAGCGC
GTCATGATGACCTACCAGGTGCCCTCCAGGGACATGACCTCTGTGCTGAAGCAGGATCGG
GAGCGGCTCCGAGCCATGCAGAAGCAGCAGCCTCGGTTTTCTGAGGACCAGCGGCGGGAA
CTGGGTGCTGTGCACTCCTGGGTCCGGAAGGGCCAACTGCCTCGGGCTCTTGATGTGATG
GCCTGTGTGGACTGTGGGAGCAGCACCCAAGATCCTGGTCACCCTGATGACCCACTCTTC
TCAGAGCTGGATGGACTGGGGCTGGAGCCCATGGAAGAGGGTGGAGGCGAGCAGGGCAGC
AGCGGTGGCGGCAGTGGTGAGGGAGAGGGCTGCGAGGAGGCCCAAGGCGGGGCCAAGGCT
TCAAGCTCTCAGGACTTGGCTATGGAGGAGGAGGAAGAAGGCAGGAGCTCATCCAGTCCA
GCCTTACCTACAGCAGGAAACTGCACCAGCTAG

The sequence of PER2 cDNA comprises SEQ ID No.4:
>ENST00000254657.8 PER2-201 cdna:protein_coding

ATGAATGGATACGCGGAATTTCCGCCCAGCCCCAGTAACCCCACCAAGGAGCCCGTGGAG
CCCCAGCCCAGCCAGGTCCCACTGCAGGAAGATGTGGACATGAGCAGTGGCTCCAGTGGA
CATGAGACCAACGAAAACTGCTCCACGGGGCGGGACTCGCAGGGCAGTGACTGTGACGAC
AGTGGGAAGGAGCTGGGGATGCTGGTGGAGCCACCGGATGCCCGCCAGAGTCCAGATACC
TTTAGCCTGATGATGGCAAAATCTGAACACAACCCATCTACAAGTGGCTGCAGTAGCGAC
CAGTCTTCGAAAGTGGACACACACAAAGAACTGATAAAAACACTAAAGGAGCTGAAGGTC

CACCTCCCTGCAGACAAGAAGGCCAAGGGCAAGGCCAGTACGCTGGCCACCTTGAAGTAC GCCCTCAGGAGCGTGAAGCAGGTGAAAGCCAATGAAGAGTATTACCAGCTGCTGATGTCC AGCGAGGGTCACCCCTGTGGAGCAGACGTGCCCTCCTACACCGTGGAGGAGATGGAGAGC GTTACCTCTGAGCACATTGTGAAGAATGCCGATATGTTTGCGGTGGCCGTGTCCCTGGTG TCTGGGAAGATCCTGTACATCTCTGACCAGGTTGCATCCATATTTCACTGTAAAAGAGAT GCCTTCAGCGATGCCAAGTTTGTGGAGTTCCTGGCGCCTCACGATGTGGGCGTGTTCCAC AGTTTCACCTCCCCGTACAAGCTTCCCTTGTGGAGCATGTGCAGTGGAGCAGATTCTTTT ACTCAAGAATGCATGGAGGAGAAATCTTTCTTTTGCCGTGTCAGTGTCCGGAAAAGCCAC GAGAATGAAATCCGCTACCACCCCTTCCGCATGACGCCCTACCTGGTCAAGGTGCGGGAC CAACAAGGTGCTGAGAGTCAGCTTTGCTGCCTTCTGCTGGCAGAGAGAGTGCACTCTGGT TATGAAGCCCCTAGAATTCCTCCTGAAAAGAGAATTTTTACAACCACCCATACACCAAAT TGTTTGTTCCAGGATGTGGATGAAAGGGCGGTCCCTCTCCTGGGCTACCTACCTCAGGAC CTGATTGAAACCCCAGTGCTCGTGCAGCTCCACCCTAGTGACAGGCCCTTGATGCTGGCC ATCCACAAAAAGATCCTGCAGTCAGGCGGGCAGCCTTTCGACTATTCTCCCATTCGGTTT CGCGCCCGGAACGGAGAGTACATCACGTTGGACACCAGCTGGTCCAGCTTCATCAACCCA TGGAGCAGGAAAATCTCCTTCATCATTGGGAGGCACAAAGTCAGGGTGGGCCCTTTGAAT GAGGACGTGTTTGCAGCCCACCCCTGCACAGAGGAGAAGGCCCTGCACCCCAGCATTCAG GAGCTCACAGAGCAGATCCACCGGCTCCTGCTGCAGCCCGTCCCCCACAGCGGCTCCAGT GGCTACGGGAGTCTGGGCAGCAACGGGTCCCACGAGCACCTTATGAGCCAGACCTCCTCC AGCGACAGCAACGGCCATGAGGACTCACGCCGGAGGAGAGCCGAAATTTGTAAAAATGGT AACAAGACCAAAAATAGAAGTCATTATTCTCATGAATCTGGAGAACAAAAGAAAAAATCC GTTACAGAAATGCAAACTAATCCCCCAGCTGAGAAGAAAGCTGTCCCTGCCATGGAAAAG GACAGCCTGGGGGTCAGCTTCCCCGAGGAGTTGGCCTGCAAGAACCAGCCCACCTGCTCC TACCAGCAGATCAGCTGCTTGGACAGCGTCATCAGGTACTTGGAGAGCTGCAATGAGGCT GCCACCCTGAAGAGGAAATGCGAGTTCCCAGCAAACGTCCCAGCGCTAAGGTCCAGTGAT AAGCGGAAGGCCACAGTCAGCCCAGGGCCACACGCTGGAGAGGCAGAGCCGCCCTCCAGG GTGAACAGCCGCACGGGAGTAGGTACGCACCTGACCTCGCTGGCACTGCCGGGCAAGGCA GAGAGTGTGGCGTCGCTCACCAGCCAGTGCAGCTACAGCAGCACCATCGTCCATGTGGGA GACAAGAAGCCGCAGCCGGAGTTAGAGATGGTGGAAGATGCTGCGAGTGGGCCAGAATCC CTGGACTGCCTGGCGGGCCCTGCCCTGGCCTGTGGTCTCAGCCAAGAGAAGGAGCCCTTC AAGAAGCTGGGCCTCACCAAGGAGGTACTCGCTGCACACACACAGAAGGAGGAGCAGAGC TTCCTGCAGAAGTTCAAAGAAATAAGAAAACTCAGCATTTTCCAGTCCCACTGCCATTAC TACTTGCAAGAAAGATCCAAGGGGCAGCCAAGTGAACGAACTGCCCCTGGACTAAGAAAT ACTTCCGGAATAGATTCACCTTGGAAAAAAACAGGAAAGAACAGAAAATTGAAGTCCAAG CGGGTCAAACCTCGAGACTCATCTGAGAGCACCGGATCTGGGGGGCCCGTGTCCGCCCGG CCCCCGCTGGTGGGCTTGAACGCCACAGCCTGGTCACCCTCAGACACGTCCCAGTCCAGC TGCCCAGCCGTGCCCTTTCCCGCCCCAGTGCCAGCAGCTTATTCACTGCCCGTGTTTCCA GCGCCAGGGACTGTGGCAGCACCCCCGGCACCTCCCCACGCCAGCTTCACAGTGCCTGCT GTGCCCGTGGACCTCCAGCACCAGTTTGCAGTCCAGCCCCCACCTTTCCCTGCCCCTTTG GCGCCTGTCATGGCATTCATGCTACCCAGTTATTCCTTCCCCTCGGGGACCCCAAACCTG CCCCAGGCCTTCTTCCCCAGCCAGCCTCAGTTTCCGAGCCACCCCACACTCACATCCGAG ATGGCCTCTGCCTCACAGCCTGAGTTCCCCAGCCGGACCTCGATCCCCAGACAGCCATGT GCTTGTCCAGCCACCCGGGCCACCCCACCATCGGCCATGGGTAGGGCCTCCCCACCGCTC TTTCAGTCCCGCAGCAGCTCGCCCCTGCAGCTCAACCTGCTGCAGCTGGAGGAAGCCCCT

GAGGGTGGCACTGGAGCCATGGGGACCACAGGGGCCACAGAGACAGCAGCTGTAGGGGCG
GACTGCAAACCTGGCACTTCTCGGGACCAGCAGCCGAAGGCGCCTCTGACCCGTGATGAA
CCCTCAGACACACAGAACAGTGACGCCCTTTCCACGTCAAGCGGCCTCCTAAACCTCCTG
CTGAATGAGGACCTCTGCTCAGCCTCGGGCTCTGCTGCTTCGGAGTCTCTGGGCTCCGGC
TCACTGGGCTGCGACGCCTCCCCGAGTGGGGCAGGCAGTAGTGACACAAGTCATACCAGC
AAATATTTTGGAAGCATTGACTCCTCAGAGAATAATCACAAAGCAAAAATGAACACTGGT
ATGGAAGAAAGTGAGCATTTCATTAAGTGCGTCCTGCAGGATCCCATCTGGCTGCTGATG
GCAGATGCGGACAGCAGCGTCATGATGACGTACCAGCTGCCTTCCCGAAATTTAGAAGCG
GTTTTGAAGGAGGACAGAGAGAAGCTGAAGCTCCTACAGAAACTCCAGCCCAGGTTCACG
GAGAGTCAGAAGCAGGAGCTGCGCGAGGTCCACCAGTGGATGCAGACGGGCGGCCTGCCC
GCAGCCATCGACGTGGCAGAATGTGTTTACTGTGAAAACAAGGAAAAAGGTAATATTTGC
ATACCATATGAGGAAGATATTCCTTCTCTGGGACTCAGCGAAGTGTCGGACACCAAAGAA
GACGAAAATGGATCCCCCTTGAATCACAGGATCGAAGAGCAGACGTAA

The sequence of PER3 cDNA comprises SEQ ID No.5:
>ENST00000613533.4 PER3-208 cdna:protein_coding

ATGCCCCGCGGGGAAGCTCCTGGCCCCGGGAGACGGGGGGCTAAGGACGAGGCCCTGGGC
GAAGAATCGGGGGAGCGGTGGAGCCCCGAGTTCCATCTGCAGAGGAAATTGGCGGACAGC
AGCCACAGTGAACAGCAAGATCGAAACAGAGTTTCTGAAGAACTTATCATGGTTGTCCAA
GAAATGAAAAAATACTTCCCCTCGGAGAGACGCAATAAACCAAGCACTCTAGATGCCCTC
AACTATGCTCTCCGCTGTGTCCACAGCGTTCAAGCAAACAGTGAGTTTTTCCAGATTCTC
AGTCAGAATGGAGCACCTCAGGCAGATGTGAGCATGTACAGTCTTGAGGAGCTGGCCACT
ATCGCTTCAGAACACACTTCCAAAAACACAGATACCTTTGTGGCAGTATTTTCATTTCTG
TCTGGAAGGTTAGTGCACATTTCTGAACAGGCTGCTTTGATCCTGAATCGTAAGAAAGAT
GTCCTGGCGTCTTCTCACTTTGTTGACCTGCTTGCACCTCAAGACATGAGGGTATTCTAC
GCGCACACTGCCAGAGCTCAGCTTCCTTTCTGGAACAACTGGACCCAAAGAGCAGCTGCA
CGGTATGAATGTGCTCCGGTGAAACCTTTTTTCTGCAGGATCCGTGGAGGTGAAGACAGA
AAGCAAGAGAAGTGTCACTCCCCATTCCGGATCATCCCCTATCTGATTCATGTACATCAC
CCTGCCCAGCCAGAATTGGAATCGGAACCTTGCTGTCTCACTGTGGTTGAAAAGATTCAC
TCTGGTTATGAAGCTCCTCGGATCCCAGTGAATAAAAGAATCTTCACCACCACACACACC
CCAGGGTGTGTTTTTCTTGAAGTAGATGAAAAAGCAGTGCCTTTGCTGGGTTACCTACCT
CAGGACCTGATTGGAACATCGATCCTAAGCTACCTGCACCCTGAAGATCGTTCTCTGATG
GTTGCCATACACCAAAAAGTTTTGAAGTATGCAGGGCATCCTCCCTTTGAACATTCTCCC
ATTCGATTTTGTACTCAAAACGGAGACTACATCATACTGGATTCCAGTTGGTCCAGCTTT
GTGAATCCCTGGAGCCGGAAGATTTCTTTCATCATTGGTCGGCATAAAGTTCGAACGAGC
CCACTAAATGAGGATGTTTTTGCTACCAAAATTAAAAAGATGAACGATAATGACAAAGAC
ATAACAGAATTACAAGAACAAATTTACAAACTTCTCTTACAGCCAGTTCACGTGAGCGTG
TCCAGCGGCTACGGGAGCCTGGGGAGCAGCGGGTCGCAGGAGCAGCTTGTCAGCATCGCC
TCCTCCAGTGAGGCCAGTGGGCACCGTGTGGAGGAGACGAAGGCGGAGCAGATGACCTTG
CAGCAGGTCTATGCCAGTGTGAACAAAATTAAAAATCTGGGTCAGCAGCTCTACATTGAG
TCAATGACCAAATCATCATTCAAGCCAGTGACGGGGACACGCACAGAACCGAATGGTGGT
GGTGAGTCAGCGAATGGTGGTGGTGAATGTAAGACCTTTACTTCCTTCCACCAAACACTG
AAAAACAATAGTGTGTACACTGAGCCCTGTGAGGATTTGAGGAACGATGAGCACAGCCCA

TCCTATCAACAGATCAACTGTATCGACAGTGTCATCAGATACCTGAAGAGCTACAACATT
CCAGCTTTGAAAAGAAAGTGTATCTCCTGTACAAATACAACTTCTTCCTCCTCAGAAGAA
GACAAACAGAACCACAAGGCAGATGATGTCCAAGCCTTACAAGCTGGTTTGCAAATCCCA
GCCATACCTAAATCAGAAATGCCAACAAATGGACGGTCCATAGACACAGGAGGAGGAGCT
CCACAGATCCTGTCCACGGCGATGCTGAGCTTGGGGTCGGGCATAAGCCAATGCGGTTAC
AGCAGCACCATTGTCCATGTCCCACCCCCAGAGACAGCCAGGGATGCTACCCTCTTCTGT
GAGCCCTGGACCCTGAACATGCAGCCAGCCCCTTTGACCTCGGAAGAATTTAAACACGTG
GGGCTCACAGCGGCTGTTCTGTCAGCGCACACCCAGAAGGAAGAGCAGAATTATGTTGAT
AAATTCCGAGAAAAGATCCTGTCATCACCCTACAGCTCCTATCTTCAGCAAGAAAGCAGG
AGCAAAGCTAAATATTCATATTTTCAAGGAGATTCTACTTCCAAGCAGACGCGGTCGGCC
GGCTGCAGGAAAGGGAAGCACAAGCGGAAGAAGCTGCCGGAGCCGCCAGACAGCAGCAGC
TCGAACACCGGCTCTGGTCCCCGCAGGGGAGCGCATCAGAACGCACAGCCCTGCTGCCCC
TCCGCGGCCTCCTCTCCGCACACCTCGAGCCCGACCTTCCCACCTGCCGCCATGGTGCCC
AGCCAGGCCCCTTACCTCGTCCCAGCTTTTCCCCTCCCAGCCGCGACCTCACCCGGAAGA
GAATACGCAGCCCCCGGAACTGCACCGGAAGGCCTGCATGGGCTGCCCTTGTCCGAGGGC
TTGCAGCCTTACCCAGCTTTCCCTTTTCCTTACTTGGATACTTTTATGACCGTTTTCCTG
CCTGACCCCCCTGTCTGTCCTCTGTTGTCGCCATCGTTTTTGCCATGTCCATTCCTGGGG
GCGACAGCCTCTTCTGCGATATCACCCTCAATGTCGTCAGCAATGAGTCCAACTCTGGAC
CCACCCCCTTCAGTCACCAGCCAAAGGAGAGAGGAGGAAAAGTGGGAGGCACAAAGCGAG
GGGCACCCGTTCATTACTTCGAGAAGCAGCTCACCCTTGCAGTTAAACTTACTTCAGGAA
GAGATGCCCAGACCCTCTGAATCTCCAGATCAGATGAGAAGGAACACGTGCCCACAAACT
GAGTATCAGTGTGTTACAGGCAACAATGGCAGTGAGAGCAGTCCTGCTACTACCGGTGCA
CTGTCCACGGGGTCACCTCCCAGGGAGAATCCATCCCATCCTACTGCCAGCGCTCTGTCC
ACAGGATCGCCTCCCATGAAGAATCCATCCCATCCTACTGCCAGCGCTCTGTCCACAGGA
TCGCCTCCCATGAAGAATCCATCCCATCCTACTGCCAGCACACTGTCCATGGGATTGCCT
CCCAGCAGGACTCCATCCCATCCTACTGCCACTGTTCTGTCCACGGGGTCACCTCCCAGC
GAATCCCCATCCAGAACTGGTTCAGCAGCATCAGGAAGCAGCGACAGCAGTATATACCTT
ACTAGTAGTGTTTATTCTTCTAAAATCTCCCAAAATGGGCAGCAATCTCAGGACGTACAG
AAAAAAGAAACATTTCCTAATGTCGCCGAAGAGCCCATCTGGAGAATGATACGGCAGACA
CCTGAGCGCATTCTCATGACATACCAGGTACCTGAGAGGGTTAAAGAAGTTGTACTAAAA
GAAGACCTGGAAAAGCTAGAAAGTATGAGGCAGCAGCAGCCCCAGTTTTCTCATGGGCAA
AAGGAGGAGCTGGCTAAGGTGTATAATTGGATTCAAAGCCAGACTGTCACTCAAGAAATC
GACATTCAAGCCTGTGTCACTTGTGAAAATGAAGATTCAGCTGATGGTGCGGCCACATCC
TGTGGTCAGGTTCTGGTAGAAGACAGCTGTTGA

The sequence of cDNA CLOCK comprises SEQ ID No.6
>ENST00000513440.6 CLOCK-211 cdna:protein_coding

ATGTTGTTTACCGTAAGCTGTAGTAAAATGAGCTCGATTGTTGACAGAGATGACAGTAGT
ATTTTTGATGGGTTGGTGGAAGAAGATGACAAGGACAAAGCGAAAAGAGTATCTAGAAAC
AAATCTGAAAAGAAACGTAGAGATCAATTTAATGTTCTCATTAAAGAACTGGGATCCATG
CTTCCTGGTAATGCTAGAAAGATGGACAAATCTACTGTTCTGCAGAAAAGCATTGATTTT
TTACGAAAACATAAAGAAATCACTGCACAGTCAGATGCTAGTGAAATTCGACAGGACTGG
AAACCTACATTCCTTAGTAATGAAGAGTTTACACAATTAATGTTAGAGGCTCTTGATGGT
TTTTTTTTAGCAATCATGACAGATGGAAGCATAATATATGTGTCTGAGAGTGTAACTTCA

TTACTTGAACATTTACCATCTGATCTTGTGGATCAAAGTATATTTAATTTTATCCCAGAAGGGGAACATTCAGAGGTTTATAAAATACTCTCTACTCATCTGCTGGAAAGTGATTCATTAACCCCAGAATATTTAAAATCAAAAAATCAGTTAGAATTCTGTTGTCACATGCTGCGAGGAACAATAGACCCAAAGGAGCCATCTACCTATGAATATGTAAAATTTATAGGAAATTTCAAATCTTTAAACAGTGTATCCTCTTCAGCACACAATGGTTTTGAAGGAACTATACAACGCACACATAGGCCATCTTATGAAGATAGAGTTTGTTTTGTAGCTACTGTCAGGTTAGCTACACCTCAGTTCATCAAGGAAATGTGCACTGTTGAAGAACCCAATGAAGAGTTTACATCTAGACATAGTTTAGAATGGAAGTTTCTGTTTCTAGATCACAGGGCACCACCCATAATAGGGTATTTGCCATTTGAAGTTCTGGGAACATCAGGCTATGATTACTATCATGTGGATGACCTAGAAAATTTGGCAAAATGTCATGAGCACTTAATGCAATATGGGAAAGGCAAATCATGTTATTATAGGTTCCTGACTAAGGGGCAACAGTGGATTTGGCTTCAGACTCATTATTATATCACTTACCATCAGTGGAATTCAAGGCCAGAGTTTATTGTTTGTACTCACACTGTAGTAAGTTATGCAGAAGTTAGGGCTGAAAGACGACGAGAACTTGGCATTGAAGAGTCTCTTCCTGAGACAGCTGCTGACAAAAGCCAAGATTCTGGGTCAGATAATCGTATAAACACAGTCAGTCTCAAGGAAGCATTGGAAAGGTTTGATCACAGCCCAACCCCTTCTGCCTCTTCTCGGAGTTCAAGAAAATCATCTCACACGGCCGTCTCAGACCCTTCCTCAACACCAACCAAGATCCCGACGGATACGAGCACTCCACCCAGGCAGCATTTACCAGCTCATGAGAAGATGGTGCAAAGAAGGTCATCATTTAGTAGTCAGTCCATAAATTCCCAGTCTGTTGGTTCATCATTAACACAGCCAGTGATGTCTCAAGCTACAAATTTACCAATTCCACAAGGCATGTCCCAGTTTCAGTTTTCAGCTCAATTAGGAGCCATGCAACATCTGAAAGACCAATTGGAACAACGGACACGCATGATAGAAGCAAATATTCATCGGCAACAAGAAGAACTAAGAAAAATTCAAGAACAACTTCAGATGGTCCATGGTCAGGGGCTGCAGATGTTTTTGCAACAATCAAATCCTGGGTTGAATTTTGGTTCCGTTCAACTTTCTTCTGGAAATTCATCTAATATCCAGCAACTTGCACCTATAAATATGCAAGGCCAAGTTGTTCCTACTAACCAGATTCAAAGTGGAATGAATACTGGACACATTGGCACAACTCAGCACATGATACAACAACAGACTTTACAGAGTACATCAACTCAGAGTCAACAAAATGTACTGAGTGGGCACAGTCAGCAAACATCTCTACCCAGTCAGACACAGAGCACTCTTACAGCCCCACTGTATAACACTATGGTGATTTCTCAGCCTGCAGCCGGAAGCATGGTCCAGATTCCATCTAGTATGCCACAAAACAGCACCCAGAGTGCTGCAGTAACTACATTCACTCAGGACAGGCAGATAAGATTTTCTCAAGGTCAACAACTTGTGACCAAATTAGTGACTGCTCCTGTAGCTTGTGGGGCAGTCATGGTACCTAGTACTATGCTTATGGGCCAGGTGGTGACTGCATATCCTACTTTTGCTACACAACAGCAACAGTCACAGACATTGTCAGTAACGCAGCAGCAGCAGCAGCAGAGCTCCCAGGAGCAGCAGCTCACTTCAGTTCAGCAACCATCTCAGGCTCAGCTGACCCAGCCACCGCAACAATTTTTACAGACTTCTAGGTTGCTCCATGGGAATCCCTCAACTCAACTCATTCTCTCTGCTGCATTTCCTCTACAACAGAGCACCTTCCCTCAGTCACATCACCAGCAACATCAGTCTCAGCAACAGCAGCAACTCAGCCGGCACAGGACTGACAGCTTGCCCGACCCTTCCAAGGTTCAACCACAGTAG

The sequence of cDNA NPAS2 comprises SEQ ID No.7
>ENST00000335681.10 cdna:protein_coding

ATGGATGAAGATGAGAAAGACAGAGCCAAGAGAGCTTCTCGAAACAAGTCTGAGAAGAAG
CGTCGGGACCAGTTCAATGTTCTCATCAAAGAGCTCAGTTCCATGCTCCCTGGCAACACG
CGGAAAATGGACAAAACCACCGTGTTGGAAAAGGTCATCGGATTTTTGCAGAAACACAAT
GAAGTCTCAGCGCAAACGGAAATCTGTGACATTCAGCAAGACTGGAAGCCTTCATTCCTC
AGTAATGAAGAATTCACCCAGCTGATGTTGGAGGCATTAGATGGCTTCATTATCGCAGTG

ACAACAGACGGCAGCATCATCTATGTCTCTGACAGTATCACGCCTCTCCTTGGGCATTTA
CCGTCGGATGTCATGGATCAGAATTTGTTAAATTTCCTCCCAGAACAAGAACATTCAGAA
GTTTATAAAATCCTTTCTTCCCATATGCTTGTGACGGATTCCCCCTCCCCAGAATACTTA
AAATCTGACAGCGATTTAGAGTTTTATTGCCATCTTCTCAGAGGCAGCTTGAACCCAAAG
GAATTTCCAACTTATGAATACATAAAATTTGTAGGAAATTTTCGCTCTTACAACAATGTG
CCTAGCCCCTCCTGTAATGGTTTTGACAACACCCTTTCAAGACCTTGCCGGGTGCCACTA
GGAAAGGAGGTTTGCTTCATTGCCACCGTTCGTCTGGCAACACCACAATTCTTAAAGGAA
ATGTGCATAGTTGACGAACCTTTAGAGGAATTCACTTCAAGGCATAGCTTGGAATGGAAA
TTTTTATTTCTGGATCACAGAGCACCTCCAATCATAGGATACCTGCCTTTTGAAGTGCTG
GGAACCTCAGGCTATGACTACTACCACATTGATGACCTGGAGCTCCTGGCCAGGTGTCAC
CAGCACCTGATGCAGTTTGGCAAAGGGAAGTCGTGTTGCTACCGGTTTCTGACCAAAGGT
CAGCAGTGGATCTGGCTGCAGACTCACTACTACATCACCTACCATCAGTGGAACTCCAAG
CCCGAGTTCATCGTGTGCACACACTCGGTGGTCAGTTACGCAGATGTCCGGGTGGAAAGG
AGGCAGGAGCTGGCTCTGGAAGACCCGCCATCCGAGGCCCTCCACTCCTCAGCACTAAAG
GACAAGGGCTCAAGCCTGGAACCTCGGCAGCACTTTAACACACTCGACGTGGGTGCCTCG
GGCCTTAATACCAGTCATTCGCCATCGGCGTCCTCAAGAAGTTCCCACAAATCCTCGCAC
ACAGCCATGTCAGAACCCACCTCCACTCCCACCAAGCTGATGGCAGAGGCCAGCACCCCG
GCTTTGCCAAGATCAGCCACCCTGCCCCAAGAGTTACCTGTCCCCGGGCTCAGCCAGGCA
GCCACCATGCCGGCCCCTCTGCCTTCCCCATCGTCCTGCGACCTCACACAGCAGCTCCTG
CCTCAGACCGTTCTGCAGAGCACGCCCGCTCCCATGGCACAGTTTTCGGCACAGTTCAGC
ATGTTCCAGACCATCAAAGACCAGCTAGAGCAGCGGACGCGGATCCTGCAGGCCAATATC
CGGTGGCAACAGGAAGAGCTCCACAAGATCCAGGAGCAGCTCTGCCTGGTCCAGGACTCC
AACGTCCAGATGTTCCTGCAGCAGCCAGCTGTATCCCTGAGCTTCAGCAGCACCCAGCGA
CCTGAGGCTCAGCAGCAGCTACAGCAAAGGTCAGCTGCAGTGACTCAGCCCCAGCTCGGG
GCGGGCCCCCAACTTCCAGGGCAGATCTCCTCTGCCCAGGTCACAAGCCAGCACCTGCTC
AGAGAATCAAGTGTGATATCAACCCAGGGTCCAAAGCCAATGAGAAGCTCACAGCTAATG
CAGAGCAGCGGCCGCTCTGGAAGCAGCCTAGTGTCCCCGTTCAGCAGCGCCACAGCTGCG
CTCCCGCCAAGTCTGAATCTGACCACACCTGCTTCCACCTCCCAGGATGCCAGCCAGTGC
CAGCCCAGCCCAGACTTCAGCCATGATCGGCAGCTCAGGCTGTTGCTGAGCCAGCCCATC
CAGCCCATGATGCCCGGGTCCTGTGACGCAAGGCAGCCCTCGGAAGTCAGCAGGACGGGA
CGGCAAGTCAAGTACGCCCAGAGCCAGACCGTGTTTCAAAATCCAGACGCACACCCCGCC
AACAGCAGCAGCGCCCCGATGCCCGTCCTGCTGATGGGGCAGGCGGTGCTCCACCCCAGC
TTCCCTGCCTCCCAACCATCGCCCCTGCAGCCTGCACAGGCCCGGCAGCAGCCACCGCAG
CACTACCTGCAGGTACAGGCACCAACCTCTTTGCACAGTGAGCAGCAGGACTCGCTACTT
CTCTCCACCTACTCACAACAGCCAGGGACCCTGGGCTACCCCCAACCACCCCCAGCACAG
CCCCAGCCCCTACGTCCTCCCCGAAGGGTCAGCAGTCTGTCTGAGTCGTCAGGCCTCCAG
CAGCCGCCCCGATAA

The sequence of cDNA CRY1 comprises SEQ ID No.8
>ENST00000008527.10 CRY1-201 cdna:protein_coding

ATGGGGGTGAACGCCGTGCACTGGTTCCGAAAGGGGCTCCGGCTCCACGACAACCCCGCC
CTGAAGGAGTGCATTCAGGGCGCCGACACCATCCGCTGCGTCTACATCCTGGACCCCTGG

TTCGCCGGCTCCTCCAATGTGGGCATCAACAGGTGGCGATTTTTGCTTCAGTGTCTTGAG
GATCTTGATGCCAATCTACGAAAATTAAACTCCCGTCTGTTTGTGATTCGTGGACAACCA
GCAGATGTGTTTCCCAGGCTTTTCAAGGAATGGAACATTACTAAACTTTCAATTGAGTAT
GATTCTGAGCCCTTTGGAAAGGAACGAGACGCAGCTATTAAGAAACTGGCAACTGAAGCT
GGAGTAGAAGTCATTGTAAGAATTTCACATACATTATATGACCTAGACAAGATCATAGAA
CTCAATGGTGGACAACCGCCTCTAACTTATAAAAGATTCCAGACTCTCATCAGCAAAATG
GAACCACTAGAGATACCAGTAGAGACAATTACTTCAGAAGTGATAGAAAAGTGCACAACT
CCTCTGTCTGATGACCATGATGAGAAATATGGAGTCCCTTCACTGGAAGAGCTAGGTTTT
GATACAGATGGCTTATCCTCTGCAGTGTGGCCAGGTGGAGAAACTGAAGCACTTACTCGT
TTGGAAAGGCATTTGGAAAGAAAAGCTTGGGTGGCAAATTTTGAAAGACCTCGAATGAAT
GCGAATTCTCTGCTTGCAAGCCCTACTGGACTTAGTCCTTATCTCCGATTTGGTTGTTTG
TCATGTCGACTGTTTTACTTCAAACTAACAGATCTCTACAAAAAGGTAAAGAAGAACAGT
TCCCCTCCCCTTTCCCTTTATGGGCAACTGTTATGGCGTGAATTTTTCTATACAGCAGCA
ACAAATAATCCACGCTTTGATAAAATGGAAGGAAACCCTATCTGTGTTCAGATTCCTTGG
GATAAAAATCCTGAGGCTTTAGCCAAATGGGCGGAAGGCCGGACAGGCTTTCCATGGATT
GATGCCATCATGACACAGCTTCGTCAGGAGGGTTGGATTCATCATCTAGCCAGGCATGCA
GTTGCTTGCTTCCTGACACGAGGGGACCTGTGGATTAGTTGGGAAGAAGGAATGAAGGTA
TTTGAAGAATTATTGCTTGATGCAGATTGGAGCATAAATGCTGGAAGTTGGATGTGGCTG
TCTTGTAGTTCCTTTTTTCAACAGTTTTTTCACTGCTATTGCCCTGTTGGTTTTGGTAGG
AGAACAGATCCCAATGGAGACTATATCAGGCGTTATTTGCCTGTCCTAAGAGGCTTCCCT
GCAAAATATATCTATGATCCCTGGAATGCACCAGAAGGTATCCAAAAGGTAGCCAAATGT
TTGATAGGAGTTAATTATCCTAAACCAATGGTGAACCATGCTGAGGCAAGCCGTTTGAAT
ATCGAAAGGATGAAACAGATCTATCAGCAGCTTTCACGATATAGAGGACTAGGTCTTCTG
GCATCAGTACCTTCTAATCCTAATGGGAATGGAGGCTTCATGGGATATTCTGCAGAAAAT
ATCCCAGGTTGTAGCAGCAGTGGAAGTTGCTCTCAAGGGAGTGGTATTTTACACTATGCT
CATGGCGACAGTCAGCAAACTCACCTGTTGAAGCAAGGAAGAAGCTCCATGGGCACTGGT
CTCAGTGGTGGGAAACGTCCTAGTCAGGAAGAGGACACACAGAGTATTGGTCCTAAAGTC
CAGAGACAGAGCACTAATTAG

The sequence of cDNA CRY2 comprises SEQ ID No.9
>ENST00000616623.4 CRY2-212 cdna:protein_coding

ATGGGCGGGGTCCACGTCGCCTACCGGGGCGGAGCGGGGGTGGCTGGAGCAGTCTGGACA
GTCATGGCGGCGACTGTGGCGACGGCGGCAGCTGTGGCCCCGGCGCCAGCGCCCGGCACG
GACAGCGCCTCTTCGGTGCACTGGTTCCGCAAAGGGCTGCGACTCCACGACAACCCGGCG
TTGCTGGCGGCCGTGCGCGGGGCGCGCTGCGTGCGCTGCGTTTACATTCTCGACCCGTGG
TTCGCGGCCTCCTCCTCAGTCGGGATCAACCGATGGAGGTTCCTACTTCAGTCTCTGGAA
GATTTGGACACAAGTTTAAGGAAACTGAACTCCCGCCTGTTTGTAGTCCGGGGACAGCCA
GCCGACGTGTTCCCAAGGCTGTTCAAGGAATGGGGAGTGACCCGCTTGACCTTTGAATAT
GACTCTGAACCCTTTGGGAAAGAACGGGATGCAGCCATCATGAAGATGGCCAAGGAGGCT
GGTGTGGAAGTAGTGACGGAGAATTCTCATACCCTCTATGACCTGGACAGGATCATTGAG
CTGAATGGGCAGAAGCCACCCCTTACATACAAGCGCTTTCAGGCCATCATCAGCCGCATG
GAGCTGCCCAAGAAGCCAGTGGGCTTGGTGACCAGCCAGCAGATGGAGAGCTGCAGGGCC
GAGATCCAGGAGAACCACGACGAGACCTACGGCGTGCCCTCCCTGGAGGAGCTGGGGTTC

CCCACTGAAGGACTTGGTCCAGCTGTCTGGCAGGGAGGAGAGACAGAAGCTCTGGCCCGC
CTGGATAAGCACTTGGAACGGAAGGCCTGGGTTGCCAACTATGAGAGACCCCGAATGAAC
GCCAACTCCCTCCTGGCCAGCCCCACAGGCCTCAGCCCCTACCTGCGCTTTGGTTGTCTC
TCCTGCCGCCTCTTCTACTACCGCCTGTGGGACCTGTATAAAAAGGTGAAGCGGAACAGC
ACACCTCCCCTCTCCCTATTTGGGCAACTCCTATGGCGAGAGTTCTTCTACACGGCAGCT
ACCAACAACCCCAGGTTTGACCGCATGGAGGGGAACCCCATCTGCATCCAGATCCCCTGG
GACCGCAATCCTGAGGCCCTGGCCAAGTGGGCTGAGGGCAAGACAGGCTTCCCTTGGATT
GATGCCATCATGACCCAACTGAGGCAGGAGGGCTGGATCCACCACCTGGCCCGGCATGCC
GTGGCCTGCTTCCTGACCCGCGGGGACCTCTGGGTCAGCTGGGAGAGCGGGGTCCGGGTA
TTTGATGAGCTGCTCCTGGATGCAGATTTCAGCGTGAACGCAGGCAGCTGGATGTGGCTG
TCCTGCAGTGCTTTCTTCCAGCAGTTCTTCCACTGCTACTGCCCTGTGGGCTTTGGCCGT
CGCACGGACCCCAGTGGGGACTACATCAGGCGATACCTGCCCAAATTGAAAGCGTTCCCC
TCTCGATACATCTATGAGCCCTGGAATGCCCCAGAGTCAATTCAGAAGGCAGCCAAGTGC
ATCATTGGTGTGGACTACCCACGGCCCATCGTCAACCATGCCGAGACCAGCCGGCTTAAC
ATTGAACGAATGAAGCAGATTTACCAGCAGCTTTCGCGCTACCGGGGACTCTGTCTACTG
GCATCTGTCCCTTCCTGTGTGGAAGACCTCAGTCACCCTGTGGCAGAGCCCAGCTCGAGC
CAGGCTGGCAGCATGAGCAGTGCAGGCCCAAGACCACTACCCAGTGGCCCAGCATCCCCC
AAACGCAAGCTGGAAGCAGCCGAGGAACCACCTGGTGAAGAACTCAGCAAACGGGCCCGG
GTGGCAGAGTTGCCAACCCCAGAGCTGCCGAGCAAGGATGCCTGA

The sequence of cDNA NR1D1comprises SEQ ID No. 10
>ENST00000246672.4 NR1D1-201 cdna:protein_coding

ATGACGACCCTGGACTCCAACAACAACACAGGTGGCGTCATCACCTACATTGGCTCCAGT
GGCTCCTCCCCAAGCCGCACCAGCCCTGAATCCCTCTATAGTGACAACTCCAATGGCAGC
TTCCAGTCCCTGACCCAAGGCTGTCCCACCTACTTCCCACCATCCCCCACTGGCTCCCTC
ACCCAAGACCCGGCTCGCTCCTTTGGGAGCATTCCACCCAGCCTGAGTGATGACGGCTCC
CCTTCTTCCTCATCTTCCTCGTCGTCATCCTCCTCCTCCTTCTATAATGGGAGCCCCCCT
GGGAGTCTACAAGTGGCCATGGAGGACAGCAGCCGAGTGTCCCCCAGCAAGAGCACCAGC
AACATCACCAAGCTGAATGGCATGGTGTTACTGTGTAAAGTGTGTGGGGACGTTGCCTCG
GGCTTCCACTACGGTGTGCACGCCTGCGAGGGCTGCAAGGGCTTTTTCCGTCGGAGCATC
CAGCAGAACATCCAGTACAAAAGGTGTCTGAAGAATGAGAATTGCTCCATCGTCCGCATC
AATCGCAACCGCTGCCAGCAATGTCGCTTCAAGAAGTGTCTCTCTGTGGGCATGTCTCGA
GACGCTGTGCGTTTTGGGCGCATCCCCAAACGAGAGAAGCAGCGGATGCTTGCTGAGATG
CAGAGTGCCATGAACCTGGCCAACAACCAGTTGAGCAGCCAGTGCCCGCTGGAGACTTCA
CCCACCCAGCACCCCACCCCAGGCCCCATGGGCCCCTCGCCACCCCCTGCTCCGGTCCCC
TCACCCCTGGTGGGCTTCTCCCAGTTTCCACAACAGCTGACGCCTCCCAGATCCCCAAGC
CCTGAGCCCACAGTGGAGGATGTGATATCCCAGGTGGCCCGGGCCCATCGAGAGATCTTC
ACCTACGCCCATGACAAGCTGGGCAGCTCACCTGGCAACTTCAATGCCAACCATGCATCA
GGTAGCCCTCCAGCCACCACCCCACATCGCTGGGAAAATCAGGGCTGCCCACCTGCCCCC
AATGACAACAACACCTTGGCTGCCCAGCGTCATAACGAGGCCCTAAATGGTCTGCGCCAG
GCTCCCTCCTCCTACCCTCCCACCTGGCCTCCTGGCCCTGCACACCACAGCTGCCACCAG
TCCAACAGCAACGGGCACCGTCTATGCCCCACCCACGTGTATGCAGCCCCAGAAGGCAAG
GCACCTGCCAACAGTCCCCGGCAGGGCAACTCAAAGAATGTTCTGCTGGCATGTCCTATG

AACATGTACCCGCATGGACGCAGTGGGCGAACGGTGCAGGAGATCTGGGAGGATTTCTCC
ATGAGCTTCACGCCCGCTGTGCGGGAGGTGGTAGAGTTTGCCAAACACATCCCGGGCTTC
CGTGACCTTTCTCAGCATGACCAAGTCACCCTGCTTAAGGCTGGCACCTTTGAGGTGCTG
ATGGTGCGCTTTGCTTCGTTGTTCAACGTGAAGGACCAGACAGTGATGTTCCTAAGCCGC
ACCACCTACAGCCTGCAGGAGCTTGGTGCCATGGGCATGGGAGACCTGCTCAGTGCCATG
TTCGACTTCAGCGAGAAGCTCAACTCCCTGGCGCTTACCGAGGAGGAGCTGGGCCTCTTC
ACCGCGGTGGTGCTTGTCTCTGCAGACCGCTCGGGCATGGAGAATTCCGCTTCGGTGGAG
CAGCTCCAGGAGACGCTGCTGCGGGCTCTTCGGGCTCTGGTGCTGAAGAACCGGCCCTTG
GAGACTTCCCGCTTCACCAAGCTGCTGCTCAAGCTGCCGGACCTGCGGACCCTGAACAAC
ATGCATTCCGAGAAGCTGCTGTCCTTCCGGGTGGACGCCCAGTGA

The sequence of cDNA NR1D2 comprises SEQ ID No. 11
>ENST00000312521.9 NR1D2-201 cdna:protein_coding

ATGGAGGTGAATGCAGGAGGTGTGATTGCCTATATCAGTTCTTCCAGCTCAGCCTCAAGC
CCTGCCTCTTGTCACAGTGAGGGTTCTGAGAATAGTTTCCAGTCCTCCTCCTCTTCTGTT
CCATCTTCTCCAAATAGCTCTAATTCTGATACCAATGGTAATCCCAAGAATGGTGATCTC
GCCAATATTGAAGGCATCTTGAAGAATGATCGAATAGATTGTTCTATGAAAACAAGCAAA
TCGAGTGCACCTGGGATGACAAAAAGTCATAGTGGTGTGACAAAATTTAGTGGCATGGTT
CTACTGTGTAAAGTCTGTGGGGATGTGGCGTCAGGATTCCACTATGGAGTTCATGCTTGC
GAAGGCTGTAAGGGTTTCTTTCGGAGAAGTATTCAACAAAACATCCAGTACAAGAAGTGC
CTGAAGAATGAAAACTGTTCTATAATGAGAATGAATAGGAACAGATGTCAGCAATGTCGC
TTCAAAAAGTGTCTGTCTGTTGGAATGTCAAGAGATGCTGTTCGGTTTGGTCGTATTCCT
AAGCGTGAAAAACAGAGGATGCTAATTGAAATGCAAAGTGCAATGAAGACCATGATGAAC
AGCCAGTTCAGTGGTCACTTGCAAAATGACACATTAGTAGAACATCATGAACAGACAGCC
TTGCCAGCCCAGGAACAGCTGCGACCCAAGCCCCAACTGGAGCAAGAAAACATCAAAAGC
TCTTCTCCTCCATCTTCTGATTTTGCAAAGGAAGAAGTGATTGGCATGGTGACCAGAGCT
CACAAGGATACCTTTATGTATAATCAAGAGCAGCAAGAAAACTCAGCTGAGAGCATGCAG
CCCCAGAGAGGAGAACGGATTCCCAAGAACATGGAGCAATATAATTTAAATCATGATCAT
TGCGGCAATGGGCTTAGCAGCCATTTTCCCTGTAGTGAGAGCCAGCAGCATCTCAATGGA
CAGTTCAAAGGGAGGAATATAATGCATTACCCAAATGGTCATGCCATTTGTATTGCAAAT
GGACATTGTATGAACTTCTCCAATGCTTATACTCAAAGAGTATGTGATAGAGTTCCGATA
GATGGATTTTCTCAGAATGAGAACAAGAATAGTTACCTGTGCAACACTGGAGGAAGAATG
CATCTGGTTTGTCCAATGAGTAAGTCTCCATATGTGGATCCTCATAAATCAGGACATGAA
ATCTGGGAAGAATTTTCGATGAGCTTCACTCCAGCAGTGAAAGAAGTGGTGGAATTTGCA
AAGCGTATTCCTGGGTTCAGAGATCTCTCTCAGCATGACCAGGTCAACCTTTTAAAGGCT
GGGACTTTTGAGGTTTTAATGGTACGGTTCGCATCATTATTTGATGCAAAGGAACGTACT
GTCACCTTTTTAAGTGGAAAGAAATATAGTGTGGATGATTTACACTCAATGGGAGCAGGG
GATCTGCTAAACTCTATGTTTGAATTTAGTGAGAAGCTAAATGCCCTCCAACTTAGTGAT
GAAGAGATGAGTTTGTTTACAGCTGTTGTCCTGGTATCTGCAGATCGATCTGGAATAGAA
AACGTCAACTCTGTGGAGGCTTTGCAGGAAACTCTCATTCGTGCACTAAGGACCTTAATA
ATGAAAAACCATCCAAATGAGGCCTCTATTTTTACAAAACTGCTTCTAAAGTTGCCAGAT
CTTCGATCTTTAAACAACATGCACTCTGAGGAGCTCTTGGCCTTTAAAGTTCACCCTTAA

The sequence of cDNA RORA comprises SEQ ID No. 12
>ENST00000335670.11 RORA-203 cdna:protein_coding

ATGGAGTCAGCTCCGGCAGCCCCCGACCCCGCCGCCAGCGAGCCAGGCAGCAGCGGCGCG
GACGCGGCCGCCGGCTCCAGGGAGACCCCGCTGAACCAGGAATCCGCCCGCAAGAGCGAG
CCGCCTGCCCCGGTGCGCAGACAGAGCTATTCCAGCACCAGCAGAGGTATCTCAGTAACG
AAGAAGACACATACATCTCAAATTGAAATTATTCCATGCAAGATCTGTGGAGACAAATCA
TCAGGAATCCATTATGGTGTCATTACATGTGAAGGCTGCAAGGGCTTTTTCAGGAGAAGT
CAGCAAAGCAATGCCACCTACTCCTGTCCTCGTCAGAAGAACTGTTTGATTGATCGAACC
AGTAGAAACCGCTGCCAACACTGTCGATTACAGAAATGCCTTGCCGTAGGGATGTCTCGA
GATGCTGTAAAATTTGGCCGAATGTCAAAAAAGCAGAGAGACAGCTTGTATGCAGAAGTA
CAGAAACACCGGATGCAGCAGCAGCAGCGCGACCACCAGCAGCAGCCTGGAGAGGCTGAG
CCGCTGACGCCCACCTACAACATCTCGGCCAACGGGCTGACGGAACTTCACGACGACCTC
AGTAACTACATTGACGGGCACACCCCTGAGGGGAGTAAGGCAGACTCCGCCGTCAGCAGC
TTCTACCTGGACATACAGCCTTCCCCAGACCAGTCAGGTCTTGATATCAATGGAATCAAA
CCAGAACCAATATGTGACTACACACCAGCATCAGGCTTCTTTCCCTACTGTTCGTTCACC
AACGGCGAGACTTCCCCAACTGTGTCCATGGCAGAATTAGAACACCTTGCACAGAATATA
TCTAAATCGCATCTGGAAACCTGCCAATACTTGAGAGAAGAGCTCCAGCAGATAACGTGG
CAGACCTTTTTACAGGAAGAAATTGAGAACTATCAAAACAAGCAGCGGGAGGTGATGTGG
CAATTGTGTGCCATCAAAATTACAGAAGCTATACAGTATGTGGTGGAGTTTGCCAAACGC
ATTGATGGATTTATGGAACTGTGTCAAAATGATCAAATTGTGCTTCTAAAAGCAGGTTCT
CTAGAGGTGGTGTTTATCAGAATGTGCCGTGCCTTTGACTCTCAGAACAACACCGTGTAC
TTTGATGGGAAGTATGCCAGCCCCGACGTCTTCAAATCCTTAGGTTGTGAAGACTTTATT
AGCTTTGTGTTTGAATTTGGAAAGAGTTTATGTTCTATGCACCTGACTGAAGATGAAATT
GCATTATTTTCTGCATTTGTACTGATGTCAGCAGATCGCTCATGGCTGCAAGAAAAGGTA
AAAATTGAAAAACTGCAACAGAAAATTCAGCTAGCTCTTCAACACGTCCTACAGAAGAAT
CACCGAGAAGATGGAATACTAACAAAGTTAATATGCAAGGTGTCTACCTTAAGAGCCTTA
TGTGGACGACATACAGAAAAGCTAATGGCATTTAAAGCAATATACCCAGACATTGTGCGA
CTTCATTTTCCTCCATTATACAAGGAGTTGTTCACTTCAGAATTTGAGCCAGCAATGCAA
ATTGATGGGTAA

The sequence of cDNA RORB comprises SEQ ID No. 13
>ENST00000376896.8 RORB-201 cdna:protein_coding

ATGCGAGCACAAATTGAAGTGATACCATGCAAAATTTGTGGCGATAAGTCCTCTGGGATC
CACTACGGAGTCATCACATGTGAAGGCTGCAAGGGATTCTTTAGGAGGAGCCAGCAGAAC
AATGCTTCTTATTCCTGCCCAAGGCAGAGAAACTGTTTAATTGACAGAACGAACAGAAAC
CGTTGCCAACACTGCCGACTGCAGAAGTGTCTTGCCCTAGGAATGTCAAGAGATGCTGTG
AAGTTTGGGAGGATGTCCAAGAAGCAAAGGGACAGCCTGTATGCTGAGGTGCAGAAGCAC
CAGCAGCGGCTGCAGGAACAGCGGCAGCAGCAGAGTGGGGAGGCAGAAGCCCTTGCCAGG
GTGTACAGCAGCAGCATTAGCAACGGCCTGAGCAACCTGAACAACGAGACCAGCGGCACT
TATGCCAACGGGCACGTCATTGACCTGCCCAAGTCTGAGGGTTATTACAACGTCGATTCC

GGTCAGCCGTCCCCTGATCAGTCAGGACTTGACATGACTGGAATCAAACAGATAAAGCAA
GAACCTATCTATGACCTCACATCCGTACCCAACTTGTTTACCTATAGCTCTTTCAACAAT
GGGCAGTTAGCACCAGGGATAACCATGACTGAAATCGACCGAATTGCACAGAACATCATT
AAGTCCCATTTGGAGACATGTCAATACACCATGGAAGAGCTGCACCAGCTGGCGTGGCAG
ACCCACACCTATGAAGAAATTAAAGCATATCAAAGCAAGTCCAGGGAAGCACTGTGGCAA
CAATGTGCCATCCAGATCACTCACGCCATCCAATACGTGGTGGAGTTTGCAAAGCGGATA
ACAGGCTTCATGGAGCTCTGTCAAAATGATCAAATTCTACTTCTGAAGTCAGGTTGCTTG
GAAGTGGTTTTAGTGAGAATGTGCCGTGCCTTCAACCCATTAAACAACACTGTTCTGTTT
GAAGGAAAATATGGAGGAATGCAAATGTTCAAAGCCTTAGGTTCTGATGACCTAGTGAAT
GAAGCATTTGACTTTGCAAAGAATTTGTGTTCCTTGCAGCTGACCGAGGAGGAGATCGCT
TTGTTCTCATCTGCTGTTCTGATATCTCCAGACCGAGCCTGGCTTATAGAACCAAGGAAA
GTCCAGAAGCTTCAGGAAAAAATTTATTTTGCACTTCAACATGTGATTCAGAAGAATCAC
CTGGATGATGAGACCTTGGCAAAGTTAATAGCCAAGATACCAACCATCACGGCAGTTTGC
AACTTGCACGGGGAGAAGCTGCAGGTATTTAAGCAATCTCATCCAGAGATAGTGAATACA
CTGTTTCCTCCGTTATACAAGGAGCTCTTTAATCCTGACTGTGCCACCGGCTGCAAATGA

The sequence of cDNA RORC comprises SEQ ID No. 14
>ENST00000318247.7 RORC-201 cdna:protein_coding

ATGGACAGGGCCCCACAGAGACAGCACCGAGCCTCACGGGAGCTGCTGGCTGCAAAGAAG
ACCCACACCTCACAAATTGAAGTGATCCCTTGCAAAATCTGTGGGGACAAGTCGTCTGGG
ATCCACTACGGGGTTATCACCTGTGAGGGGTGCAAGGGCTTCTTCCGCCGGAGCCAGCGC
TGTAACGCGGCCTACTCCTGCACCCGTCAGCAGAACTGCCCCATCGACCGCACCAGCCGA
AACCGATGCCAGCACTGCCGCCTGCAGAAATGCCTGGCGCTGGGCATGTCCCGAGATGCT
GTCAAGTTCGGCCGCATGTCCAAGAAGCAGAGGGACAGCCTGCATGCAGAAGTGCAGAAA
CAGCTGCAGCAGCGGCAACAGCAGCAACAGGAACCAGTGGTCAAGACCCCTCCAGCAGGG
GCCCAAGGAGCAGATACCCTCACCTACACCTTGGGGCTCCCAGACGGGCAGCTGCCCCTG
GGCTCCTCGCCTGACCTGCCTGAGGCTTCTGCCTGTCCCCCTGGCCTCCTGAAAGCCTCA
GGCTCTGGGCCCTCATATTCCAACAACTTGGCCAAGGCAGGGCTCAATGGGGCCTCATGC
CACCTTGAATACAGCCCTGAGCGGGGCAAGGCTGAGGGCAGAGAGAGCTTCTATAGCACA
GGCAGCCAGCTGACCCCTGACCGATGTGGACTTCGTTTTGAGGAACACAGGCATCCTGGG
CTTGGGGAACTGGGACAGGGCCCAGACAGCTACGGCAGCCCCAGTTTCCGCAGCACACCG
GAGGCACCCTATGCCTCCCTGACAGAGATAGAGCACCTGGTGCAGAGCGTCTGCAAGTCC
TACAGGGAGACATGCCAGCTGCGGCTGGAGGACCTGCTGCGGCAGCGCTCCAACATCTTC
TCCCGGGAGGAAGTGACTGGCTACCAGAGGAAGTCCATGTGGGAGATGTGGGAACGGTGT
GCCCACCACCTCACCGAGGCCATTCAGTACGTGGTGGAGTTCGCCAAGAGGCTCTCAGGC
TTTATGGAGCTCTGCCAGAATGACCAGATTGTGCTTCTCAAAGCAGGAGCAATGGAAGTG
GTGCTGGTTAGGATGTGCCGGGCCTACAATGCTGACAACCGCACGGTCTTTTTTGAAGGC
AAATACGGTGGCATGGAGCTGTTCCGAGCCTTGGGCTGCAGCGAGCTCATCAGCTCCATC
TTTGACTTCTCCCACTCCCTAAGTGCCTTGCACTTTTCCGAGGATGAGATTGCCCTCTAC
ACAGCCCTTGTTCTCATCAATGCCCATCGGCCAGGGCTCCAAGAGAAAAGGAAAGTAGAA
CAGCTGCAGTACAATCTGGAGCTGGCCTTTCATCATCATCTCTGCAAGACTCATCGCCAA
AGCATCCTGGCAAAGCTGCCACCCAAGGGGAAGCTTCGGAGCCTGTGTAGCCAGCATGTG
GAAAGGCTGCAGATCTTCCAGCACCTCCACCCCATCGTGGTCCAAGCCGCTTTCCCTCCA
CTCTACAAGGAGCTCTTCAGCACTGAAACCGAGTCACCTGTGGGGCTGTCCAAGTGA

In one embodiment of the method according to the invention assessing the circadian rhythm of said subject comprises determining a periodic function for each of at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, that approximates said expression levels for each of at least two core clock genes, in particular for said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, preferably comprising curve fitting of a non-linear periodic model function to the respective expression levels, wherein the curve fitting is preferably carried out by means of harmonic regression.

The core clock genes may be selected from the group comprising *Arntl (Bmal1), Arntl2, Clock, Per1, Per2, Per3, Npas2, Cry1, Cry2, Nr1d1, Nrld2, Rora, Rorb and Robc.*

In one embodiment of the method according to the invention assessing the circadian rhythm of said subject comprises determining a periodic function for each of ARNTL (BMAL1) and PER2 that approximates said expression levels for each of ARNTL (BMAL1) and PER2, preferably comprising curve fitting of a non-linear periodic model function to the respective expression levels, wherein the curve fitting is preferably carried out by means of harmonic regression.

It will be appreciated that other curve fitting methods may be applied for determining a mathematical function that has the best fit to the series of the measured gene expressions (here: expression levels for each of e.g. ARNTL (BMAL1) and PER2. The skilled person will understand that curve fitting in the context of this disclosure aims at finding a periodic function (oscillatory function) because of the periodicity of the circadian clock(s). While curve fitting may generally aim at finding an interpolation for exact fitting of the data points, methods that approximate the series of measure gene expressions will be preferred, e.g. smoothing, in which a "smooth" function is constructed that approximately fits the data.

It has been found that regression analysis methods are more appropriate here, which use statistical data, not least because the determined periodic function shall represent not only the measured data points but particularly future values. Polynomial interpolation or polynomial regression may be alternatively applied. Preferably, harmonic regression is used, which is based on the trigonometric functions sine and cosine. As will be appreciated by a person skilled in the art, various methods for minimizing an error between the fitted curve and the measured data points may be applied, such as square errors, which is set forth in more detail below. In the method of harmonic regression, the model $y(t) = m + a\cos(\omega t) + b\sin(\omega t)$ is fitted to the measured data to determine the absolute ($A = \sqrt{(a2 + b2)}$) and relative amplitude as well as the phase ($\tan\varphi = b/a$), the p-value and the confidence interval. The significance level p may be selected as $p < 0.05$.

In one embodiment of the method according to the invention the computational step comprises processing the determined expression levels and/or the respectively fitted periodic functions to derive characteristic data for each of at least two core clock genes, in particular of said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, said processing comprising determining the mean expression level of expression of at least two core clock genes, in particular of said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and normalizing the expression levels using the mean expression level.

In one embodiment of the method according to the invention the computational step comprises processing the determined expression levels and/or the respectively fitted periodic functions to derive characteristic data for each of ARNTL (BMAL1), and PER2, said processing comprising determining the mean expression level of expression of ARNTL (BMAL1), and PER2 and normalizing the expression levels using the mean expression level.

Particularly in view of the machine learning processes as described below, the "raw data", i.e. the measured gene expression levels for each the core clock genes, e.g. of ARNTL (BMAL1), and PER2, including the obtained periodic functions resulting from the curve fitting, have to be preprocessed to bring them into a form that is suitable for the intended machine learning algorithm. For instance, the preprocessing includes extracting data of interest (characteristic data) and setting the dimensionality for the machine learning, i.e. number of parameters. Further, in order to get comparable parameters, normalization is typically required to achieve a common scale for all parameters. It has been found that using the mean expression level for normalizing the measured data is a suitable approach. Further, in order not to lose the absolute values, the mean level is added to the parameter space. This will be set forth also in more detail below.

In one embodiment of the method according to the invention said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes, and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or

- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
  wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In one embodiment of the method according to the invention said characteristic data comprise:

- the amplitude of change of expression of ARNTL (BMAL1) and/or PER2 over the day, and/or
- the mean expression level of expression of ARNTL (BMAL1) and/or PER2, and/ or
- the peak expression level of ARNTL (BMAL1) and/or PER2 over the day, and/or
- the relative expression levels of expression of ARNTL (BMAL1) and PER2, and/or
- the time of the peak expression level of ARNTL (BMAL1) and/or PER2, and/or the difference in time of the peak expression levels of ARNTL (BMAL1) and PER2.

The amplitude, period and phase expression level of expression of ARNTL (BMAL1) and/or PER2 are extracted from the determined expression levels and/or the respectively fitted periodic function.

In one embodiment of the method according to the invention of said characteristic data only the timing of the peak expression level of PER2 and the mean expression level of ARNTL (BMAL1) are used in said computational step.

In one embodiment of the method according to the invention the computational step further comprises

- fitting a network computational model to the derived characteristic data that comprises a representation of the periodic time course of the expression levels for each of at least two core clock genes, in particular of said at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2 as well as a representation of the periodic time course of the expression level for at least one, preferably a plurality of further gene(s) included in a gene regulatory network that includes BMAL1 and PER2; and/or
- training a machine learning algorithm on the derived characteristic data of the network computational model, particularly optimize in terms of the representation of the periodic time course of the expression level for the at least one further gene.

In particular, the network computational model is built to obtain data for at least one further gene that has not been directly measured in the saliva samples, i.e. the network computational model represents a gene network which contains the clock elements (those genes of the aforementioned group of core clock genes, which are measured, such as ARNTL (BMAL1) and PER2 and further elements relevant for determining the peak time for sport performance, which further elements cannot (or at least not with reasonable effort) be measured particularly in the saliva samples. This mathematical modelling may use differential equations and also statistical data.

In one embodiment of the method according to the invention assessing and/or predicting the individual diurnal athletic performance times comprises in the computational step fitting a prediction computational model on data obtained from said fitted periodic functions and/or said network computational model, wherein the prediction computational model is based on machine learning, including at least one classification method and/or at least one clustering method wherein said method(s) are preferably selected from the group comprising: K-nearest neighbor algorithm, unsupervised clustering, deep neural networks, random forest algorithm, and support vector machines.

In one embodiment of the method according to the invention additional physiological data of the subject are provided for fitting the prediction computational model. Said physiological data may be selected from the group comprising: body temperature, heart rate, eating/fasting patterns and/or sleep/wake patterns. It will be appreciated that one or more of the aforementioned physiological data or other physiological parameters from the subject may be provided. While such data may be obtained manually by the subject (user) and/or by medical staff, it may be envisioned to obtain at least some of the physiological data by means of a portable electronic device, particularly a wearable, such as a fitness watch, wristband or the like. Vice versa, the result of the method of the present invention may be presented on such wearable device so that the user directly sees e.g. their circadian profile (just like they are used to see other physiological or fitness data, e.g. how long and how fast their jogging was, or how their sleep quality was). Of course, the result may be provided by other electronic devices, like a smartphone, tablet or personal computer.

In one embodiment of the method according to the invention the oscillation amplitude and/or peak time of the individual diurnal athletic performance during the day are assessed and/or predicted, wherein predicting the peak time of the individual diurnal athletic performance preferably comprises selecting at least one period of time from at least two distinct periods of time during the day as the peak time. This simple approach may allow determining the peak performance peak at least in two "categories" (i.e. periods of time), such as "early" or "morning" and "late" or "afternoon"/"evening". Depending on the available data, i.e. the power of the prediction computation model, more precise predictions may be envisioned, e.g. selecting between more (and shorter) time windows per day, specific "peak hours" or even specific points in time that enable the subject to even more precisely select a time for a work out, training or the like.

In one embodiment of the method according to the invention the network computational model and/or the prediction computational model form a personalized model for said subject. The personalization particularly comes from the molecular data, i.e. the measurements of the gene expression which are unique for each person. Additional physiological data like temperature, heart rate, sleep/wake cycles as mentioned above can also be used for personalization. These are all circadian events, too, meaning they vary within 24 hours. While such physiological data may be of additional value, the models, and thus predictions are primarily based on the molecular data, i.e. the gene expressions. It is noted that while the network computational model may be personalized because there is a new model for each new person (using the personal gene expressions), the prediction computational model is not. There is one prediction model relating subject data to prediction for all subjects, and this model should generalize to future subjects without being retrained. This means that, a model for the gene network is built individually for each person, and also each person gets his or her own and individual prediction for the sport peak performance time but using the same model here for each person. However, when using the differential equations model, each new person gets a new model.

In one embodiment of the method according to the invention in addition the expression levels of at least one gene selected from the group comprising AKT1, MYOD1, ACE, PPARGC1A, Elovl5 and Slc2a4 is determined or predicted base on a model of the underlying genetic network and used for said assessment and/or prediction.

The sequence of AKT1 comprises SEQ ID No. 15:

>ENST00000554581.5 AKT1-208 cdna:protein_coding

ATGAGCGACGTGGCTATTGTGAAGGAGGGTTGGCTGCACAAACGAGGGGAGTACATCAAG
ACCTGGCGGCCACGCTACTTCCTCCTCAAGAATGATGGCACCTTCATTGGCTACAAGGAG
CGGCCGCAGGATGTGGACCAACGTGAGGCTCCCCTCAACAACTTCTCTGTGGCGCAGTGC
CAGCTGATGAAGACGGAGCGGCCCCGGCCCAACACCTTCATCATCCGCTGCCTGCAGTGG
ACCACTGTCATCGAACGCACCTTCCATGTGGAGACTCCTGAGGAGCGGGAGGAGTGGACA
ACCGCCATCCAGACTGTGGCTGACGGCCTCAAGAAGCAGGAGGAGGAGGAGATGGACTTC
CGGTCGGGCTCACCCAGTGACAACTCAGGGGCTGAAGAGATGGAGGTGTCCCTGGCCAAG
CCCAAGCACCGCGTGACCATGAACGAGTTTGAGTACCTGAAGCTGCTGGGCAAGGGCACT
TTCGGCAAGGTGATCCTGGTGAAGGAGAAGGCCACAGGCCGCTACTACGCCATGAAGATC
CTCAAGAAGGAAGTCATCGTGGCCAAGGACGAGGTGGCCCACACACTCACCGAGAACCGC
GTCCTGCAGAACTCCAGGCACCCCTTCCTCACAGCCCTGAAGTACTCTTTCCAGACCCAC
GACCGCCTCTGCTTTGTCATGGAGTACGCCAACGGGGGCGAGCTGTTCTTCCACCTGTCC
CGGGAGCGTGTGTTCTCCGAGGACCGGGCCCGCTTCTATGGCGCTGAGATTGTGTCAGCC
CTGGACTACCTGCACTCGGAGAAGAACGTGGTGTACCGGGACCTCAAGCTGGAGAACCTC
ATGCTGGACAAGGACGGGCACATTAAGATCACAGACTTCGGGCTGTGCAAGGAGGGGATC
AAGGACGGTGCCACCATGAAGACCTTTTGCGGCACACCTGAGTACCTGGCCCCCGAGGTG

CTGGAGGACAATGACTACGGCCGTGCAGTGGACTGGTGGGGGCTGGGCGTGGTCATGTAC
GAGATGATGTGCGGTCGCCTGCCCTTCTACAACCAGGACCATGAGAAGCTTTTTGAGCTC
ATCCTCATGGAGGAGATCCGCTTCCCGCGCACGCTTGGTCCCGAGGCCAAGTCCTTGCTT
TCAGGGCTGCTCAAGAAGGACCCCAAGCAGAGGCTTGGCGGGGGCTCCGAGGACGCCAAG
GAGATCATGCAGCATCGCTTCTTTGCCGGTATCGTGTGGCAGCACGTGTACGAGAAGAAG
CTCAGCCCACCCTTCAAGCCCCAGGTCACGTCGGAGACTGACACCAGGTATTTTGATGAG
GAGTTCACGGCCCAGATGATCACCATCACACCACCTGACCAAGATGACAGCATGGAGTGT
GTGGACAGCGAGCGCAGGCCCCACTTCCCCCAGTTCTCCTACTCGGCCAGCGGCACGGCC
TGA

The sequence of MYOD1 cDNA comprises SEQ ID No. 16:
>ENST00000250003.4 MYOD1-201 cdna:protein_coding

ATGGAGCTACTGTCGCCACCGCTCCGCGACGTAGACCTGACGGCCCCCGACGGCTCTCTC
TGCTCCTTTGCCACAACGGACGACTTCTATGACGACCCGTGTTTCGACTCCCCGGACCTG
CGCTTCTTCGAAGACCTGGACCCGCGCCTGATGCACGTGGGCGCGCTCCTGAAACCCGAA
GAGCACTCGCACTTCCCCGCGGCGGTGCACCCGGCCCCGGGCGCACGTGAGGACGAGCAT
GTGCGCGCGCCCAGCGGGCACCACCAGGCGGGCCGCTGCCTACTGTGGGCCTGCAAGGCG
TGCAAGCGCAAGACCACCAACGCCGACCGCCGCAAGGCCGCCACCATGCGCGAGCGGCGC
CGCCTGAGCAAAGTAAATGAGGCCTTTGAGACACTCAAGCGCTGCACGTCGAGCAATCCA
AACCAGCGGTTGCCCAAGGTGGAGATCCTGCGCAACGCCATCCGCTATATCGAGGGCCTG
CAGGCTCTGCTGCGCGACCAGGACGCCGCGCCCCCTGGCGCCGCAGCCGCCTTCTATGCG
CCGGGCCCGCTGCCCCCGGGCCGCGGCGGCGAGCACTACAGCGGCGACTCCGACGCGTCC
AGCCCGCGCTCCAACTGCTCCGACGGCATGATGGACTACAGCGGCCCCCCGAGCGGCGCC
CGGCGGCGGAACTGCTACGAAGGCGCCTACTACAACGAGGCGCCCAGCGAACCCAGGCCC
GGGAAGAGTGCGGCGGTGTCGAGCCTAGACTGCCTGTCCAGCATCGTGGAGCGCATCTCC
ACCGAGAGCCCTGCGGCGCCCGCCCTCCTGCTGGCGGACGTGCCTTCTGAGTCGCCTCCG
CGCAGGCAAGAGGCTGCCGCCCCCAGCGAGGGAGAGAGCAGCGGCGACCCCACCCAGTCA
CCGGACGCCGCCCCGCAGTGCCCTGCGGGTGCGAACCCCAACCCGATATACCAGGTGCTC
TGA

The sequence of ACE cDNA comprises SEQ ID No. 17:
>ENST00000290866.10 ACE-202 cdna:protein_coding

ATGGGGGCCGCCTCGGGCCGCCGGGGGCCGGGGCTGCTGCTGCCGCTGCCGCTGCTGTTG
CTGCTGCCGCCGCAGCCCGCCCTGGCGTTGGACCCCGGGCTGCAGCCCGGCAACTTTTCT
GCTGACGAGGCCGGGGCGCAGCTCTTCGCGCAGAGCTACAACTCCAGCGCCGAACAGGTG
CTGTTCCAGAGCGTGGCCGCCAGCTGGGCGCACGACACCAACATCACCGCGGAGAATGCA
AGGCGCCAGGAGGAAGCAGCCCTGCTCAGCCAGGAGTTTGCGGAGGCCTGGGGCCAGAAG
GCCAAGGAGCTGTATGAACCGATCTGGCAGAACTTCACGGACCCGCAGCTGCGCAGGATC
ATCGGAGCTGTGCGCACCCTGGGCTCTGCCAACCTGCCCCTGGCTAAGCGGCAGCAGTAC
AACGCCCTGCTAAGCAACATGAGCAGGATCTACTCCACCGCCAAGGTCTGCCTCCCCAAC

AAGACTGCCACCTGCTGGTCCCTGGACCCAGATCTCACCAACATCCTGGCTTCCTCGCGA
AGCTACGCCATGCTCCTGTTTGCCTGGGAGGGCTGGCACAACGCTGCGGGCATCCCGCTG
AAACCGCTGTACGAGGATTTCACTGCCCTCAGCAATGAAGCCTACAAGCAGGACGGCTTC
ACAGACACGGGGGCCTACTGGCGCTCCTGGTACAACTCCCCCACCTTCGAGGACGATCTG
GAACACCTCTACCAACAGCTAGAGCCCCTCTACCTGAACCTCCATGCCTTCGTCCGCCGC
GCACTGCATCGCCGATACGGAGACAGATACATCAACCTCAGGGGACCCATCCCTGCTCAT
CTGCTGGGAGACATGTGGGCCCAGAGCTGGGAAAACATCTACGACATGGTGGTGCCTTTC
CCAGACAAGCCCAACCTCGATGTCACCAGTACTATGCTGCAGCAGGGCTGGAACGCCACG
CACATGTTCCGGGTGGCAGAGGAGTTCTTCACCTCCCTGGAGCTCTCCCCCATGCCTCCC
GAGTTCTGGGAAGGGTCGATGCTGGAGAAGCCGGCCGACGGGCGGGAAGTGGTGTGCCAC
GCCTCGGCTTGGGACTTCTACAACAGGAAAGACTTCAGGATCAAGCAGTGCACACGGGTC
ACGATGGACCAGCTCTCCACAGTGCACCATGAGATGGGCCATATACAGTACTACCTGCAG
TACAAGGATCTGCCCGTCTCCCTGCGTCGGGGGGCCAACCCCGGCTTCCATGAGGCCATT
GGGGACGTGCTGGCGCTCTCGGTCTCCACTCCTGAACATCTGCACAAAATCGGCCTGCTG
GACCGTGTCACCAATGACACGGAAAGTGACATCAATTACTTGCTAAAAATGGCACTGGAA
AAAATTGCCTTCCTGCCCTTTGGCTACTTGGTGGACCAGTGGCGCTGGGGGGTCTTTAGT
GGGCGTACCCCCCCTTCCCGCTACAACTTCGACTGGTGGTATCTTCGAACCAAGTATCAG
GGGATCTGTCCTCCTGTTACCCGAAACGAAACCCACTTTGATGCTGGAGCTAAGTTTCAT
GTTCCAAATGTGACACCATACATCAGGTACTTTGTGAGTTTTGTCCTGCAGTTCCAGTTC
CATGAAGCCCTGTGCAAGGAGGCAGGCTATGAGGGCCCACTGCACCAGTGTGACATCTAC
CGGTCCACCAAGGCAGGGGCCAAGCTCCGGAAGGTGCTGCAGGCTGGCTCCTCCAGGCCC
TGGCAGGAGGTGCTGAAGGACATGGTCGGCTTAGATGCCCTGGATGCCCAGCCGCTGCTC
AAGTACTTCCAGCCAGTCACCCAGTGGCTGCAGGAGCAGAACCAGCAGAACGGCGAGGTC
CTGGGCTGGCCCGAGTACCAGTGGCACCCGCCGTTGCCTGACAACTACCCGGAGGGCATA
GACCTGGTGACTGATGAGGCTGAGGCCAGCAAGTTTGTGGAGGAATATGACCGGACATCC
CAGGTGGTGTGGAACGAGTATGCCGAGGCCAACTGGAACTACAACACCAACATCACCACA
GAGACCAGCAAGATTCTGCTGCAGAAGAACATGCAAATAGCCAACCACACCCTGAAGTAC
GGCACCCAGGCCAGGAAGTTTGATGTGAACCAGTTGCAGAACACCACTATCAAGCGGATC
ATAAAGAAGGTTCAGGACCTAGAACGGGCAGCACTGCCTGCCCAGGAGCTGGAGGAGTAC
AACAAGATCCTGTTGGATATGGAAACCACCTACAGCGTGGCCACTGTGTGCCACCCGAAT
GGCAGCTGCCTGCAGCTCGAGCCAGATCTGACGAATGTGATGGCCACGTCCCGGAAATAT
GAAGACCTGTTATGGGCATGGGAGGGCTGGCGAGACAAGGCGGGGAGAGCCATCCTCCAG
TTTTACCCGAAATACGTGGAACTCATCAACCAGGCTGCCCGGCTCAATGGCTATGTAGAT
GCAGGGGACTCGTGGAGGTCTATGTACGAGACACCATCCCTGGAGCAAGACCTGGAGCGG
CTCTTCCAGGAGCTGCAGCCACTCTACCTCAACCTGCATGCCTACGTGCGCCGGGCCCTG
CACCGTCACTACGGGGCCCAGCACATCAACCTGGAGGGGCCCATTCCTGCTCACCTGCTG
GGGAACATGTGGGCGCAGACCTGGTCCAACATCTATGACTTGGTGGTGCCCTTCCCTTCA
GCCCCCTCGATGGACACCACAGAGGCTATGCTAAAGCAGGGCTGGACGCCCAGGAGGATG
TTTAAGGAGGCTGATGATTTCTTCACCTCCCTGGGGCTGCTGCCCGTGCCTCCTGAGTTC
TGGAACAAGTCGATGCTGGAGAAGCCAACCGACGGGCGGGAGGTGGTCTGCCACGCCTCG
GCCTGGGACTTCTACAACGGCAAGGACTTCCGGATCAAGCAGTGCACCACCGTGAACTTG
GAGGACCTGGTGGTGGCCCACCACGAAATGGGCCACATCCAGTATTTCATGCAGTACAAA
GACTTACCTGTGGCCTTGAGGGAGGGTGCCAACCCCGGCTTCCATGAGGCCATTGGGGAC
GTGCTAGCCCTCTCAGTGTCTACGCCCAAGCACCTGCACAGTCTCAACCTGCTGAGCAGT

GAGGGTGGCAGCGACGAGCATGACATCAACTTTCTGATGAAGATGGCCCTTGACAAGATC
GCCTTTATCCCCTTCAGCTACCTCGTCGATCAGTGGCGCTGGAGGGTATTTGATGGAAGC
ATCACCAAGGAGAACTATAACCAGGAGTGGTGGAGCCTCAGGCTGAAGTACCAGGGCCTC
TGCCCCCCAGTGCCCAGGACTCAAGGTGACTTTGACCCAGGGGCCAAGTTCCACATTCCT
TCTAGCGTGCCTTACATCAGGTACTTTGTCAGCTTCATCATCCAGTTCCAGTTCCACGAG
GCACTGTGCCAGGCAGCTGGCCACACGGGCCCCCTGCACAAGTGTGACATCTACCAGTCC
AAGGAGGCCGGGCAGCGCCTGGCGACCGCCATGAAGCTGGGCTTCAGTAGGCCGTGGCCG
GAAGCCATGCAGCTGATCACGGGCCAGCCCAACATGAGCGCCTCGGCCATGTTGAGCTAC
TTCAAGCCGCTGCTGGACTGGCTCCGCACGGAGAACGAGCTGCATGGGGAGAAGCTGGGC
TGGCCGCAGTACAACTGGACGCCGAACTCCGCTCGCTCAGAAGGGCCCCTCCCAGACAGC
GGCCGCGTCAGCTTCCTGGGCCTGGACCTGGATGCGCAGCAGGCCCGCGTGGGCCAGTGG
CTGCTGCTCTTCCTGGGCATCGCCCTGCTGGTAGCCACCCTGGGCCTCAGCCAGCGGCTC
TTCAGCATCCGCCACCGCAGCCTCCACCGGCACTCCCACGGGCCCCAGTTCGGCTCCGAG
GTGGAGCTGAGACACTCCTGA

The sequence of PPARGC1A cDNA comprises SEQ ID No. 18:
>ENST00000264867.7 PPARGC1A-201 cdna:protein_coding

ATGGCGTGGGACATGTGCAACCAGGACTCTGAGTCTGTATGGAGTGACATCGAGTGTGCT
GCTCTGGTTGGTGAAGACCAGCCTCTTTGCCCAGATCTTCCTGAACTTGATCTTTCTGAA
CTAGATGTGAACGACTTGGATACAGACAGCTTTCTGGGTGGACTCAAGTGGTGCAGTGAC
CAATCAGAAATAATATCCAATCAGTACAACAATGAGCCTTCAAACATATTTGAGAAGATA
GATGAAGAGAATGAGGCAAACTTGCTAGCAGTCCTCACAGAGACACTAGACAGTCTCCCT
GTGGATGAAGACGGATTGCCCTCATTTGATGCGCTGACAGATGGAGACGTGACCACTGAC
AATGAGGCTAGTCCTTCCTCCATGCCTGACGGCACCCCTCCACCCCAGGAGGCAGAAGAG
CCGTCTCTACTTAAGAAGCTCTTACTGGCACCAGCCAACACTCAGCTAAGTTATAATGAA
TGCAGTGGTCTCAGTACCCAGAACCATGCAAATCACAATCACAGGATCAGAACAAACCCT
GCAATTGTTAAGACTGAGAATTCATGGAGCAATAAAGCGAAGAGTATTTGTCAACAGCAA
AAGCCACAAAGACGTCCCTGCTCGGAGCTTCTCAAATATCTGACCACAAACGATGACCCT
CCTCACACCAAACCCACAGAGAACAGAAACAGCAGCAGAGACAAATGCACCTCCAAAAAG
AAGTCCCACACACAGTCGCAGTCACAACACTTACAAGCCAAACCAACAACTTTATCTCTT
CCTCTGACCCCAGAGTCACCAAATGACCCCAAGGGTTCCCCATTTGAGAACAAGACTATT
GAACGCACCTTAAGTGTGGAACTCTCTGGAACTGCAGGCCTAACTCCACCCACCACTCCT
CCTCATAAAGCCAACCAAGATAACCCTTTTAGGGCTTCTCCAAAGCTGAAGTCCTCTTGC
AAGACTGTGGTGCCACCACCATCAAAGAAGCCCAGGTACAGTGAGTCTTCTGGTACACAA
GGCAATAACTCCACCAAGAAAGGGCCGGAGCAATCCGAGTTGTATGCACAACTCAGCAAG
TCCTCAGTCCTCACTGGTGGACACGAGGAAAGGAAGACCAAGCGGCCCAGTCTGCGGCTG
TTTGGTGACCATGACTATTGCCAGTCAATTAATTCCAAAACAGAAATACTCATTAATATA
TCACAGGAGCTCCAAGACTCTAGACAACTAGAAAATAAAGATGTCTCCTCTGATTGGCAG
GGGCAGATTTGTTCTTCCACAGATTCAGACCAGTGCTACCTGAGAGAGACTTTGGAGGCA
AGCAAGCAGGTCTCTCCTTGCAGCACAAGAAAACAGCTCCAAGACCAGGAAATCCGAGCC
GAGCTGAACAAGCACTTCGGTCATCCCAGTCAAGCTGTTTTTGACGACGAAGCAGACAAG
ACCGGTGAACTGAGGGACAGTGATTTCAGTAATGAACAATTCTCCAAACTACCTATGTTT

ATAAATTCAGGACTAGCCATGGATGGCCTGTTTGATGACAGCGAAGATGAAAGTGATAAA
CTGAGCTACCCTTGGGATGGCACGCAATCCTATTCATTGTTCAATGTGTCTCCTTCTTGT
TCTTCTTTTAACTCTCCATGTAGAGATTCTGTGTCACCACCCAAATCCTTATTTTCTCAA
AGACCCCAAAGGATGCGCTCTCGTTCAAGGTCCTTTTCTCGACACAGGTCGTGTTCCCGA
TCACCATATTCCAGGTCAAGATCAAGGTCTCCAGGCAGTAGATCCTCTTCAAGATCCTGC
TATTACTATGAGTCAAGCCACTACAGACACCGCACGCACCGAAATTCTCCCTTGTATGTG
AGATCACGTTCAAGATCGCCCTACAGCCGTCGGCCCAGGTATGACAGCTACGAGGAATAT
CAGCACGAGAGGCTGAAGAGGGAAGAATATCGCAGAGAGTATGAGAAGCGAGAGTCTGAG
AGGGCCAAGCAAAGGGAGAGGCAGAGGCAGAAGGCAATTGAAGAGCGCCGTGTGATTTAT
GTCGGTAAAATCAGACCTGACACAACACGGACAGAACTGAGGGACCGTTTTGAAGTTTTT
GGTGAAATTGAGGAGTGCACAGTAAATCTGCGGGATGATGGAGACAGCTATGGTTTCATT
ACCTACCGTTATACCTGTGATGCTTTTGCTGCTCTTGAAAATGGATACACTTTGCGCAGG
TCAAACGAAACTGACTTTGAGCTGTACTTTTGTGGACGCAAGCAATTTTTCAAGTCTAAC
TATGCAGACCTAGATTCAAACTCAGATGACTTTGACCCTGCTTCCACCAAGAGCAAGTAT
GACTCTCTGGATTTTGATAGTTTACTGAAAGAAGCTCAGAGAAGCTTGCGCAGGTAA

The sequence of Elovl5 cDNA comprises SEQ ID No. 19:
>ENST00000304434.11 ELOVL5-201 cdna:protein_coding

ATGGAACATTTTGATGCATCACTTAGTACCTATTTCAAGGCATTGCTAGGCCCTCGAGAT
ACTAGAGTAAAAGGATGGTTTCTTCTGGACAATTATATACCCACATTTATCTGCTCTGTC
ATATATTTACTAATTGTATGGCTGGGACCAAAATACATGAGGAATAAACAGCCATTCTCT
TGCCGGGGGATTTTAGTGGTGTATAACCTTGGACTCACACTGCTGTCTCTGTATATGTTC
TGTGAGTTAGTAACAGGAGTATGGGAAGGCAAATACAACTTCTTCTGTCAGGGCACACGC
ACCGCAGGAGAATCAGATATGAAGATTATCCGTGTCCTCTGGTGGTACTACTTCTCCAAA
CTCATAGAATTTATGGACACTTTCTTCTTCATCCTGCGCAAGAACAACCACCAGATCACG
GTCCTGCACGTCTACCACCATGCCTCGATGCTGAACATCTGGTGGTTTGTGATGAACTGG
GTCCCCTGCGGCCACTCTTATTTTGGTGCCACACTTAATAGCTTCATCCACGTCCTCATG
TACTCTTACTATGGTTTGTCGTCAGTCCCTTCCATGCGTCCATACCTCTGGTGGAAGAAG
TACATCACTCAGGGGCAGCTGCTTCAGTTTGTGCTGACAATCATCCAGACCAGCTGCGGG
GTCATCTGGCCGTGCACATTCCCTCTTGGTTGGTTGTATTTCCAGATTGGATACATGATT
TCCCTGATTGCTCTCTTCACAAACTTCTACATTCAGACCTACAACAAGAAAGGGGCCTCC
CGAAGGAAAGACCACCTGAAGGACCACCAGAATGGGTCCATGGCTGCTGTGAATGGACAC
ACCAACAGCTTTTCACCCCTGGAAAACAATGTGAAGCCAAGGAAGCTGCGGAAGGATTGA

The sequence of Slc2a4 cDNA comprises SEQ ID No.20:
>ENST00000317370.13 SLC2A4-201 cdna:protein_coding

ATGCCGTCGGGCTTCCAACAGATAGGCTCCGAAGATGGGGAACCCCCTCAGCAGCGAGTG
ACTGGGACCCTGGTCCTTGCTGTGTTCTCTGCGGTGCTTGGCTCCCTGCAGTTTGGGTAC
AACATTGGGGTCATCAATGCCCCTCAGAAGGTGATTGAACAGAGCTACAATGAGACGTGG
CTGGGGAGGCAGGGGCCTGAGGGACCCAGCTCCATCCCTCCAGGCACCCTCACCACCCTC

TGGGCCCTCTCCGTGGCCATCTTTTCCGTGGGCGGCATGATTTCCTCCTTCCTCATTGGT
ATCATCTCTCAGTGGCTTGGAAGGAAAAGGGCCATGCTGGTCAACAATGTCCTGGCGGTG
CTGGGGGGCAGCCTCATGGGCCTGGCCAATGCTGCTGCCTCCTATGAAATGCTCATCCTT
GGACGATTCCTCATTGGCGCCTACTCAGGGCTGACATCAGGGCTGGTGCCCATGTACGTG
GGGGAGATTGCTCCCACTCACCTGCGGGGCGCCCTGGGGACGCTCAACCAACTGGCCATT
GTTATCGGCATTCTGATCGCCCAGGTGCTGGGCTTGGAGTCCCTCCTGGGCACTGCCAGC
CTGTGGCCACTGCTCCTGGGCCTCACAGTGCTACCTGCCCTCCTGCAGCTGGTCCTGCTG
CCCTTCTGTCCCGAGAGCCCCCGCTACCTCTACATCATCCAGAATCTCGAGGGGCCTGCC
AGAAAGAGTCTGAAGCGCCTGACAGGCTGGGCCGATGTTTCTGGAGTGCTGGCTGAGCTG
AAGGATGAGAAGCGGAAGCTGGAGCGTGAGCGGCCACTGTCCCTGCTCCAGCTCCTGGGC
AGCCGTACCCACCGGCAGCCCCTGATCATTGCGGTCGTGCTGCAGCTGAGCCAGCAGCTC
TCTGGCATCAATGCTGTTTTCTATTATTCGACCAGCATCTTCGAGACAGCAGGGGTAGGC
CAGCCTGCCTATGCCACCATAGGAGCTGGTGTGGTCAACACAGTCTTCACCTTGGTCTCG
GTGTTGTTGGTGGAGCGGGCGGGGCGCCGGACGCTCCATCTCCTGGGCCTGGCGGGCATG
TGTGGCTGTGCCATCCTGATGACTGTGGCTCTGCTCCTGCTGGAGCGAGTTCCAGCCATG
AGCTACGTCTCCATTGTGGCCATCTTTGGCTTCGTGGCATTTTTTGAGATTGGCCCTGGC
CCCATTCCTTGGTTCATCGTGGCCGAGCTCTTCAGCCAGGGACCCCGCCCGGCAGCCATG
GCTGTGGCTGGTTTCTCCAACTGGACGAGCAACTTCATCATTGGCATGGGTTTCCAGTAT
GTTGCGGAGGCTATGGGGCCCTACGTCTTCCTTCTATTTGCGGTCCTCCTGCTGGGCTTC
TTCATCTTCACCTTCTTAAGAGTACCTGAAACTCGAGGCCGGACGTTTGACCAGATCTCA
GCTGCCTTCCACCGGACACCCTCTCTTTTAGAGCAGGAGGTGAAACCCAGCACAGAACTT
GAGTATTTAGGGCCAGATGAGAACGACTGA

In one embodiment of the method according to the invention samples of at least two consecutive days of said subject are provided and the amount of gene expression is determined and used for said assessment and/or prediction, preferably at least three samples per day, more preferably at least four samples per day.

Subject matter of the present invention is a method of predicting the individual diurnal athletic performance time(s) of a subject, wherein each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h. The specific times can be chosen based on the individual wake up time. For e.g. for someone who usually wakes up at 11h one would start at 11h.

Subject matter of the present invention is a kit for sampling saliva for use in a method according to the present invention comprising:

- sampling tubes for receiving the samples of saliva, wherein each of the sampling tubes contains RNA protect reagent and is configured to enclose one of the samples of saliva to be taken together with the reagent,
- wherein preferably each of the sampling tubes is labelled with the time point at which the respective sample is to be taken and/or includes an indication about the amount of saliva to be taken for one sample,

The kit may further comprise at least one of a box, a cool pack, at least one form including instructions and/or information about the kit and the method for the subject.

RNA protect agents are known in the art and may be selected from the group comprising EDTA disodium, dihydrate; sodium citrate trisodium salt, dihydrate; ammonium sulfate, powdered; sterile water, wherein a single reagent or a combination of different reagents may be used.

In one embodiment of the kit said sampling tubes are configured to receive a sample of saliva of 1 mL in addition to 1 mL of the RNA protect reagent. The sampling tubes may be at least 2 mL tubes, preferably at least 3 mL tubes, more preferably at least 4 mL tubes, still preferably at least 5 mL tubes. While the size for the tubes of 2 mL would be sufficient, it may be more convenient for collecting the saliva samples if the tubes are bigger, such as e.g. 5 mL tubes. In order to collect the required number of samples, the kit may at least six sampling tubes, preferably at least eight sampling tubes (i.e. at least three and four, respectively, samples for two days).

While the kit is used for collecting the samples, it may be designed to be used also for storage and transport of the samples. For this purpose, it is advantageous to have a cool pack in the kit. For instance, if someone is outside and needs to collect the samples, the samples could be stored at room temperature anyway for a few hours, or if one know that there will be no fridge for the next two days, one could still freeze the cool pack before the sampling, then place the cool pack in the box and sample as needed, since the box would remain cold for several hours (maybe even for two days, depending on the outside temperature). After the sampling is completed, the same box can be used to send the samples back to a lab, if applicable with all the forms inside as well (it may be required to pack the box in a post box for sending, which however may be enough for preparing the kit to be sent).

**Detailed description of the invention**

Exemplary embodiments of the present invention will be explained by way of example with reference to the drawings. In the drawings:

Fig. 1 illustrates the circadian core-clock network;

Fig. 2 illustrates two examples of fits of saliva data to a core-clock mathematical model;

Fig. 3 illustrates time-course measurements of unstimulated saliva show fluctuations in gene expression across 45 hours for two core-clock genes (Bmal1 and PER2) as an example;

Fig. 4 illustrates how gene expression of Arntl (Bmal1) and Akt1 covary. Furthermore, it is depicted that Arntl (Bmal1), Per2 and AKT1 vary in time for the different participants (A). It further shows that the variations in Akt could be correlated with variation in one of the clock genes-Bmall (B). It shows as well that circadian variation in Akt could be measured for the exemplified participants in the saliva.

Fig. 5 illustrates correlations between molecular rhythms of core-clock genes and athletic performance; (A) The peak time of PER2 correlates with the time of peak performance of the HST (linear regression with $p = 0.014$). (B) Performance change over the day (max. compared to min.), colour code as in (C). (C) Black and grey groups have an early and late ARNTL (BMAL1) peak time, respectively. (D) Standard deviation calculated on the normalized HST performance for data from different (i) repetitions and timepoints ($p = 0.0095$), (ii) timepoints ($p = 0.0095$), (iii) repetitions ($p = 0.057$). (E) Separating the groups by the mean expression level of ARNTL (BMAL1) instead of the peak time results in significant differences in the standard deviation of the sports performance of HST and CMJ (left, all $p = 0.0476$) and of the hand muscle frequency (right, $p = 0.0286$, $p = 0.11$, $p = 0.0286$). (F) Histogram of the time of the day with the highest ARNTL (BMAL1) expression based on the eight saliva samples. Significantly earlier peaks are found for the group with low ARNTL (BMAL1) expression (ranksum, $p = 0.044$). (G) Logarithm of ARNTL (BMAL1) expression levels for all sampling times ordered by male and female participants. Males show a significant higher ARNTL (BMAL1) expression compared to females (Welch's t-test, $p < 0.0001$). (H) Logarithm of the ratio of PER2 and ARNTL (BMAL1) expression levels for all sampling times ordered by male and female participants. Females show a significant higher ARNTL (BMAL1) expression compared to males (Welch's t-test, $p < 0.0001$). (I) Early or late ARNTL (BMAL1) peaks occur in any of the three investigated MEQ chronotype.

Fig. 6 illustrates standard deviations of normalized sports and muscle tone data; Standard deviations of normalized sports and muscle tone data (L: group with low *BMAL1,* H: group with high *BMAL1*). Mean standard deviation calculated on the normalized sports performance and the normalized muscle tone data for different (i) repetitions and timepoints, (ii) timepoints, (iii) repetitions (for details see Methods). **(A)** HST, **(B)** CMJ, **(C)** SRT (no repetitions were measured, thus the standard deviation (i) over all data is the same as (ii) over timepoints), **(D)** muscle tone of the leg muscles (*M. rectus femoris, M. biceps femoris, M. gastrocnemius*).

Fig. 7 illustrates a mathematical model extension, in which the core clock genetic network is complemented with additional genes associated to metabolism and sports performance and provides as an output performance variation in time in a personalized manner;

Fig. 8 illustrates an example for a personalized model fit for the core-clock genes (a) and genes important for athletic performance and metabolism (b and c) based on the expression experimental data;

Fig. 9 illustrates the computed prediction result for the athletic performance based on the expression values from Fig. 8.

Fig. 10 illustrates ARNTL (BMAL1) and PER2 expression display variation during the day in human blood, hair and saliva samples. (A) Three time-point comparison of ARNTL (BMAL1) and PER2 expression for the averaged data of all Participants in figure 1. Expression data is compared to the first time-point (Early). For hair and saliva data Early, Middle and Late time-points represent 9h, 17h and 21h, respectively. For PBMCs data Early, Middle and Late time-points represent 10h, 16h and 19h, respectively. Depicted are mean + SEM. (B) Time-course RT-qPCR measurements normalised to the mean of all time points ($\Delta\Delta$CT) of ARNTL (BMAL1) and PER2 of Participant 1, 2, and 13 with a fitted linear sine-cosine function (period = 24 h). For Participant 1, we collected one additional sample at 21h on the 2nd day. Harmonic regression best p-values for tested periods (20-28h): Participant 1; ARNTL (BMAL1) (0.517, period = 21.4h), PER2 (0.353, period = 24.0h). Participant 2; ARNTL (BMAL1) (0.038, period = 20.0h), PER2 (0.276, period = 28.0h). Participant 13; ARNTL (BMAL1) (0.014, period = 20h), PER2 (0.086, period = 21.4h). (C) Time-course RT-qPCR measurements of human PBMCs normalised to the mean of all time points (ΔΔCT) of BMAL1 CLOCK, NPAS2, PER2, CRY2, NR1D1 and RORB of Participant 2 and 5 with a fitted linear sine-cosine function (period = 24 h). Harmonic regression best p-values: Participant 2; BMAL1 (3.05E-01, period = 20h), CLOCK (6.31E-02, period = 28h), NPAS2 (1.67E-01, period = 20h), PER2 (4.78E-04, period = 20.8h), CRY2 (7.17E-01, period = 20h), NR1D1 (1.48E-01, period = 28h) and RORB (7.58E-01, period = 20h). Participant 5; BMAL1 (5.56E-01, period = 20h), CLOCK (6.81E-01, period = 28h), NPAS2 (9.75E-02, period = 28h, PER2 (1.23E-01, period = 28h), CRY2 (5.40E-01, period = 28h), NR1D1 (6.43E-01, period = 28h) and RORB (7.73E-01, period = 28h). (D) Average PER2 expression compared to BMAL1 using saliva time-course data for each participant (mean + SEM).

Fig. 11 illustrates HST base line measurements.

Fig. 12 illustrates Myotonometric analysis shows daily variation in muscle tone (frequency, F) for female and male participants. Only participants who completed all training sessions were included in the MyotonPRO measurements (N=12). Mean of normalized scores for the myotonometric parameter frequency [Hz] for each training session (T1-9h, T2-12h, T3-15h, T4-18h) and each muscle: M. Deltoideus, M. Triceps Brachii, M.adductor pollicis, M. rectus femoris, M. biceps femoris, M. gastrocnemius. The measurements were carried out from top to bottom on the right (Right bar) and the corresponding left (Left bar) side of the body.

The aim of the invention is to predict, optimal timing of behavior, more specifically the timing of best sports performance, possibly to monitor (over time) the circadian rhythms and adjust the timing it if needed. Previous studies focused on predicting the circadian time which means a 24hours-rhythm. However, given that the prediction of the circadian time is in an application used for a second prediction, the prediction of the timing of behavior, the error accumulates with each prediction. The present invention instead relies on a direct measurement of the behavioral relevant timing directly based on the genetic expression. That means if the genes have a 20h, or 30h or 12h rhythm in expression, the method of the present invention would also be able to detect that. These would be non-circadian rhythms and include infradian and ultradian rhythms. Thus, the present invention assesses and monitors the circadian profile. The circadian profile could be a circadian or non-circadian rhythm.

According to the present invention "assessing the circadian or non-circadian rhythm" or "assessing the athletic performance" also includes "monitoring the circadian or non-circadian rhythm" or "monitoring the athletic performance". "Monitoring" means at least twice "assessing".

As an objective measure, gene expression may be quantified four times a day (the times mentioned in this disclosure serve as an e.g. of possible sampling times), two days in a row. In particular, four samples of saliva may be taken on two consecutive days, and the gene expression of selected genes in accordance with the present invention is determined in each of the samples. While other studies have focused on a prediction of circadian time (exact estimation of precise internal time), with the aim to allow for a subsequent prediction of the optimal time for a behavior, such as high sports performance, the present invention focuses on a direct prediction of the relevant timing including a circadian profile, without the deviation through circadian time. This means previous studies attempted to tell the exact internal time. The present invention provides a full 24h profile, it may provide a 48h profile, if measured during two consecutive days, each day e.g. 4 saliva samples are taken. If more samples are taken over more days longer profiles may be provided.

The computational analysis of the measured gene expressed obtained from the saliva samples as set forth above can be separated into three different approaches, which will be discussed in the next sections. First, experimental data can be fitted with a periodic function, in order to establish oscillatory behavior and extract oscillation properties. Machine learning can then be used to predict the timing of behavior based on the gene expression. Modeling the molecular network underlying the circadian rhythm as well as the behavior under consideration can add information. Background and general considerations in view of the present invention and the overall process will be explained first. Following, the procedure according to the present invention will be described with respect to the specific application.

A general problem in chronobiology is the screening for circadian oscillations in data, such as in the series of eight data points obtained from the saliva samples. It has to be determined whether the observed variation is due to some circadian rhythm, or only due to noise. To distinguish oscillating from non-oscillating measures, a periodic, non-constant function is fit to the data, and if the fit is significant, the measure is considered oscillatory. Successful fits allow to read off the oscillation phase, amplitude and period. Fitting the oscillatory data by curve fitting is described below.

If a trigonometric function is fit to the data, this is called harmonic regression, which may be done as set forth in the following. It will be appreciated that this is described by way of example only. Below, other approaches are briefly outlined. Circadian rhythmicity of genes may be tested (significance e.g. bounded by a fit with p-value $< 0.05$) and circadian parameters (phase and relative amplitude) may be determined for sample sets with at least 7 data points (3 hours sampling interval) for a period range of 20 to 28 hours with a 0.1 hour sampling interval by fitting a linear sine-cosine function to the time-course data (ΔΔCT normalized to the mean of all time points), for instance using known tools, e.g. the R package HarmonicRegression (Luck et al. 2014). The harmonic regression procedure fits the model y(t) = m + a·cos(ωt) + b·sin(ω) in order to estimate absolute amplitudes (A = √(a2 + b2)) and phases (φ = a·tan2(b,a)) along with confidence intervals and p-values (Luck et al. 2014). The fit uses a least-squares minimization. Extensions to this fit method are reviewed in as cosinor-based rhythmometry in (Cornelissen 2014).

A combination of sine waves are also used by other rhythmicity detection methods (Halberg et al., 1967; Straume, 2004; Wichert et al., 2004; Wijnen et al., 2005; Thaben and Westermark, 2014). Yet, Fourier-based methods can have the drawback that they require evenly sampled data. Other alternatives are named in the following. It will be appreciated that the invention is not limited to these packages but any other suitable method for fitting a periodic function to the measured gene expression data may be applied.

The software-packages RAIN (a robust nonparametric method for the detection of rhythms of prespecified periods in biological data that can detect arbitrary wave forms (Thaben and Westermark 2014), which improves on older methods: a nonparametric method implemented as the program "JTK_CYCLE", which assumes symmetric curves (Hughes et al., 2010), as well as its improvement eJTK_CYCLE that includes multiple hypothesis testing and more general waveforms (Hutchison et al. 2015)[Ref: Hutchison AL, Maienschein-Cline M, Chiang AH, et al. Improved statistical methods enable greater sensitivity in rhythm detection for genome-wide data. PLoS Comput Biol 2015;11:e1004094.], while the HAYSTACK method (Michael et al., 2008) can also detect chain saw type rhythmicity, but relies on a small set of predefined wave form alternatives and is thus not really general.

BIO_CYCLE: "We first curate several large synthetic and biological time series datasets containing labels for both periodic and aperiodic signals. We then use deep learning methods to develop and train BIO_CYCLE, a system to robustly estimate which signals are periodic in high-throughput circadian experiments, producing estimates of amplitudes, periods, phases, as well as several statistical significance measures." (Agostinelli et al. 2016).

A modified version of the empirical Bayes periodicity test to detect periodic expression patterns (Kocak and Mozhui 2020). Their results demonstrate that this approach can capture cyclic patterns from relatively noisy expression data sets.

Especially to find higher harmonics, some studies have exploited Fisher's G-test and COSOPT jointly to recognize rhythmic transcripts, classified, depending on the length of the oscillation period, as circadian (24 ± 4 h) and ultradian (12 ± 2 h and 8 ± 1 h) (Hughes et al. 2009; Genov et al. 2019).

Once the curve fitting to the measured data points has been done, machine learning methods are applied to predict circadian time for human subjects, which has in principle been proposed by several studies. Some example studies are outlined to provide background for the process of the present invention, which will be described in detail thereafter.

While the present invention focusses on studies based on gene expression, continues measures of light exposure and skin temperature as well as metabolites from blood or breath sampling can be used to predict circadian time (Kolodyazhniy et al. 2012; Kasukawa et al. 2012; Sinues et al. 2014). Also skin temperature in combination with questionnaires and activity measurements can predict circadian time, by a method called INTime (Komarzynski et al. 2019). The following studies predict circadian time or time-of-the-day from gene expression data extracted from human blood: BioClock (though only mouse data so far) (Agostinelli et al. 2016): Normalization is Z-score data (subtraction of mean and then divided by standard deviation - this removes any amplitude information), their method is a deep neural network, they use BioCycle to derive rhythmicity, and standard gradient descent with momentum to train the network, the original publication uses different tissues but only from mice.

ZeitZeiger (Hughey 2017): Data normalized and batch-normalized. Discretized and scaled spline fits are used to calculate sparse principal components (SPC), predictions based on fitted splines to SPC with maximum likelihood. They use 15 genes from human blood, only two of those are part of the core-clock. Due to the batch-normalization, the incorporation of new data requires retraining of the algorithm, their algorithm was improved for humans. One sample is enough for predictions.

Partial least squares regression (PLSR) (Laing et al. 2017): Training data is batch-corrected and quantile normalization is applied. No batch correction on test set, to prevent the need for retraining whenever new data is added. Their algorithm uses 100 genes out of 26,000 available ones from blood. One sample is enough for predictions, more is better.

TimeSignature (Braun et al. 2018): Mean-normalized genes, algorithm is optimized with a least squares approach plus elastic net for regularization. They use 40 genes from two samples of blood, 12 h or less apart. This study seems to generalize well, it was validated in 3 studies, one of them with a different experimental method to measure gene expression.

BodyTime (Wittenbrink et al. 2018): ZeitZeiger (see above) + NanoString platform (an experimental machine which allows for high quality counts of gene abundance without the need of an amplification step, thus it measures the original abundances). They use 12 genes from human blood, but also get good predictions for as few as 2 genes (one of which is PER2 which also is used in the sports study). Their algorithm is validated in 1 independent study that uses the same method.

TimeTeller, preprint (Vlachou et al. 2020): They aim to predict clock functionality from a single gene sample. Application to breast cancer, showing that their prediction relates to patient survival. Rhythmicity and synchronicity were analysed to choose a set of 10-16 genes used for the prediction (all core-clock or clock-controlled). Their algorithm is trained with a set of repeated samples and extracts from them the probability to observe a particular gene expression profile given some time t. The prediction inverts this information; they use a maximal likelihood function to predict for a given gene expression profile the time t. A model of the core-clock was used to test their algorithm.

Machine learning can be used to predict some output based on a (high-dimensional) input consisting of a set of so-called features, i.e. the different dimensions of the input space. A set of input-output pairs is used to train the algorithm, i.e. the algorithm performs some kind of optimization that allows it to optimally predict the output based on the input. This set is called training set. To evaluate the performance of the algorithm, it is fed with an independent set of inputs from a so-called test set, while not presenting the associated outputs. The predictions of the algorithm are then compared to the associated outputs, and the number of correct predictions is counted. Several measures can be used to quantify prediction quality; for instance the accuracy, i.e. the number of correct predictions divided by the total number of predictions, may be used.

For small data sets, as typical with human subjects, the separation of the data into two independent training and test sets means that it would not be taken advantage of the full amount of information available for the prediction. The solution is cross-validation, for which the total set is repeatedly separated into different training and test sets. Especially for very small data sets, one can use all but one subjects to form the training set, and test the algorithm only on the left-out subject. This is called leave-one-out cross-validation (sometimes also leave-one-subject-out cross-validation). Given n subjects, the training on all-but-one subjects is repeated n times, such that each subject has been once selected as test set. The accuracy of the prediction is in this case calculated as the number of correct predictions over n.

While the application of machine learning to genetic data is generally known, the benefits and value of the results highly depend on the input data. Thus, the inventors put their focus on the input of the data, both via normalization and presentation of derived features, and also on understanding in detail what information the algorithm uses. Most published studies focus on their machine learning algorithm, why it is best suited for the prediction at hand, while mentioning their data preparation only on the side, and their discussion of the workings of the algorithm is often restricted to a single evaluation approach (for example, showing that a restricted set of genes is most relevant for prediction, but without stating which characteristics of the genes are important).

When comparing the performance of different machine learning algorithm on standard training and test sets, their performance differs often just in a few percent - an order of magnitude hardly relevant for biological data, which often consists of only few samples with a high level of noise compared to other typical machine learning applications. It has been found that the particular algorithm will not make much difference, but what makes a large difference is the way in which the input is prepared for machine learning. Many machine-learning algorithms are optimized for data with zero mean and a standard deviation of one for each dimension individually. Dimension-wise normalization makes however no sense for time-series data, where dimensions are not independent of each other, but where the information on the temporal development can only be accessed by comparing different dimensions.

As mentioned above, eight saliva samples may be collected, preferably distributed over the day, e.g. at 9h, 13h, 17h and 21h over two consecutive days. This results in a feature space with 8 dimensions. Instead of normalizing each of these dimensions independently over all subjects, the data may be normalized by their temporal mean for each subject independently. In addition, this subject-wise mean normalization of each gene has the advantage of keeping the temporal structure of the data intact (phase and relative amplitude of the oscillation), thus preserving this information for the machine learning algorithm. Yet, what is lost by this normalization is the mean values of the oscillations, and thus also their relative expression mean. To preserve this information for the machine-learning algorithm, this may be added as additional features to the feature space. Subject-wise normalization has to be reconsidered when the molecular profile of a subject was measured repeatedly over a longer interval of time. For the example of multiple measurements during disease progression, we have already published one possibility to normalize data from subsequent molecular profiles such that the result can be compared between sampling dates and even between different experimental procedures (Yalçin et al. 2020).

In many cases, machine learning algorithms are considered as "black boxes". However, as the machine learning algorithms are faced with noisy biological data, derived of a system where lots of additional information are available, the approach of the present disclosure is to let the machine learning optimize the prediction, but then to uncover the underlying information flow from input to prediction output, with the aim to double-check the generalizability of the solution found by the machine learning. Optimally, any additional information can be added as either input to the machine learning or as constraints (in form of a cost function), but in a first step, the formulation of these inputs and constraints is more difficult than to take the algorithm and check a posteriori whether any additionally known information is violated.

Once the prediction was done using the complex feature space created in the step explained above, a simplification of the feature space may be carried out. This serves to identify the relevant features. For example when predicting the optimal sports time it may be tested whether the peak time of the genes would suffice for the prediction. It was found that this was not the case, i.e. the algorithm uses more than this information. In general, dimensionality reduction methods may be used first, which results in fewer, new features that are combinations of the original features. Then it may be tested which combinations of individual original features is sufficient for successful predictions, and compare whether that fits the features which are dominant in the features resulting from dimensionality reduction. This is an important step to understand based on which information the prediction is made by the algorithm, which is relevant to double check its generalizability to new data.

Related to the first point, machine-learning algorithms are preferred which may be called interpretable, i.e. they provide some information on the prediction. Examples for such algorithms are sparse principle components analysis as used as an intermediate step in (Hughey 2017), and partial least squares regression, as used in (Laing et al. 2017). In both cases, the prediction is made based on a combination of the features into few most informative features, and it is for example possible to plot two of them against each other in order to see how the data of the training set and test set is distributed in these features. It is expected that subjects with optimal times that are neighboring are also neighbors in this component space. If this is not the case, the algorithm is unlikely to generalize well.

Then, prediction performance may be benchmarked using a neural network model, which may be used as an approximation for an upper border of prediction performance. Neural networks do not require normalization, as they are universal computing machines and can hence implement the optimal normalization for the problem at hand on their own. However, this is at the same time the problem with neural networks. As they decide for themselves which are the relevant features of the data, there is no controlling whether they use biologically relevant information, or noise information that - by chance - fits the prediction. Furthermore, their high flexibility facilitates overfitting of the data and the resulting algorithm are difficult to interpret, such that we cannot a posteriori enhance our trust in the method by understanding the information flows from input to prediction output. Despite these disadvantages, neural networks may be used at least as benchmark algorithms, to test which performance can be expected when the information is provided without constraints. The present invention aims at providing an algorithm with a performance similar to that of the neural network, but not by means of overfitting the experimental data, as suspected for the neural network, but by means of focusing on the biologically relevant information.

A linear support-vector-machine (SVM) can be used to predict two different outputs based on a high-dimensional input data (see below for details). Linear SVMs are extremely simple compared to the non-linear methods explained above. They have the advantage of a fast implementation, and, as their complexity is low, they are not so prone to overfitting. For these reasons, a linear SVM may be used to predict the optimal sports timing, and it turned out that this was sufficient for prediction. However, it is noted that the prediction problem was "linearly separable", and as there is no reason to assume that any application is "linearly separable", it may be preferable to use in general non-linear methods. Yet, testing how well a linear model performs compared to the non-linear model can help to benchmark how much complexity is needed for the prediction. For example, if a linear model results in an accuracy of 0.85 and a non-linear model in an accuracy of 0.9, it is probably not worth using the non-linear model for the application, as it performs only slightly better on the test set, but has a larger probability of overfitting the data, which might lead to less performance on a new set of data. If the difference is larger, a non-linear model is likely more appropriate.

As mentioned above, linear support-vector-machine (SVM) can be used to predict two different outputs based on a high-dimensional input data. For training, the linear SVM is fed with multi-dimensional input data and a binary output. The training set consists of n subjects, and the input with *p* dimensions is denoted as $x_i \in R^p$, \$i. The output $y_i$ is encoded as -1 for the first type of output and as +1 for the second type of output, $y \in 1, -1^n$. The training of the SVM fits a hyper-plane into the input space such that it separates the two output types as best as possible and such that the distance to the input data points is maximal.

Mathematically, the following minimization problem is solved:

$$\min_{w,b} \frac{1}{2} w^T w + C \sum_{i=1} \max\left(0, y_i(w^T \phi(x_i) + b)\right),$$

where $\phi$ is the identity function, $(w^T\phi(x_i) + b)$ is the predicted output for the *i*st input. For the application to the sports data, the regularization constant C is set to 1.0 (default of the python implementation).

Predictions for some input $x_{test}$ then be calculated as $w^T\phi(x_{test}) + b$ with the *w* and b resulting from the above minimization, and compared with the correct output. Leave-one-subject-out cross-validation implies that this step is repeated n times, each time with another participant removed to form the training set.

With regards to the data obtained from the saliva samples, in accordance with an example of the present invention, the machine learning requires optimally eight timepoints, four is less optimal: Using the two-day measurement of *PER2,* consisting of eight data points, a linear support vector machine can predict early versus late HST sports performance with an accuracy of 1.0 (100% correct predictions). The accuracy drops to 0.8 or 0.4 (80% or 40% correct predictions), if the prediction is based on only the first or the second day with four data points each (as the machine learning cannot handle missing data points, those are thereby filled with appropriate values: the expression of PER2 is set to zero if ARNTL (BMAL1) was measured successfully while there was too little PER2 to be detectable in the experiment, and the value of the other day was used if the whole measurement was unsuccessful).

The present invention provides a methodology for the detection of circadian rhythms based on saliva sampling, which is introduced as a non-invasive and practical approach. While this methodology may be particularly beneficial for future sports studies, it may be useful for more general applications and for anyone, for example anyone who just wants to know or to follow up their circadian profile e.g. across the years or across the seasons. The methodology relies on the fact that ARNTL (BMAL1) and PER2 expression shows daily changes in human blood, hair and saliva cells, which are distinctive for every individual tested. Also sport performance displays daily variations, e.g. between 09h, 12h, 15h, and 18h, and peak performance is time-of-day dependent, with different optimal timing for strength exercises compared to endurance exercises. Biomechanical muscle properties in resting muscles undergo daily fluctuations, which correlate with sport performance and clock gene expression variations in saliva. Therefore, the method of the present invention utilizes salivary gene expression of ARNTL (BMAL1) and PER2 as personalized predictors of athletic fluctuations and individual peak times in performance.

The sample collection can be performed in almost any location. The samples of saliva are collected at the predetermined points in time in a tube containing an RNA stabilizing reagent followed by RNA extraction as described below. In order to minimize RNA degradation through material transfer, according to a preferred exemplary method an amount of 1mL of unstimulated saliva may be collected directly into a 5mL Eppendorf tube containing 1mL of a non-toxic RNA-stabilizing reagent called RNAprotect Tissue Reagent (Qiagen) which should be mixed immediately to stabilise the saliva RNA. The direct addition of saliva to the RNA stabilizing reagent, which is mixed immediately, was found to generate good quality/quantity RNA suitable for gene expression analysis and by using 5mL tubes instead of 2mL tubes (wider opening for sample collection), the sampling procedure was more comfortable to perform. Other tested sampling protocols had shown to lead to poor quality and quantity of RNA that was not suitable for the downstream application. For example, 200μL saliva was collected in a 50mL tube on ice, which was immediately transferred to a 2mL tube containing 1mL RNA stabilizing reagent followed by RNA. In another protocol, 1000μL saliva was collected in a 50mL tube and processed as described above, in which the extracted RNA did not pass the desired quality/quantity either.

With only four sampling time-points per day (9am, 1pm, 5pm and 9pm) and over two consecutive days, it is possible to assess precise circadian rhythms in gene expression of any individual. For best sampling quality, the individuals should refrain from eating and drinking one hour prior to sample collection. Individuals can optionally wash their mouths with water five minutes before sampling without swallowing the water. The stabilized samples can be kept at room temperature for a few days, optimally at 4 □ C during several weeks, for posterior molecular analyses via different possible methods, such as RT-qPCR, Nanostring, microarrays, and sequencing. In one embodiment any other method known by a person skilled in the art to measure gene expression could be used. One could of course do the same with protein expression instead of gene expression in principle.

A method for RNA extraction from saliva samples is provided to effectively extract RNA, preferably using TRIzol (Invitrogen, Thermo Fisher Scientific) and the RNeasy Micro Kit (Qiagen). It has proven to be particularly beneficial to use a combination of both, rather than only one of them (typically either TRIzol or RNeasy Kit is used). For this, the samples were centrifuged at 10,000 x g for 10 min at room temperature to generate cell pellets. The supernatant was removed and the pellets were homogenized with 500μL TRIzol followed by the addition of 100μL chloroform and mixed for 15sec at room temperature. After a 2min incubation at room temperature, the samples were centrifuged at 12,000 x g for 15min at 4°C. The mixture will separate into a lower red phenol-chloroform phase, an interphase, and a colourless upper aqueous phase. The upper aqueous phase contains the RNA, which was transferred into a new 2.0mL microfuge tube using a 1ml pipette with filtered tip, being careful not to transfer any of the interphase layer. After the transfer, the samples were processed according to the manufacturer's instructions of the RNeasy Micro Kit (Qiagen) on a QIAcube Connect device (Qiagen). Finally, the RNA is eluted in RNA-free water and can be used directly for gene expression analysis. It has been developed the secondary purification and elution step of the saliva RNA using RNeasy Micro Kit in order to:

a) Increase sample quality and purity, which is necessary for downstream applications and is otherwise lost with the traditional and commercial methods, since the RNA content in saliva is low.
b) Automate the sample processing and RNA extraction using the QIAcube Connect automation device. With this, it is possible to perform sample handling much faster and reduce sample contamination induced by human errors.

In one embodiment, gene expression analysis is carried out via cDNA synthesis and RT-PCR as follows. For RT-qPCR analysis, the extracted RNA is reverse transcribed into cDNA using M-MLV reverse transcriptase (Invitrogen, Thermo Fisher Scientific), random hexamers (Thermo Fisher Scientific) and dNTPs Mix (Thermo Fisher Scientific). RT-PCR is performed using SsoAdvanced Universal SYBR Green Supermix (Bio-Rad laboratories) in 96-well plates (Bio-Rad laboratories). The RT-PCR reaction is performed using a CFX Connect Real-Time PCR Detection System (Bio-Rad laboratories) using primers from QuantiTect Primer Assay (Qiagen) as well as custom made primers.
The experimental data obtained from the saliva samples as explained above will be further analysed with a computational model in order to provide scientifically justified and personalized suggestions for best timing of sports (wherein applications for other certain daily activities, such as light exposure, sleep, food and medicine intake may be envisioned), to avoid circadian rhythm disruption, and thus enhancing health. As will be described in detail below, a mathematical model for the circadian clock is created, which may include core-clock and clock-controlled metabolic genes in about 50 elements, based on which models for relevenat gene networks, particularly related to physical performance in connection to the circadian clock can be generated. By feeding each network with specific gene expression data obtained from saliva samples, accurate predictions for day-/night-time activities can be generated.
Based on the experimental data from the saliva samples, more specifically the measured gene expressions and the resulting fitted oscillatory curves, a core-clock model will be generated, which may include a larger number of other genes that were not included in the measurements but that may be relevant for the desired prediction (this model is also referred to as "network computational model" in this disclosure). The core-clock is located in the brain (suprachiasmatic nucleus) and its oscillations entrain the peripheral clocks. The oscillations result from feedback loops, which can be investigated by experimental and theoretical means.

Rather than relying only on a model for the circadian rhythm that simply shows oscillations such as phase-oscillators, the present disclosure uses a molecular model, which models (part of) the molecular interactions underlying the circadian clock. This is because, as already mentioned, molecular models contain biological information that might be useful for predictions. Molecular models with simple feedback loops models are often based on Goodwin's oscillator, e.g. (Ruoff and Rensing 1996), but the level of detail may also be extensive (Forger and Peskin 2003). According to an exemplary embodiment of the present invention, a model at an intermediate state of complexity is generated, complex enough to capture a significant part of the genetic network, but not too complex, as this may affect fitting of the data to the model without significant overfitting. Relogio et al. have published a model at this level of complexity, with 19 dynamical variables, which is used in the following (Relógio et al. 2011).

Now referring to Fig. 2, two examples of fits of the saliva data to the core-clock model are shown. (Relógio et al. 2011). Left: Saliva data is plotted as dots, including data for ARNTL (BMAL1) and PER2, where the measurements of both measured days within the same 24 hours are plotted, which is then plotted for two consecutive days. The curves result from the model fit. Middle and right: The model contains 19 dynamical variables, 17 are plotted in these two panels. Fig. 2 illustrates that the dynamical model may restrict the shape of the fitted curves. In this exemplary embodiment, the curves are more complex than a simple sine-cosine function, but they are also not perfectly fitted to the data, as may happen when a spline is used to fit the data, because the model can only produce shapes that result from the interacting dynamics.
The data base for the fit are the experimental, non-logarithmic gene expression values, $2^{\Delta CT}$. In order to get the experimental values on the same scale as the model output (which is on the order of one), the gene expression of both PER2 and ARNTL (BMAL1) are normalized in this exemplary embodiment by the mean of ARNTL (BMAL1) expression; that way the relative amplitude of both genes is preserved. In order to allow for a fairer comparison both the simulated and the experimental data may be normalized by their respective mean ARNTL (BMAL1) expression.

The complexity of the model with around 80 parameters makes a meaningful fit that prevents overfitting challenging. At least one of or a combination of one or more (including all) of the following approaches may be used to fit the model to the saliva data. It will be appreciated that other approaches may be used alternatively or in addition to adjust the model. To constrain the model to parameter regions in which continuous oscillations occur, a bifurcation analysis may be used to delineate the regions with limit cycles (i.e. continuous oscillations), and restrict the parameter optimization to these regions. This prevents fits that show a (slow) relaxation to a steady state, as the model may be expected to have a stable limit cycle. Considering the bifurcation structure, fits will be faster because less parameters need to be checked and because less simulation time is required to ensure relaxation of the oscillatory behavior.

To minimize the number of parameters with large deviations from the original model, standard regression methods may be used that are also added to the cost function, such as ridge regression, which has proven useful so far.

Based on biological and dynamical considerations, certain parameters may be fixed at the original value and exclude them from the fit. This may be for example done for parameters that show only minor impact on the resulting curves, parameters for which no inter-individual variation is expected (i.e. diffusion constants which result from biochemical properties) and parameters which have been repeatedly measured in experiments for humans.

Finally, least-squares minimization (details see below) may be used to minimize the distance between experimental data and fitted curve. The associated cost function used for least-squared error minimization may be extended with additional terms that can restrict the period (should be between 20 and 28 hours for human material), amplitude (no constant amplitude, e.g.) and the position of peaks and troughs.

According to an exemplary fitting procedure, the two days of gene expression data are interpreted as replicates, and the model is fitted to both data points for 9h, 13h, 17h and 21h at the same time, as indicated by two data points for each time point in the above figure. In order to fit the model to the gene expression data, the model may be run for 72 hours with a time resolution of 0.01 hours. The last 48 hours are used for the analysis. As model and experiments have no common time, all possible time-shifts between experiments and model output are considered (0 up to 24 hours). For each shifted variant of the model output, the least-squares cost function *C* may be calculated between the experimental values $x_{exp}$ and the model output $x_{mod}$ as $C_0 = 2\sqrt{1 + \left(x_{exp} - x_{mod}\right)^2 - 2}$ for both genes (ARNTL (BMAL1) and PER2), and then the time-shift with the minimal summed cost for both genes may be selected. To optimize the fit, a selection of the following additional cost function terms may be added to $C_0$:

- A regression term, that penalizes large parameters: Given the parameter vector $p = (p_i)$ where *i* is between 1 and the number of parameters, ridge regression adds a term $C_{ridge} = c\sum p_i^2$ to the cost function, with a prefactor c that was set to 1.
- A term that penalizes deviating periods. One may first measure the period *p* of the model, and then compare it to the standard human circadian period of 24.5 hours: $C_p = c_p(p - 24.5)^2$, with weighting factor $c_p = 1$.
- A term that penalizes the amplitude deviations: For experimental and simulated amplitudes $A_{exp}$ and $A_{sim}$, the cost function is $C_a = C_a(A_{sim} - A_{exp})^2$, with weighting factor $c_a = 0.05$.
- A term that penalizes if the peak or trough position deviates. Peak times of the experimental data $t_{exp}$ and of the model trace with the optimal time-shift applied $t_{sim}$ may be derived and the cost term calculated as $C_{top} = c_t(p - 24.5)^2$, with weighting factor $c_t = 0.1$ for the peaks. The same may be done for the trough, resulting in a cost $C_{down}$, with weighting factor $c_d = 0.05$.

The cost function sums the individual costs weighted by a factor chosen to optimize the influence of each cost. $C_{total} = C_0 + C_{ridge} + C_p + C_a + C_{top} + C_{down}$.

In one embodiment the present invention provides a method of assessing the circadian rhythm or circadian profile of a subject and/or assessing and predicting the athletic performance of said subject, wherein said method comprises the steps of:

- Providing at least three samples of saliva, more preferably four samples of saliva, from said subject, wherein said samples have been taken at different time points over the day,
- Determining gene expression of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, in each of said samples, and
- Assessing and predicting by means of a computational step based on said expression levels of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, over the day the circadian rhythm of said subject and/or the individual diurnal athletic performance times.

In a second embodiment the present invention provides a method wherein the gene expression is determined using a method selected from quantitative PCR (RT-qPCR), NanoString, sequencing and microarray.

In another embodiment the present invention provides a method wherein the gene expression is determined using quantitative PCR (RT-qPCR).

In another embodiment the present invention provides a method wherein the gene expression is determined using NanoString.

In another embodiment the present invention provides a method which allows assessing the circadian rhythm of said subject comprises determining a periodic function for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, that approximates said expression levels for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2,, preferably comprising curve fitting of a non-linear periodic model function to the respective expression levels, wherein the curve fitting is preferably carried out by means of harmonic regression.

In a further embodiment the present invention provides a method wherein the computational step comprises

- processing the determined expression levels and/or the respectively fitted periodic functions to derive characteristic data for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, said processing comprising determining the mean expression level of expression of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and normalizing the expression levels using the mean expression level.

In a further embodiment the present invention provides a method wherein said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes., and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

In a further embodiment the present invention provides a method wherein from the characteristic data only the timing of the peak expression level of PER2 and the mean expression level of BMAL1 are used in said computational step.

In a further embodiment the present invention provides a method wherein the computational step further comprises

- fitting a network computational model to the derived characteristic data that comprises a representation of the periodic time course of the expression levels for each of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, as well as a representation of the periodic time course of the expression level for at least one, preferably a plurality of further gene(s) included in a gene regulatory network that includes said at least two members the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2,; and/or

- training a machine learning algorithm on the derived characteristic data of the network computational model, particularly optimize in terms of the representation of the periodic time course of the expression level for the at least one further gene.

In another embodiment the present invention provides a method which allows assessing and/or predicting the individual diurnal athletic performance times comprises in the computational step fitting a prediction computational model on data obtained from said fitted periodic functions and/or said network computational model, wherein the prediction computational model is based on machine learning, including at least one classification method and/or at least one clustering method wherein said method(s) are preferably selected from the group comprising:
K-nearest neighbor algorithm, unsupervised clustering, deep neural networks, random forest algorithm, and support vector machines.
In a further embodiment the present invention provides a method wherein additional physiological data of the subject are provided for fitting the prediction computational model.
In a further embodiment the present invention provides a method wherein the oscillation amplitude and/or peak time of the individual diurnal athletic performance during the day are assessed and/or predicted, wherein predicting the peak time of the individual diurnal athletic performance preferably comprises selecting at least one period of time from at least two distinct periods of time during the day as the peak time.
In another embodiment the present invention provides a method wherein the network computational model and/or the prediction computational model form a personalized model for said subject.
In a further embodiment the present invention provides a method wherein in addition the expression levels of at least one gene selected from the group comprising AKT1, MYOD1, ACE, PPARGC1A, Elovl5 and S12a4g is determined or predicted base on a model of the underlying genetic network and used for said assessment and/or prediction.
In a further embodiment the present invention provides a method wherein samples of at least two consecutive days of said subject are provided and the amount of gene expression is determined and used for said assessment and/or prediction, preferably at least four samples per day.
In a further embodiment the present invention provides a method of predicting the individual diurnal athletic performance time(s) of a subject according to any of claims 1 to 15, wherein each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h.
In a further embodiment the present invention provides a kit for sampling saliva for use in a method, comprising

- sampling tubes for receiving the samples of saliva, wherein each of the sampling tubes contains RNA protect reagent and is configured to enclose one of the samples of saliva to be taken together with the reagent,

wherein preferably each of the sampling tubes is labelled with the time point at which the respective sample is to be taken and/or includes an indication about the amount of saliva for one sample.
In a further embodiment the present invention provides a kit, which further comprises at least one of:

- a box,
- a cool pack,
- at least one form including instructions and/or information about the kit and the method for the subject.

In a further embodiment the present invention provides a kit, wherein the RNA protect reagent is selected from the group comprising EDTA disodium, dihydrate; sodium citrate trisodium salt, dihydrate; ammonium sulfate, powdered; sterile water.
In a further embodiment the present invention provides a kit, wherein said sampling tubes are configured to receive a sample of saliva of 1 mL in addition to 1 mL of the RNA protect reagent, wherein the sampling tubes preferably are at least 2 mL tubes, preferably at least 3 mL tubes, more preferably at least 4 mL tubes, still preferably at least 5 mL tubes.
In a further embodiment the present invention provides a kit, comprising at least six sampling tubes, preferably at least eight sampling tubes.
In a further embodiment the present invention provides a kit for collecting samples of saliva for providing the collected samples of saliva.

## Examples

In view of the aforementioned explanations, an exemplary embodiment for a general workflow to establish a prediction for the best time for a "behavior B" is outlined below. After that, more specific aspects of the work flow according to an exemplary embodiment for are explained for the "behavior B" being the peak time for sport performance.

1. A priori gene selection:

A set of relevant genes is selected: Core-clock genes, saliva specific oscillating genes (which may show stronger oscillations than the core-clock genes in saliva), and a set of genes that should relate to the behavior B (for sports metabolic genes; for cancer treatment-related genes or drug target genes, etc.). To identify the latter, existing databases are scanned for (1) connections to the core-clock in the genetic network, (2) oscillatory behavior (at least for some tissue, potentially from mice or human), (3) expression level in saliva of healthy human samples or saliva from non-healthy people.

2. Establishment of a data set:

Subjects are asked to perform behavior B several times per day, and their performance is recorded. From the same subjects, saliva is sampled for two days 4 times a day.

3. Experimental analysis:

Gene expression is measured from the saliva samples.

4. Computational analysis (see also description above):

i. The gene expression data is screen for oscillatory behavior, non-oscillating genes are excluded from the analysis.
ii. The expression data of oscillating genes is used to predict the optimal time for behavior B, as recorded for the subjects. The resulting machine learning algorithm can then be used to predict the optimal time for further subjects for which only saliva data is available.
iii. A core-clock model is supplemented with the genetic network that includes the oscillatory genes identified in step 4i. The model is fitted to the gene expression data.
iv. The model is used to generate data for the prediction of the optimal timing of behavior B. This data has three advantages: 1. The temporal resolution of the data is arbitrarily detailed. 2. In consequence, peak time, phase, amplitude and period can be extracted with more precision. 3. The data includes all genes implemented in the model, which are far more than the genes measured in step 2. With this data as input, step 4ii is repeated, and the performance is compared to the prediction based directly on the data, while accounting for the enhanced potential for overfitting due to a larger amount of processing (more parameters and potentially more data points for the prediction).

Although also a simple fit of the data as in step i gives us curves with high temporal resolution, this is not the same as the model fit: A prediction based on the simple fit cannot outperform the prediction from step 4ii, because no information was added. By contrast, the model fit contains information on the biological interaction between the genes (the gene dynamics), which, when added to the prediction, can improve the prediction.

v. Even more important, the model can illuminate the mechanism behind the prediction of step 4ii and iv. This is an important step to enhance trustworthiness of the prediction - the more we understand, the more we can evaluate whether the prediction makes sense (see sports example below: The correlation between the peak times of PER2 and sport performance is likely to explain why PER2 can be used to predict sports performance. Although we have a small sample size of only 10 participants, this gives us confidence that the prediction is not happening by chance, but is really catching some salient feature in the data).
vi. With the idea about the working of the prediction mechanism from step 4v, genes with an even higher expected predictive power can be identified. Those genes can be added to the set from step 1, if the whole workflow is repeated to optimize the prediction even further.

5. A posteriori gene selection:

Based on the computational analysis, the a priori set of genes is stripped to the genes essential for prediction, in order to minimize the cost of the analysis.

6. Commercial application:

People provide saliva samples, and the machine learning algorithm resulting from step 4 is used to predict optimal timing of behavior B based on the restricted set of genes from step 5.

According to the present invention, an individual-based (machine learning) prediction of maximal sports performance is provided, wherein individual differences in the amplitude of circadian variation in sports performance are considered. It is shown that a low/high amplitude of ARNTL (BMAL1) gene expression could be used to predict high/low variation in sports performance based on the correlations shown in the results.

The gene expression predicted from the saliva samples can be fitted by a harmonic regression. The fits are done for two core-clock genes, ARNTL (BMAL1) and PER2, and one gene related to sports performance, AKT1. All genes show circadian variation, and AKT1 and ARNTL (BMAL1) show similar dynamics, with the same phase, period, and mean-normalized amplitude, but different overall (mean) expression levels.

Time-course measurements of unstimulated saliva show fluctuations in gene expression across 45 hours are exemplarily shown in Fig. 3. (A) Sampling schemes for saliva collection at 8 time points in two consecutive days (Day 1 - 9h, 13h, 17h, 21h; Day 2 - as day 1). (B) Time-course RT-qPCR measurements of human saliva normalized to the mean of all time points ($\Delta\Delta$CT) of *ARNTL (BMAL1)* (black) and *PER2* (grey dashed) of 15 participants (7 female and 8 male) with a fitted linear sine-cosine function. Furthermore, table C shows the harmonic regression analysis and table D provides additional information on the participants and tests performed.

Fig. 4 illustrates that gene expression of *ARNTL (BMAL1)* and *AKT1* covary. (A) Mean-normalized gene expression profile for the three participants for whom the gene *AKT1* was measured besides *ARNTL (BMAL1)* and *PER2.* The two days were treated as repetitions. The diurnal variation of *AKT1* follows *ARNTL (BMAL1).* (B) The data points from the mean-normalized time-series of *ARNTL (BMAL1)* and *AKT1* correlate, linear regression with $p = 0.018$. (C) Harmonic regression plots for the participants with at least 5 time points. Depicted values are based on individual best fitting period (20h - 28h). Additionally, the harmonic regression results of AKT1 for the best fitting period are shown in table A.

The analysis used expression levels (2 to the power of $\Delta$CT) of *ARNTL (BMAL1)* and *PER2.* In an RT-qPCR assay, a positive reaction is detected by accumulation of a fluorescent signal. But there is also a lot of background fluorescence which needs to be bypassed in order to glean meaningful information from the signal. The cycle threshold (Ct) (alternatively called the quantification cycle (Cq)) is defined as the number of cycles required for the fluorescent signal to cross the threshold (i.e. exceeds background level) which doubles each cycle (1 cycle = 2x original sequence abundance, 2 cycles = 4x original sequence abundance, etc.). Therefore, Ct levels are inversely proportional to the log2-normalised amount of target nucleic acid in the sample (i.e. the lower the Ct level, the greater the amount of target nucleic acid in the sample).

The expression level of a gene is dependent on the amount of input RNA or cDNA. In order to get normalised expression values for the gene of interest (the target gene), it is important to choose a suitable gene for use as a reference. A reference gene is a gene whose expression level should not differ between samples, such as a housekeeping or maintenance gene. Comparison of the Ct value of a target gene with that of the reference gene ($\Delta$CT) allows the gene expression level of the target gene to be normalised to the amount of input RNA or cDNA (Overbergh et al, 2003).

The peak time of the gene expression was identified as the time of the day of the maximum of the time series with eight data, i.e. the maximum gene expression over the two recorded days, with the reasoning that errors in the experimental measurement will rather lead to reports of too little than too high abundances.

Participants were separated into two groups that have distinguishable characteristics both on the genetic as well as on the sports level: Inspired by unsupervised clustering algorithms, the saliva data was separated into two groups with high mean *ARNTL (BMAL1)* expression ($\geq$ 0.04) and low mean *ARNTL (BMAL1)* expression (< 0.04) or early (9h and 13h) and late (17h or 21h) ARNTL (BMAL1) peak time. For sports and Myoton data, the mean over the repetitions at each timepoint was considered; for the Myoton data the mean was taken over right and left muscles as well as different muscles within two muscle groups, hand muscles (*M. adductor pollicis*) and leg muscles (*M. rectus femoris*, *M. biceps femoris*, *M. gastrocnemius*). The sports data and the Myoton data was normalized by the mean value over all data points. Measures of standard deviations were compared between the groups with low and high ARNTL (BMAL1), respectively, and statistically significant lower values in one group compared to the other were tested for by a one-tailed Wilcoxon-Mann-Whitney-Test, as implemented in matlab as ranksum().

The three different uncorrected sample standard deviations were calculated for the sports or Myoton data as: (i) The standard deviation of all data points, including all timepoints and all repetitions. (ii) The standard deviation between different timepoints, where the value for each timepoint results from a mean over the repetitions at this timepoint. (iii) The standard deviation was calculated over the repetitions for each timepoint individually, and then the mean was taken over all timepoints. The latter two measures are meant to separate circadian variations in the data from experimental or physiological noise; the standard deviation between timepoints is likely to be related to daily variations, while the standard deviation of the repetitions rather quantifies measurement noise.

With the aim to predict maximal sports performance from genetic data, we used the python package sklearn for classification. The timing of the maximum for the mean sports performance was labelled as early (9h or 12h) or late (15h or 18h). Advantageous for classification, the HST resulted in balanced classes with five participants each, while the other tests resulted in unbalanced classes with at least seven participants in the late class. In order to train the machine learning algorithm, participants were separated into a training set (here 9 participants) and a test set (here just one participant). The algorithm is fed with the full data of the training set, and is then tested on the participant of the test set, by feeding it with the genetic data, and comparing the predicted sports timing to the actual sports timing of this participant: if the predicted and actual timing are equal, this is counted as correct prediction.

For predicting early versus late HST performance with machine learning (this is also called a classification), the predictive power of different features of the saliva data was tested: the expression levels of *ARNTL (BMAL1)* and *PER2* (averaged between the two days and normalized by the mean expression), the mean expression levels, the peak times (presented in a one-hot encoding, that means that a peaktime at the first sampling time was presented as 1000, at the second as 0100, at the third as 0010, and at the last as 0001) and the relative expression levels (*PER2* divided by *ARNTL (BMAL1)*). A linear support-vector-machine (SVM, see general section on machine learning) was fitted to predict early or late maximal sports performance based on these features (sklearn.svm.LinearSVC(), the regularization constant *C* (see general section on machine learning) is set to 1.0 (default of the python implementation)). Using leave-one-subject-out cross-validation (see general section on machine learning), classification performance was evaluated by computing the accuracy, i.e. the number of correct predictions divided by the total number of predictions. For training, the linear SVM is fed with multi-dimensional input data (here e.g. the 8-dimensional mean-normalized gene expression data) and a binary output (early or late sport performance peak). During cross-validation, the training set consists of nine of the ten relevant participants, and the chosen input with *p* dimensions is denoted as $x_i \in R^p$, , $i \in [1,2,...,9]$. The output $y_i$ is encoded as -1 for early sports peak and +1 for a late sports peak, $y \in \{1, -1\}^9$. The predicted output for the participant not used in the training set, denoted $x_{10}$, is then calculated as $w^T\phi(x_{10}) + b$ with the *w* and *b* resulting from the minimization, and compared with the correct output $y_{10}$. Leave-one-subject-out cross-validation implies that this step is repeated 10 times, each time with another participant removed to form the training set. To calculate the accuracy, the number of correct predictions of the left-out subject of the resulting 10 training sets is divided by 10, the number of predictions that were made.

To evaluate the potential power of the circadian molecular profile obtained from saliva to predict sports performance, a pilot analysis was carried out of the 10 participants with both molecular and sports data (5 males, 5 females). Of major interest for athletes is the time of the best sports performance (peak performance time) as well as the amplitude of the daily variation in sports performance.

The analysis suggests that peak performance time is correlated with PER2, as a linear regression can be fitted to the PER2 peak time when plotted against the peak hand-strength test (HST) performance (Fig. 5A, p = 0.014). A linear regression fits a linear function to the data, such that the sum of least-squares (the squared distance between function and data point) is minimized. PER2 can also predict early (9h or 12h) or late (15h or 18h) peak HST performance. A more precise prediction of the actual peak time was not attempted due to the small sample size. Training a classifier ten times on nine out of ten participants in the context of a leave-one-subject-out cross validation, early or late HST performance could be predicted with an accuracy of up to 100% on the left-out participants. As input to the classifier, exclusively the normalized expression levels of PER2 resulted in a good accuracy of 90% when using individual features). The accuracy could not be improved by using the normalised expression levels of ARNTL (BMAL1), peak times of both genes or relative expression levels as additional feature. Adding the mean ARNTL (BMAL1) levels as additional feature improved the accuracy to 100%. This result is changed when for participant 5 other available saliva data is used, then the prediction accuracy is already at 100% when feeding the algorithm only with the normalized expression levels of PER2. Using as input to the machine learning the peak times of PER2 results in an accuracy of 0.8, however in this case the predictions on the training set showed errors, with one false prediction per training set of nine participants. This shows that, indeed, PER2 peak time is important for the prediction, but that the algorithm uses additional data from the mean-normalised PER2 expression that improves the prediction. Using as individual input normalised expression levels of ARNTL (BMAL1), peak times of ARNTL (BMAL1), relative expression levels or mean ARNTL (BMAL1) levels did not lead to good predictions.

For the participants, the best performance of the day is around 10% higher than the worst performance (figure 6B). There were found particularly strong diurnal changes in the HST for participants with an early ARNTL (BMAL1) peak, while small changes occurred for a late ARNTL (BMAL1) peak (ARNTL (BMAL1) level is color-coded in Fig. 5B, black/grey corresponds to early/late *ARNTL (BMAL1)* peaks as shown in Fig. 5C). To quantify this observation we compared three measures of variation: Based on the mean-normalized HST performance, we calculated the standard deviation (i) for all data, (ii) for the mean values per time point, (iii) for the repetitions at each time point (compare Methods). The standard deviation between timepoints (ii), which relates to the performance changes over the day, is significantly higher for participants with early ARNTL (BMAL1) compared to participants with late BMAL1 (Fig. 5D, p < 0.01). The difference is not significant for standard deviation (iii), which rather quantifies measurement noise (Fig. 5D, p = 0.057). A large performance change over the day is thus predicted by an early BMAL1 peak time. In addition to the here shown correlation with ARNTL (BMAL1) peak time, the amplitude of the performance changes also correlated with the mean level of ARNTL (BMAL1) expression: Repeating the analysis based on two groups with low (< 0.04) and high (> 0.04) ARNTL (BMAL1) mean expression levels, there were found significant higher standard deviations for the group with low ARNTL (BMAL1) levels for the mean of HST and CMJ (figure 6E left panel, $p < 0.05$), as well as for the muscle tone of the hand muscles (myotonometric data for 3 males, 4 females, Fig. 5E, right panel, p(i) = 0.029, p(ii) = 0.11, p(iii) = 0.029). The results hinted at larger diurnal changes in performance (HST and CMJ) and muscle tone (hand) for participants with low mean ARNTL (BMAL1) levels. This is partly explained by a relation between mean ARNTL (BMAL1) levels and ARNTL (BMAL1) peak time; the group with low mean ARNTL (BMAL1) levels shows significantly earlier ARNTL (BMAL1) peak times (Fig. 5F, Mann-Whitney-U test, p = 0.044). No relation was found for the performance of the SRT, for which no repetitions are available, or for the muscle tone of the leg muscles, potentially due to the small sample size of seven participants (Fig. 6).

While the groups with low and high mean *ARNTL (BMAL1)* levels from above consisted of 3 females, 2 males and 2 females, 3 males, respectively, our data showed a trend for higher *ARNTL (BMAL1)* levels in males compared to females (Fig. 5G, Welch's t-test, $p < 0.0001$). The gender difference is also visible in the PER2 / ARNTL (BMAL1) ratio (figure 6H, Welch's t-test, $p < 0.0001$); all participants with high ratios in Supplementary figure 1D are females. The grouping based on the peak time of ARNTL (BMAL1) does not correlate with the MEQ chronotype (Fig. 5I), neither does early and late sport performance. An overview of the 1 min warm-up sequence is provided in table F and an overview of detailed statistics is shown in table G.

The analysis suggests that the circadian oscillation of sports performance mainly depends in its amplitude on *ARNTL (BMAL1)* expression and in its phase (peak performance) on the expression of *PER2.*

Correlations between molecular rhythms of core-clock genes and athletic performance are shown in Fig. 5. (A) The peak time of *PER2* correlates with the time of peak performance of the HST (linear regression with p = 0.014). (B) Performance change over the day (max. compared to min.), colour code as in (C). (C) Black and grey groups have an early and late *ARNTL* (BMAL1) peak time, respectively. (D) Standard deviation calculated on the normalized HST performance for data from different (i) repetitions and time points ($p = 0.0095$), (ii) timepoints ($p = 0.0095$), (iii) repetitions ($p = 0.057$). (E) Separating the groups by the mean expression level of *ARNTL (BMAL1)* instead of the peak time results in significant differences in the standard deviation of the sports performance of HST and CMJ (left, all $p = 0.0476$) and of the hand muscle frequency (right, $p = 0.0286$, $p = 0.11$, $p = 0.0286$). (F) Histogram of the time of the day with the highest *ARNTL (BMAL1)* expression based on the eight saliva samples. Significantly earlier peaks are found for the group with low *ARNTL (BMAL1)* expression (ranksum, $p = 0.044$). (G) Logarithm of ARNTL (BMAL1) expression levels for all sampling times ordered by male and female participants. Males show a significant higher *ARNTL (BMAL1)* expression compared to females (Welch's t-test, $p < 0.0001$). (H) Logarithm of the ratio of *PER2* and *ARNTL (BMAL1)* expression levels for all sampling times ordered by male and female participants. Females show a significant higher *ARNTL (BMAL1)* expression compared to males (Welch's t-test, $p < 0.0001$). (I) Early or late *ARNTL (BMAL1)* peaks occur in any of the three investigated MEQ chronotype.

Fig. 6 shows diagrams of standard deviations of normalized sports and muscle tone data (L: group with low *ARNTL (BMAL1)*, H: group with high *ARNTL (BMAL1)*). Mean standard deviation calculated on the normalized sports performance and the normalized muscle tone data for different (i) repetitions and timepoints, (ii) timepoints, (iii) repetitions (for details see Methods). (A) HST, (B) CMJ, (C) SRT (no repetitions were measured, thus the standard deviation (i) over all data is the same as (ii) over timepoints), (D) muscle tone of the leg muscles *(M. rectus femoris, M. biceps femoris, M. gastrocnemius).* The performed 15 min warm-up sequence is depicted in table F an overview of detailed statistics is shown in table G.

With reference to Fig. 7, modeling the genetic network associated with sports performance is explained. The model simulates gene expression of the core-clock genes and clock-regulated genes via two interconnected feedback loops (Per/Cry loop and Rev-Erb/Ror/Bmal loop). The model parameters were fitted to the measured data of gene expression. The model predicts the rhythmicity of athletic performance based on the oscillatory behaviour of *Ace* and *Ppargc 1a* genes.

In the genetic network for sports performance extension of the core-clock illustrated in Fig. 7, the plots show the gene expression of 2 core-clock genes and 4 clock-regulated genes crucial for athletic performance and metabolism. Dots indicate the measured gene expression of *Arntl* (*Bmal1), Per2, Ace,* and *Ppargc 1a.* Solid lines represent the *in-silico* gene expression generated with the mathematical model, which was fitted to the experimental data of the previously mentioned genes. The model additionally predicts the expression of *Elovl5 and Sl2a4g* genes, important for metabolism.

An example of predicting the peak time for sport performance is described with reference to Figs. 8a to 8c, in which Fig. 8a illustrates the core-clock genes. Genes important for athletic performance and metabolism are illustrated in Figs. 8b and 8c.

The result is illustrated in Fig. 9, where the mathematical model computes the athletic performance based on the expression of *Ppargc 1a* and *Ace* genes. Accordingly, the predicted time window for maximum athletic performance is 11:00 - 15:00 hours, the peak of athletic performance occurs 5 hours since awakening, and the recommended time-window for meals is 08:00 - 18:00 hours.

Fig. 10 illustrates ARNTL (BMAL1) and PER2 expression display variation during the day in human blood, hair and saliva samples. (A) Three time-point comparison of ARNTL (BMAL1) and PER2 expression for the averaged data of all Participants in figure 1. Expression data is compared to the first time-point (Early). For hair and saliva data Early, Middle and Late time-points represent 9h, 17h and 21h, respectively. For PBMCs data Early, Middle and Late time-points represent 10h, 16h and 19h, respectively. Depicted are mean + SEM. (B) Time-course RT-qPCR measurements normalised to the mean of all time points (ΔΔCT) of ARNTL (BMAL1) and PER2 of Participant 1, 2, and 13 with a fitted linear sine-cosine function (period = 24 h). For Participant 1, we collected one additional sample at 21h on the 2nd day. Harmonic regression best p-values for tested periods (20-28h): Participant 1; BMAL1 (0.517, period = 21.4h), PER2 (0.353, period = 24.0h). Participant 2; ARNTL (BMAL1) (0.038, period = 20.0h), PER2 (0.276, period = 28.0h). Participant 13; ARNTL (BMAL1) (0.014, period = 20h), PER2 (0.086, period = 21.4h). (C) Time-course RT-qPCR measurements of human PBMCs normalised to the mean of all time points (ΔΔCT) of ARNTL (BMAL1), CLOCK, NPAS2, PER2, CRY2, NR1D1, and RORB of Participant 2 and 5 with a fitted linear sine-cosine function (period = 24 h). Harmonic regression best p-values: Participant 2; ARNTL (BMAL1) (3.05E-01, period = 20h), CLOCK (6.31E-02, period = 28h), NPAS2 (1.67E-01, period = 20h), PER2 (4.78E-04, period = 20.8h), CRY2 (7.17E-01, period = 20h), NR1D1 (1.48E-01, period = 28h) and RORB (7.58E-01, period = 20h). Participant 5; ARNTL (BMAL1) (5.56E-01, period = 20h), CLOCK (6.81E-01, period = 28h), NPAS2 (9.75E-02, period = 28h, PER2 (1.23E-01, period = 28h), CRY2 (5.40E-01, period = 28h), NR1D1 (6.43E-01, period = 28h) and RORB (7.73E-01, period = 28h). (D) Average PER2 expression compared to ARNTL (BMAL1) using saliva time-course data for each participant (mean + SEM).

Figure 11 illustrates HST base line measurements. Depicted are mean values for three participants (9h-18h in one-hour intervals, N = 3, mean ± SEM). The HST measurements were randomly distributed across three measurement days with one day break in between. The red full circles represent the time point chosen for the subsequent training sessions. For detailed HST base line measurements see table E.

Figure 12 illustrates Myotonometric analysis shows daily variation in muscle tone (frequency, F) for female and male participants. Only participants who completed all training sessions were included in the MyotonPRO measurements (N=12). Mean of normalized scores for the myotonometric parameter frequency [Hz] for each training session (T1-9h, T2-12h, T3-15h, T4-18h) and each muscle: M. Deltoideus, M. Triceps Brachii, M.adductor pollicis, M. rectus femoris, M. biceps femoris, M. gastrocnemius. The measurements were carried out from top to bottom on the right (Right bar) and the corresponding left (Left bar) side of the body. Corresponding statistics for intrapersonal variation between each time points can be found in Supplementary Table B. * $p < 0.05$, compared to time point 9h. A detailed statistical analysis is depicted in table B and H.

Tables

Table A: Harmonic regression results of AKT1 for the best fitting period in Figure 4.

| Participant | qvals | pvals | acrophase [h] | amplitude | Period [h] |
|---|---|---|---|---|---|
| Participant 3 | 0.249 | 0.178 | 18 | 1.506 | 28 |
| Participant 5 | 0.061 | 0.017 | 12 | 1.042 | 28 |
| Participant 6 | 0.011 | 0.001 | 11 | 1.068 | 26.6 |
| Participant 12 | 0.696 | 0.229 | 14 | 1.067 | 20 |
| Participant 21 | 0.249 | 0.167 | 14 | 1.386 | 28 |

s Table B: Statistical analysis for Figure 12, pairwise comparisons (Friedmann test).

| **M. Deltoideus_Left (Females)** | **T1** | **T2** | **T3** | **T4** |
|---|---|---|---|---|
| **T1** | NA | | | |
| T2 | 0.038 | NA | | |
| T3 | 0.038 | 1 | NA | |
| T4 | 0.047 | 0.902 | 0.902 | NA |
| **M. Deltoideus_Right (Females)** | **T1** | **T2** | **T3** | **T4** |
| **T1** | NA | | | |
| **T2** | 0.629 | NA | | |

(continued)

| **M. Deltoideus_Right (Females)** | **T1** | **T2** | **T3** | **T4** |
|---|---|---|---|---|
| T3 | 0.629 | 0.797 | NA | |
| T4 | 0.797 | 0.670 | 0.629 | NA |
| **M. Triceps Brachii_Left (Females)** | **T1** | **TZ** | **T3** | **T4** |
| **T1** | NA | | | |
| T2 | 0.807 | NA | | |
| T3 | 0.807 | 0.807 | NA | |
| T4 | 0.807 | 0.807 | 0.807 | NA |
| **M. Triceps Brachii_Right(Females)** | **T1** | **T2** | **T3** | **T4** |
| **T1** | NA | | | |
| T2 | 0.741 | NA | | |
| T3 | 0.741 | 0.741 | NA | |
| T4 | 1 | 0.741 | 0.741 | NA |
| **M. Rectus Femoris_Left(Females)** | **T1** | **T2** | **T3** | **T4** |
| **T1** | NA | | | |
| T2 | 0.922 | NA | | |
| T3 | 0.922 | 0.964 | NA | |
| T4 | 0.922 | 1 | 0.964 | NA |
| **M. Rectus Femoris_Right(Females)** | **T1** | **T2** | **T3** | **T4** |
| **T1** | NA | | | |
| T2 | 0.210 | NA | | |
| T3 | 0.434 | 0.539 | NA | |
| T4 | 0.049 | 0.210 | 0.142 | NA |
| **M. Biceps Femoris_Left(Females)** | **T1** | **T2** | **T3** | **T4** |
| **T1** | NA | | | |
| **T2** | 0.441 | NA | | |
| **T3** | 0.377 | 0.712 | NA | |
| **T4** | 1 | 0.441 | 0.377 | NA |
| **M. Biceps Femoris_Right(Females)** | **T1** | **T2** | **T3** | **T4** |
| **T1** | NA | | | |
| **T2** | 0.516 | NA | | |
| **T3** | 0.516 | 0.488 | NA | |
| **T4** | 0.790 | 0.516 | 0.516 | NA |
| **M. Adductor Pollicis_Left(Females)** | **T1** | **T2** | **T3** | **T4** |
| **T1** | NA | | | |
| **T2** | 0.797 | NA | | |
| **T3** | 0.670 | 0.629 | NA | |
| **T4** | 0.629 | 0.629 | 0.797 | NA |

(continued)

| M. Adductor Pollicis_Right(Females) | T1 | T2 | T3 | T4 |
|---|---|---|---|---|
| T1 | NA | | | |
| T2 | 0.473 | NA | | |
| T3 | 0.185 | 0.333 | NA | |
| T4 | 0.185 | 0.392 | 0.773 | NA |
| **M. Gastrocnemius_Left(Females)** | **T1** | **T2** | **T3** | **T4** |
| T1 | NA | | | |
| T2 | 0.263 | NA | | |
| T3 | 0.197 | 0.038 | NA | |
| T4 | 0.197 | 0.038 | 1 | NA |
| **M. Gastrocnemius_Right(Females)** | **T1** | **T2** | **T3** | **T4** |
| T1 | NA | | | |
| T2 | 0.382 | NA | | |
| T3 | 0.589 | 0.382 | NA | |
| T4 | 0.382 | 0.589 | 0.589 | NA |
| **M. Deltoideus_Left (Males)** | **T1** | **T2** | **T3** | **T4** |
| T1 | NA | | | |
| T2 | 0.963 | NA | | |
| T3 | 0.963 | 0.963 | NA | |
| T4 | 0.963 | 0.963 | 1 | NA |
| **M. Deltoideus_Right (Males)** | **T1** | **T2** | **T3** | **T4** |
| T1 | NA | | | |
| T2 | 0.481 | NA | | |
| T3 | 0.600 | 0.719 | NA | |
| T4 | 0.601 | 0.650 | 0.792 | NA |
| **M. Triceps Brachii_Left (Males)** | **T1** | **T2** | **T3** | **T4** |
| T1 | NA | | | |
| T2 | 0.458 | NA | | |
| T3 | 0.466 | 0.689 | NA | |
| T4 | 0.534 | 0.534 | 0.689 | NA |
| **M. Triceps Brachii_Right (Males)** | **T1** | **T2** | **T3** | **T4** |
| T1 | NA | | | |
| T2 | 0.037 | NA | | |
| T3 | 0.203 | 0.203 | NA | |
| T4 | 0.203 | 0.203 | 1 | NA |
| **M. Rectus Femoris_Left(Males)** | **T1** | **T2** | **T3** | **T4** |
| T1 | NA | | | |
| T2 | 0.652 | NA | | |
| T3 | 0.039 | 0.047 | NA | |

(continued)

| M. Rectus Femoris_Left(Males) | T1 | T2 | T3 | T4 |
|---|---|---|---|---|
| T4 | 0.219 | 0.129 | 0.360 | NA |
| **M. Rectus Femoris_Right(Males)** | **T1** | **T2** | **T3** | **T4** |
| T1 | NA | | | |
| T2 | 0.203 | NA | | |
| T3 | 0.203 | 0.067 | NA | |
| T4 | 1 | 0.203 | 0.203 | NA |
| **M. Biceps Femoris_Left(Males)** | **T1** | **T2** | **T3** | **T4** |
| T1 | NA | | | |
| T2 | 0.637 | NA | | |
| T3 | 1 | 0.637 | NA | |
| T4 | 1 | 0.637 | 1 | NA |
| **M. Biceps Femoris_Right(Males)** | **T1** | **T2** | **T3** | **T4** |
| T1 | NA | | | |
| T2 | 0.798 | NA | | |
| T3 | 0.798 | 0.798 | NA | |
| T4 | 0.798 | 0.798 | 0.798 | NA |
| **M. Adductor Pollicis_Left(Males)** | **T1** | **T2** | **T3** | **T4** |
| T1 | NA | | | |
| T2 | 1 | NA | | |
| T3 | 0.801 | 0.801 | NA | |
| T4 | 0.076 | 0.076 | 0.118 | NA |
| **M. Adductor Pollicis_Right(Males)** | **T1** | **T2** | **T3** | **T4** |
| T1 | NA | | | |
| T2 | 0.605 | NA | | |
| T3 | 0.605 | 0.605 | NA | |
| T4 | 0.605 | 0.605 | 0.605 | NA |
| **M. Gastrocnemius_Left(Males)** | **T1** | **T2** | **T3** | **T4** |
| T1 | NA | | | |
| T2 | 0.228 | NA | | |
| T3 | 0.584 | 0.228 | NA | |
| T4 | 0.228 | 0.584 | 0.421 | NA |
| **M. Gastrocnemius_Right(Males)** | **T1** | **T2** | **T3** | **T4** |
| T1 | NA | | | |
| T2 | 1 | NA | | |
| T3 | 0.740 | 0.740 | NA | |
| T4 | 0.740 | 0.740 | 0.740 | NA |

Table: Harmonic regression analysis (see Figure 3).

| Mesor | qvals | pvals | Acrophase [h] | Acrophase [radians] | Amplitude | Period [h] | Condition |
|---|---|---|---|---|---|---|---|
| -0.50 | 0.49 | 0.18 | 15.83 | 4.14 | 1.22 | 23.6 | BMAL1_Participant 5 |
| 0.42 | 0.60 | 0.42 | 25.32 | 6.62 | 0.92 | 26.2 | PER2_Participant 5 |
| -0.12 | 0.24 | 0.01 | 6.67 | 1.74 | 1.80 | 28 | BMAL1_Participant 15 |
| -0.03 | 0.70 | 0.54 | 6.60 | 1.72 | 0.58 | 28 | PER2_Participant 15 |
| -0.03 | 0.31 | 0.05 | 0.59 | 0.15 | 1.49 | 20.9 | BMAL1_Participant 21 |
| 0.29 | 0.35 | 0.06 | 6.30 | 1.70 | 1.48 | 20 | PER2_Participant 21 |
| -2.03 | 0.52 | 0.23 | 15.08 | 3.94 | 4.38 | 25.2 | BMAL1_Participant 8 |
| -0.63 | 0.70 | 0.58 | 12.54 | 3.28 | 1.26 | 28 | PER2_Participant 8 |
| -0.71 | 0.21 | 0.01 | 18.33 | 4.79 | 3.85 | 22.9 | BMAL1_Participant 9 |
| -0.06 | 0.51 | 0.15 | 3.29 | 0.86 | 2.33 | 20 | PER2_Participant 9 |
| 0.02 | 0.15 | 0.01 | 20.33 | 5.32 | 1.78 | 20.8 | BMAL1_Participant 17 |
| 0.38 | 0.70 | 0.40 | 0.51 | 0.13 | 0.72 | 28 | PER2_Participant 17 |
| -0.01 | 0.48 | 0.14 | 21.05 | 5.51 | 0.28 | 22.1 | BMAL1_Participant 13 |
| 0.05 | 0.56 | 0.15 | 24.33 | 6.36 | 0.11 | 28 | PER2_Participant 13 |
| -0.16 | 0.70 | 0.54 | 17.04 | 4.46 | 0.42 | 28 | PER2_Participant 11 |
| -0.16 | 0.73 | 0.63 | 15.67 | 4.10 | 0.34 | 28 | BMAL1_Participant 11 |
| -1.47 | 0.45 | 0.06 | 14.34 | 3.75 | 3.50 | 26.6 | BMAL1_Participant 1 |
| 0.29 | 0.31 | 0.02 | 21.82 | 5.71 | 1.71 | 26.9 | PER2_Participant 1 |
| -0.85 | 0.21 | 0.02 | 16.80 | 4.39 | 2.65 | 23.5 | BMAL1_Participant 3 |
| -0.61 | 0.21 | 0.01 | 13.75 | 3.59 | 1.63 | 22.6 | PER2_Participant 3 |
| -0.12 | 0.10 | 0.03 | 13.36 | 3.49 | 1.43 | 20 | BMAL1_Participant 19 |
| 0.38 | 0.56 | 0.07 | 27.44 | 7.18 | 0.76 | 28 | PER2_Participant 19 |
| 0.17 | 0.88 | 0.58 | 4.98 | 1.30 | 1.07 | 20 | BMAL1_Participant 2 |
| 0.02 | 0.69 | 0.29 | 11.28 | 2.95 | 0.50 | 20.3 | PER2_Participant 2 |
| 0.00 | 0.60 | 0.31 | 21.15 | 5.53 | 0.56 | 23.4 | BMAL1_Participant 4 |
| -0.04 | 0.63 | 0.23 | 20.07 | 5.25 | 0.28 | 20.7 | PER2_Participant 4 |
| -0.01 | 0.67 | 0.28 | 1.88 | 0.49 | 0.71 | 20 | BMAL1_Participant 12 |
| -0.13 | 0.84 | 0.53 | 18.94 | 4.95 | 0.76 | 20 | PER2_Participant 12 |
| -0.60 | 0.46 | 0.09 | 12.22 | 3.19 | 1.46 | 26.7 | BMAL1_Participant 6 |
| -0.05 | 0.40 | 0.09 | 12.83 | 3.35 | 0.42 | 22 | PER2_Participant 6 |

Table D: List of participants and tests (MEQ, Sports tests, Molecular tests, Myotonometry) performed (see Figure 3). Y = Yes, participant has carried out the test.

| | | | sports tests | | | molecular tests | | | myotonometry |
|---|---|---|---|---|---|---|---|---|---|
| Participant # | gender | MEQ | # training sessions | # round of tests | HST_long | saliva | hair | blood | MyotonPRO |
| 1 | male | intermediate | | - | - | Y | Y | - | - |
| 2 | male | intermediate | - | - | - | Y | Y | Y | |
| 3 | female | intermediate | - | - | **Y** | Y | - | - | |
| 4 | male | moderate morning | - | - | **Y** | Y | Y | - | - |
| 5 | female | moderate morning | 4 | 1 | Y | Y | - | Y | Y |
| 6 | female | moderate evening | 4 | 1 | - | Y | | - | Y |
| 7 | female | intermediate | 4 | 1 | | - | | | Y |
| 8 | female | intermediate | 4 | 1 | | Y | | | Y |
| 9 | female | moderate evening | 4 | 1 | | Y | | | Y |
| 10 | male | intermediate | 4 | 1 | | - | | | Y |
| 11 | male | intermediate | 4 | 1 | | Y | | | Y |
| 12 | female | intermediate | - | - | | Y | - | | - |
| 13 | male | intermediate | 4 | 1 | | Y | Y | | Y |
| 14 | male | intermediate | 4 | 1 | | - | - | | Y |
| 15 | male | intermediate | 4 | 1 | | Y | | | Y |
| 16 | male | moderate evening | 4 | 1 | | - | | | Y |
| 5 | female | moderate morning | 3 | 2 | | | | | |
| 8 | female | intermediate | 3 | 2 | | - | | | |
| 17 | female | moderate evening | 4 | 2 | | Y | | | |
| 18 | male | intermediate | 3 | 2 | | - | | | |
| 10 | male | intermediate | 4 | 2 | | - | | | |
| 19 | male | moderate morning | 3 | 2 | | Y | | | |
| 20 | male | moderate evening | 4 | 2 | | - | | | |
| 21 | male | moderate morning | 4 | 2 | - | Y | - | - | - |

Table E: HST base line measurements (see Figure 11) (9h-18h in one-hour intervals, N = 3, mean ± SEM)

| Time [h] | 9 | 10 | 11 | 12 | 13 | 14 | 15 | 16 | 17 | 18 |
|---|---|---|---|---|---|---|---|---|---|---|
| mean | 0.86 | 1.03 | 0.98 | 0.97 | 0.97 | 1.05 | 1.08 | 1.08 | 0.97 | 1.03 |
| SEM | 0.05 | 0.03 | 0.02 | 0.02 | 0.02 | 0.05 | 0.02 | 0.03 | 0.03 | 0.08 |

Table F: 15 min warm-up sequence carried out before the exercises: HST, CMJ, SRT (see Figures 5 and 6).

| Exercise | Repetitions | Aim and muscle group used |
|---|---|---|
| **Jogging - Forward and backwards** | 2 × 20 m Forwards<br>2 × 20 m Backwards | whole body warm up |
| **"Butt kicks"** | 2 × 20 m | ischiocrucal muscles |
| **High knees** | 2 × 20 m | lower limbs |
| **Sidesteps** | 1 × 20 m | abductors |
| **Cross-step exercise** | 1 × 20 m | foot coordination |
| **Knee to chest** | 10 × | stretching of the hamstrings (*M. biceps femoris*) |
| **Foot inside pull** | 10 × | stretching of the leg adductors |
| **Lunge** | 10 × | stretching of *M. psoas major*, activating the leg muscles and stability work |
| **Caterpillar** | 5 × | stretching the hamstrings (*M. biceps femoris*), core muscles activation and activation for the muscles of the upper limbs |
| **Jogging - Forward and backwards** | 1 × 20 m Forwards<br>1 × 20 m Backwards | whole body warm up before sprinting |
| **Intensity Sprints** | 30 % max speed<br>60 % max speed<br>90 % max speed<br>20 m sprint max speed | step by step preparation for explosive workout |

Table G - Statistics corresponding to Figures 5 & 6. Friedmann test was used for determining the pairwise intrapersonal variations between different training times.

| Test | Gender | Time point | T1 | T2 | T3 | T4 |
|---|---|---|---|---|---|---|
| **HST** | Male | T1 | NA | | | |
| **HST** | Male | T2 | 0.879 | NA | | |
| **HST** | Male | T3 | 0.879 | 1 | NA | |
| **HST** | Male | T4 | 0.879 | 1 | 1 | NA |
| **HST** | Female | T1 | NA | | | |
| **HST** | Female | T2 | 0.373 | NA | | |
| **HST** | Female | T3 | 0.373 | 0.789 | NA | |
| **HST** | Female | T4 | 0.514 | 0.514 | 0.514 | NA |
| **CMJ** | Male | T1 | NA | | | |
| **CMJ** | Male | T2 | 0.681 | NA | | |
| **CMJ** | Male | T3 | 0.015 | 0.005 | NA | |

(continued)

| Test | Gender | Time point | T1 | T2 | T3 | T4 |
|---|---|---|---|---|---|---|
| **CMJ** | Male | T4 | 0.681 | 1 | 0.005 | NA |
| **CMJ** | Female | T1 | NA | | | |
| **CMJ** | Female | T2 | 0.805 | NA | | |
| **CMJ** | Female | T3 | 0.805 | 1 | NA | |
| **CMJ** | Female | T4 | 0.622 | 0.805 | 0.805 | NA |
| **SRT** | Male | T1 | NA | | | |
| **SRT** | Male | T2 | 0.017 | NA | | |
| **SRT** | Male | T3 | 0.034 | 0.550 | NA | |
| **SRT** | Male | T4 | 0.0003 | 0.098 | 0.034 | NA |
| **SRT** | Female | T1 | NA | | | |
| **SRT** | Female | T2 | 0.481 | NA | | |
| **SRT** | Female | T3 | 0.481 | 0.893 | NA | |
| **SRT** | Female | T4 | 0.481 | 0.827 | 0.827 | NA |

Table H - Statistical analysis with Friedmann test corresponding to Figure 12.

| Muscle_Name | | T1 | T2 | T3 | T4 |
|---|---|---|---|---|---|
| **m_deltoideus_L** | **T1** | NA | | | |
| **m_deltoideus_L** | **T2** | 0.131 | NA | | |
| **m_deltoideus_L** | **T3** | 0.060 | 0.629 | NA | |
| **m_deltoideus_L** | **T4** | 0.060 | 0.707 | 0.786 | NA |
| **m_deltoideus_R** | **T1** | NA | | | |
| **m_deltoideus_R** | **T2** | 0.655 | NA | | |
| **m_deltoideus_R** | **T3** | 1 | 0.655 | NA | |
| **m_deltoldeus_R** | **T4** | 0.655 | 1 | 0.655 | NA |
| **m_triceps_brachii_L** | **T1** | | | | |
| **m-triceps_Orachii-L** | **T2** | 0.718 | NA | | |
| **m_triceps_brachii_L** | **T3** | 0.718 | 0.766 | NA | |
| **m_triceps_brachii_L** | **T4** | 0.718 | 0.792 | 0.792 | NA |
| **m_triceps_brachii_R** | **T1** | NA | | | |
| **m_triceps_brachii_R** | **T2** | 0.585 | NA | | |
| **m_triceps_brachii_R** | **T3** | 0.649 | 0.649 | NA | |
| **m_triceps_brachii_R** | **T4** | 0.585 | 0.792 | 0.720 | NA |
| **m_add_pollicis_L** | **T1** | NA | | | |
| **m_add_pollicis_L** | **T2** | 0.674 | NA | | |
| **m_add_pollicis_L** | **T3** | 0.008 | 0.012 | NA | |
| **m_add_pollicis_L** | **T4** | 0.062 | 0.113 | 0.255 | NA |
| **m_add_pollicis_R** | **T1** | NA | | | |

(continued)

| Muscle_Name | | T1 | T2 | T3 | T4 |
|---|---|---|---|---|---|
| **m_add_polllcis_R** | **T2** | 1 | NA | | |
| **m_add_pollicis_R** | **T3** | 0.051 | 0.051 | NA | |
| **m_add_pollicis_R** | **T4** | 0.028 | 0.028 | 0.699 | NA |
| **m_rectus_femoris_L** | **T1** | NA | | | |
| **m_rectus_femoris_L** | **T2** | 1 | NA | | |
| **m_rectus_femoris_L** | **T3** | 0.241 | 0.241 | NA | |
| **m_rectus_femoris_L** | **T4** | 1 | 1 | 0.241 | NA |
| **m_rectus_femoris_R** | **T1** | NA | | | |
| **m_rectus_femoris_R** | **T2** | 0.127 | NA | | |
| **m_rectus_femoris_R** | **T3** | 0.214 | 0.014 | NA | |
| **m_rectus_femoris_R** | **T4** | 1 | 0.127 | 0.214 | NA |
| **m_biceps_femoris_L** | **T1** | NA | | | |
| **m_biceps_femoris_L** | **T2** | 0.777 | NA | | |
| **m_biceps_femoris_L** | **T3** | 0.250 | 0.190 | NA | |
| **m_biceps_femoris_L** | **T4** | 0.003 | 0.003 | 0.059 | NA |
| **m_biceps_femoris_R** | **T1** | NA | | | |
| **m_biceps_femoris_R** | **T2** | 0.780 | NA | | |
| **m_biceps_femoris_R** | **T3** | 0.017 | 0.025 | NA | |
| **m_biceps_femoris_R** | **T4** | 0.017 | 0.017 | 0.780 | NA |
| **m_gastr_cm_L** | **T1** | NA | | | |
| **m_gastr_cm_L** | **T2** | 0.005 | NA | | |
| **m_gastr_cm_L** | **T3** | 0.474 | 0.0009 | NA | |
| **m_gastr_cm_L** | **T4** | 0.775 | 0.007 | 0.388 | NA |
| **m_gastr_cm_R** | **T1** | NA | | | |
| **m_gastr_cm_R** | **T2** | 0.436 | NA | | |
| **m_gastr_cm_R** | **T3** | 0.518 | 0.518 | NA | |
| **m_gastr_cm_R** | **T4** | 0.518 | 0.518 | 0.792 | NA |

## Claims

1. Method of assessing the circadian rhythm or circadian profile of a subject and/or assessing and predicting the athletic performance of said subject, wherein said method comprises the steps of:

    • Providing at least three samples of saliva, more preferably four samples of saliva, from said subject, wherein said samples have been taken at different time points over the day,
    • Determining gene expression of at least two members of genes for the core-clock network, in particular of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, in each of said samples, and
    • Assessing and predicting by means of a computational step based on said expression levels of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, over the day the circadian rhythm of said subject and/or the individual diurnal athletic performance times.

2. Method according to claim 1, wherein gene expression is determined using a method selected from quantitative PCR (RT-qPCR), NanoString, sequencing and microarray.

3. Method according to any of claims 1 to 2, wherein assessing the circadian rhythm of said subject comprises determining a periodic function for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, that approximates said expression levels for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2,, preferably comprising curve fitting of a non-linear periodic model function to the respective expression levels, wherein the curve fitting is preferably carried out by means of harmonic regression.

4. Method according to any of claims 1 to 3, wherein the computational step comprises

    • processing the determined expression levels and/or the respectively fitted periodic functions to derive characteristic data for each of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, said processing comprising determining the mean expression level of expression of at least two members of the groups comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, and normalizing the expression levels using the mean expression level.

**5.** Method according to claim 4, wherein said characteristic data comprise:

- the amplitude of change of expression of a gene, and/or the amplitude relative to one of the other genes, and/or
- the mean expression level of expression of a gene, and/ or and/or the mean relative to one of the other genes, and/or
- the peak expression level of a gene, and/or the peak relative to one of the other genes., and/or
- the amplitude of change of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the amplitudes of change of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the mean expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/ or
- the relative difference of the mean expression levels of expression of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC over the day, and/or
- the relative difference of the peak expression levels of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC, and/or
- the time of the peak expression level of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,
- the relative difference of the times of the peak expression level of any two of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC,

wherein the amplitude, period and phase expression level of expression of ARNTL (BMAL1) and/or ARNTL2 and/or CLOCK, and/or NPAS2 and/or PER1 and/or PER2 and/or PER3 and/or CRY1 and/or CRY2 and/or NR1D1 and/or NR1D2 and/or RORA and/or RORB and/or RORC are extracted from the determined expression levels and/or the respectively fitted periodic function.

**6.** Method according to any of claims 4 or 5, wherein the computational step further comprises

- fitting a network computational model to the derived characteristic data that comprises a representation of the periodic time course of the expression levels for each of at least two members of the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2, as well as a representation of the periodic time course of the expression level for at least one, preferably a plurality of further gene(s) included in a gene regulatory network that includes said at least two members the group comprising ARNTL (BMAL1), ARNTL2, CLOCK, PER1, PER2, PER3, NPAS2, CRY1, CRY2, NR1D1, NR1D2, RORA, RORB, RORC, in particular ARNTL (BMAL1) and PER2,; and/or
- training a machine learning algorithm on the derived characteristic data of the network computational model, particularly optimize in terms of the representation of the periodic time course of the expression level for the at least one further gene.

**7.** Method according to any of claims 4 to 6, wherein assessing and/or predicting the individual diurnal athletic performance times comprises in the computational step fitting a prediction computational model on data obtained from said fitted periodic functions and/or said network computational model, wherein the prediction computational model is based on machine learning, including at least one classification method and/or at least one clustering method wherein said method(s) are preferably selected from the group comprising:
K-nearest neighbor algorithm, unsupervised clustering, deep neural networks, random forest algorithm, and support vector machines.

**8.** Method according to any of claims 1 to 7, wherein the network computational model and/or the prediction computational model form a personalized model for said subject.

**9.** Method according to any of claims 1 to 8, wherein in addition the expression levels of at least one gene selected from the group comprising AKT1, MYOD1, ACE, PPARGC1A, Elovl5 and S12a4g is determined or predicted base on a model of the underlying genetic network and used for said assessment and/or prediction.

**10.** Method of predicting the individual diurnal athletic performance time(s) of a subject according to any of claims 1 to 9, wherein each of the time points at which said samples are obtained are at least 2-4 hours apart, and/or wherein the time points span a time period of at least 12 hours of the day, wherein preferably the time points are 4 hours apart, e.g. at 9h, 13h, 17h and 21h.

**11.** Kit for sampling saliva for use in a method according to any of claims 1 to 10, comprising

- sampling tubes for receiving the samples of saliva, wherein each of the sampling tubes contains RNA protect reagent and is configured to enclose one of the samples of saliva to be taken together with the reagent,

wherein preferably each of the sampling tubes is labelled with the time point at which the respective sample is to be taken and/or includes an indication about the amount of saliva for one sample.

**12.** Kit according to claim 11, further comprising at least one of:

- a box,
- a cool pack,
- at least one form including instructions and/or information about the kit and the method for the subject.

**13.** Kit according to claim 11 or 12, wherein the RNA protect reagent is selected from the group comprising EDTA disodium, dihydrate; sodium citrate trisodium salt, dihydrate; ammonium sulfate, powdered; sterile water.

**14.** Kit according to any of claims 11 or 13, wherein said sampling tubes are configured to receive a sample of saliva of 1 mL in addition to 1 mL of the RNA protect reagent, wherein the sampling tubes preferably are at least 2 mL tubes, preferably at least 3 mL tubes, more preferably at least 4 mL tubes, still preferably at least 5 mL tubes.

**15.** Use of the kit of any of claims 11 to 14 for collecting samples of saliva for providing the collected samples of saliva for a method of any of claims 1 to 10.

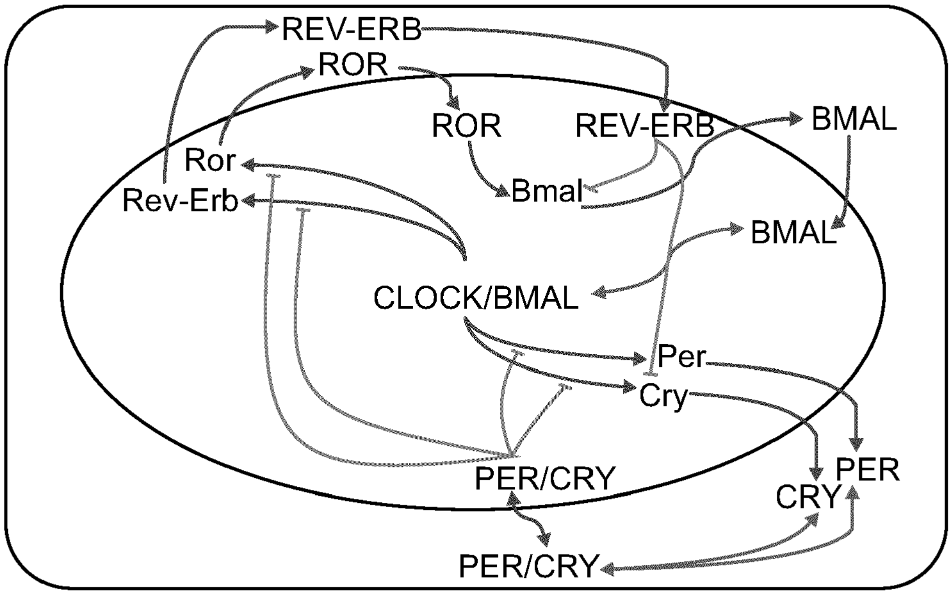
REV-ERB
ROR
ROR
REV-ERB
BMAL
Ror
Rev-Erb
Bmal
BMAL
CLOCK/BMAL
Per
Cry
PER
CRY
PER/CRY
PER/CRY

Fig. 1

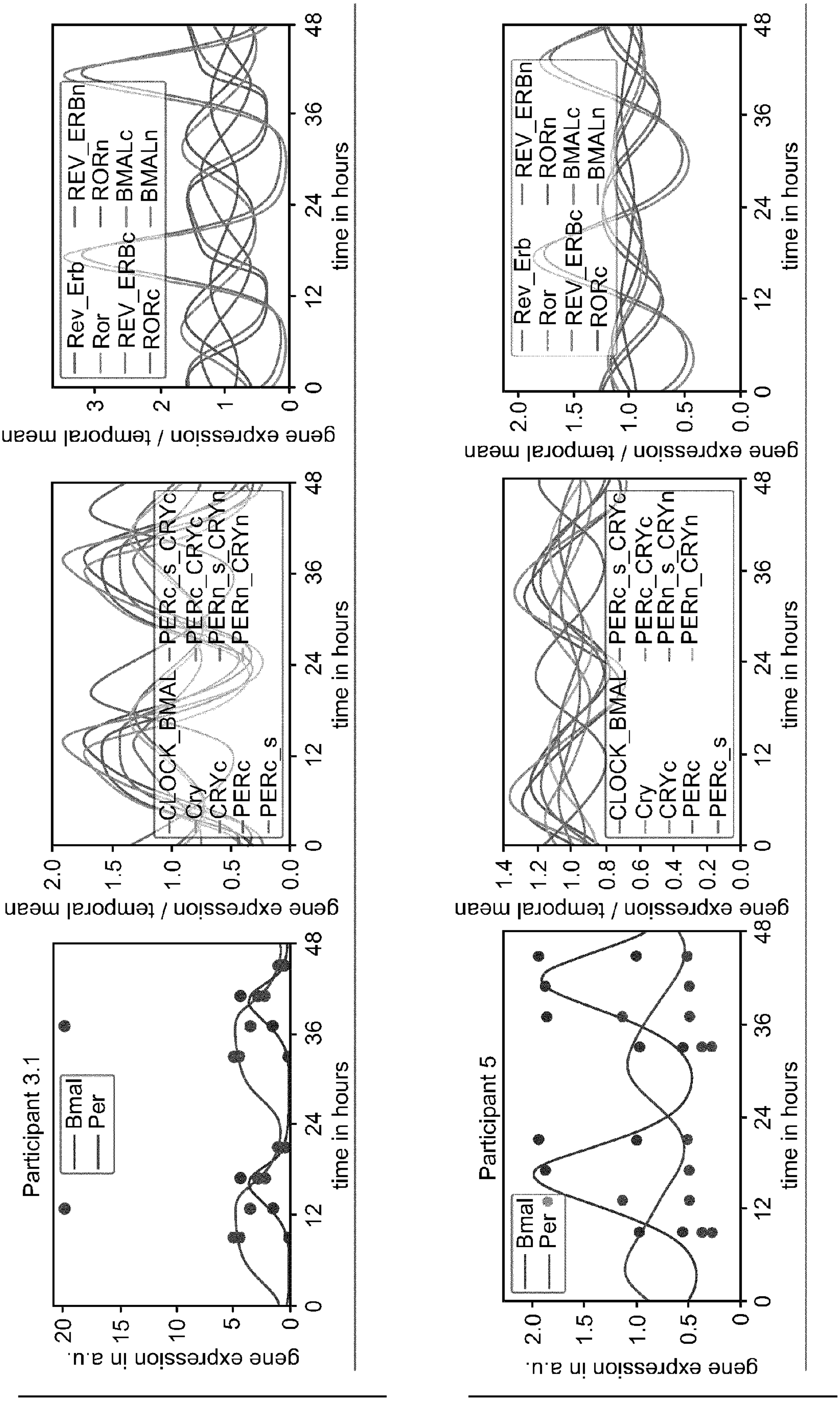
Participant 3.1
Bmal
Per
gene expression in a.u.
time in hours
CLOCK_BMAL
Cry
CRYc
PERc
PERc_s
PERc_s_CRYc
PERc_CRYc
PERn_s_CRYn
PERn_CRYn
gene expression / temporal mean
Rev_Erb
Ror
REV_ERBc
RORc
REV_ERBn
RORn
BMALc
BMALn
Participant 5

Fig. 2

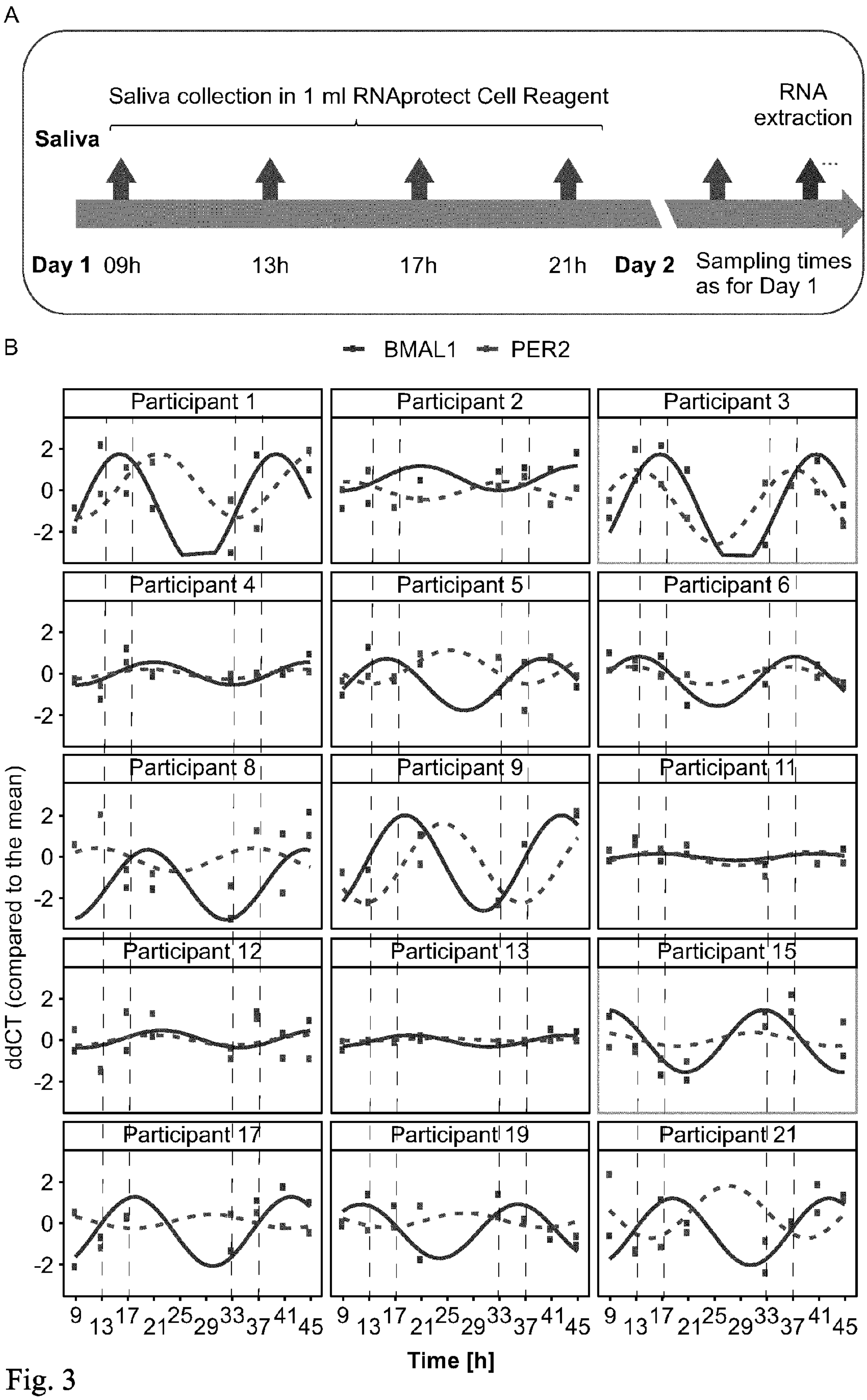
A
Saliva collection in 1 ml RNAprotect Cell Reagent
RNA extraction
Saliva
Day 1 09h
13h
17h
21h
Day 2
Sampling times as for Day 1
B
BMAL1
PER2
Participant 1
Participant 2
Participant 3
Participant 4
Participant 5
Participant 6
Participant 8
Participant 9
Participant 11
Participant 12
Participant 13
Participant 15
Participant 17
Participant 19
Participant 21
ddCT (compared to the mean)
Time [h]

Fig. 3

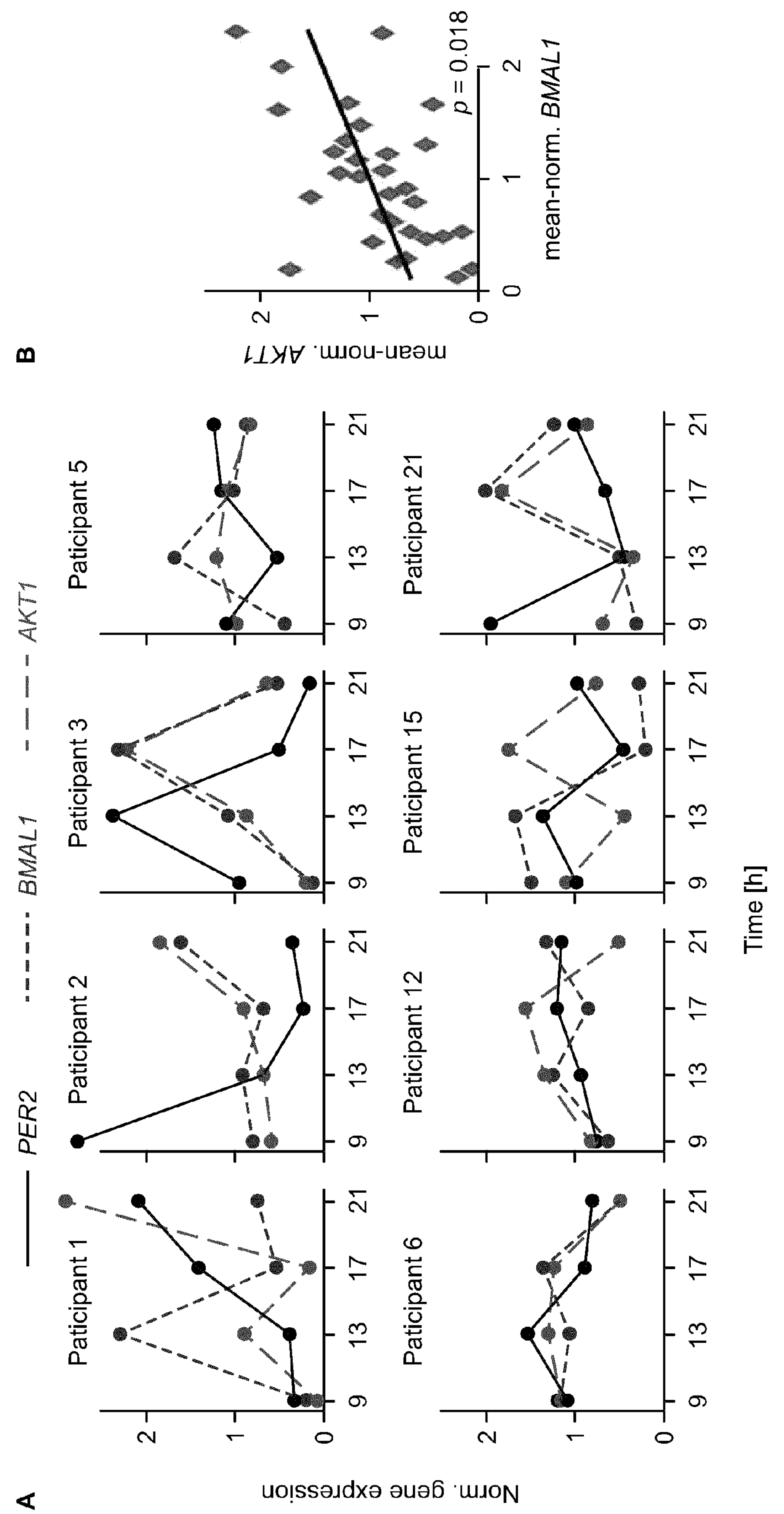
A
B
PER2
BMAL1
AKT1
Paticipant 1
Paticipant 2
Paticipant 3
Paticipant 5
Paticipant 6
Paticipant 12
Paticipant 15
Paticipant 21
Norm. gene expression
Time [h]
9
13
17
21
0
1
2
mean-norm. AKT1
mean-norm. BMAL1
p = 0.018

Fig. 4

C

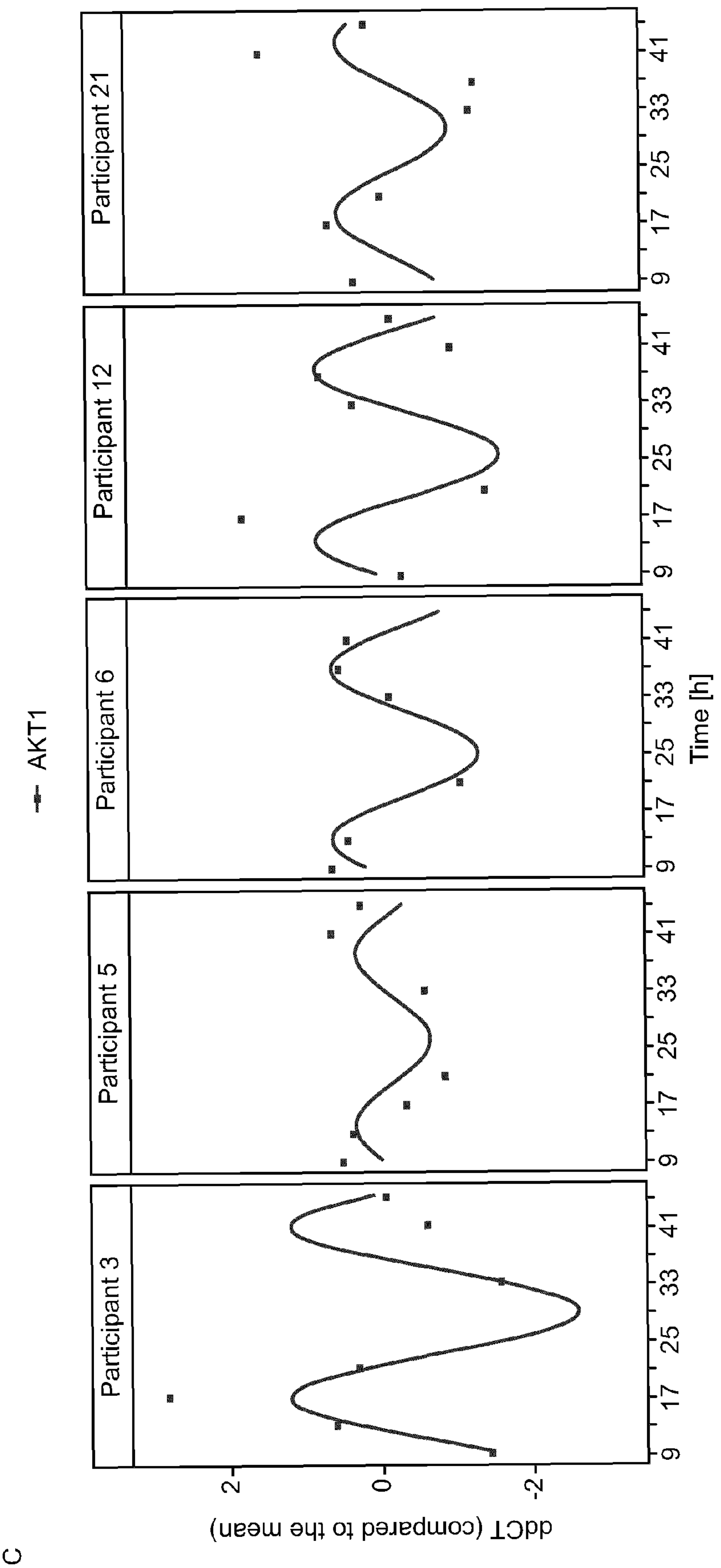
AKT1
Participant 3
Participant 5
Participant 6
Participant 12
Participant 21
ddCT (compared to the mean)
2
0
-2
9
17
25
33
41
Time [h]

Fig. 4

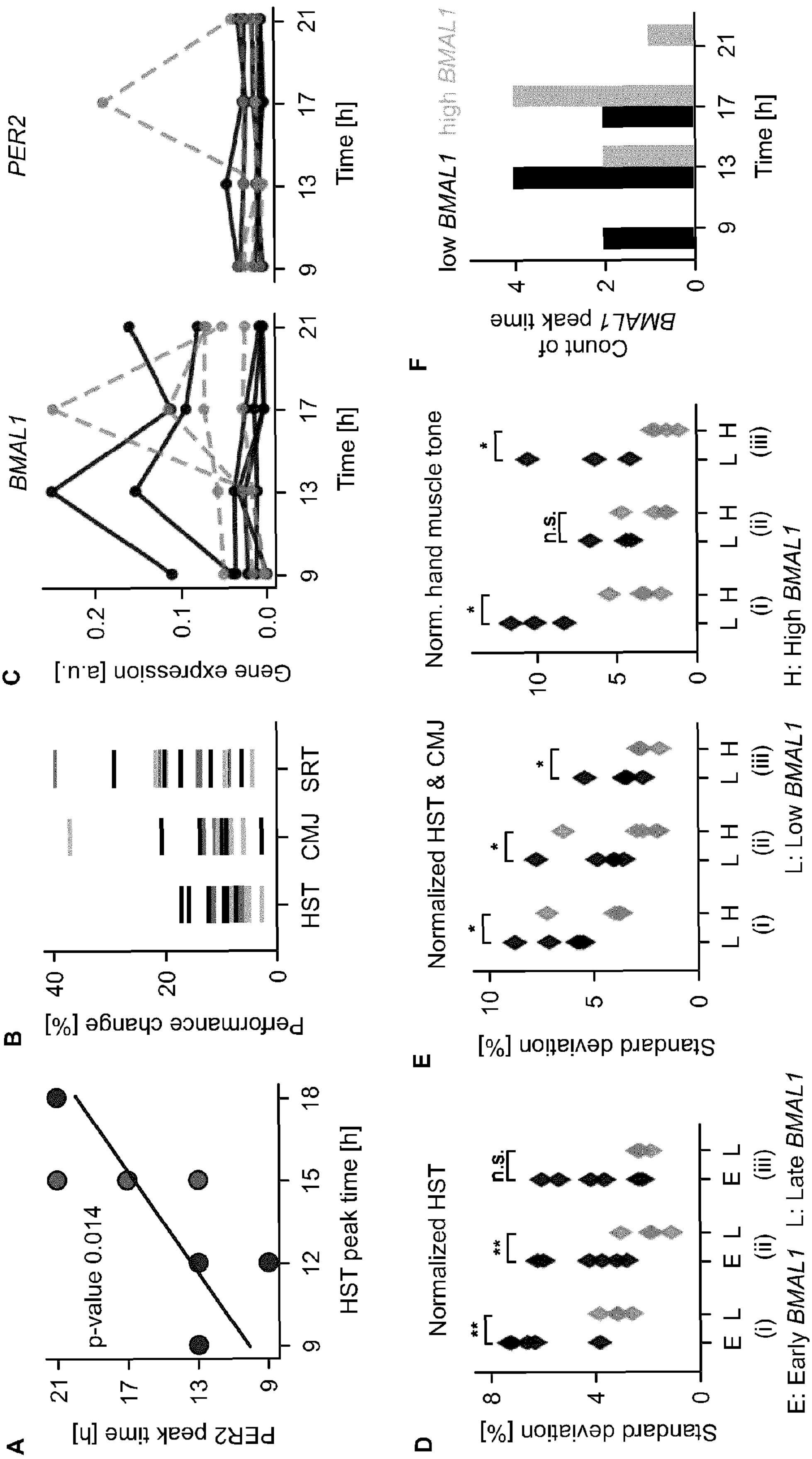
A
PER2 peak time [h]
21
17
13
9
p-value 0.014
9
12
15
18
HST peak time [h]
B
Performance change [%]
40
20
0
HST CMJ SRT
C
Gene expression [a.u.]
0.2
0.1
0.0
BMAL1
PER2
9
13
17
21
Time [h]
D
Normalized HST
Standard deviation [%]
8
4
0
**
**
n.s.
E L E L E L
(i) (ii) (iii)
E: Early BMAL1 L: Late BMAL1
E
Normalized HST & CMJ
Standard deviation [%]
10
5
0
*
*
*
L H L H L H
(i) (ii) (iii)
L: Low BMAL1
Norm. hand muscle tone
10
5
0
*
n.s.
*
L H L H L H
(i) (ii) (iii)
H: High BMAL1
F
low BMAL1 high BMAL1
Count of BMAL1 peak time
4
2
0
9
13
17
21
Time [h]

Fig. 5

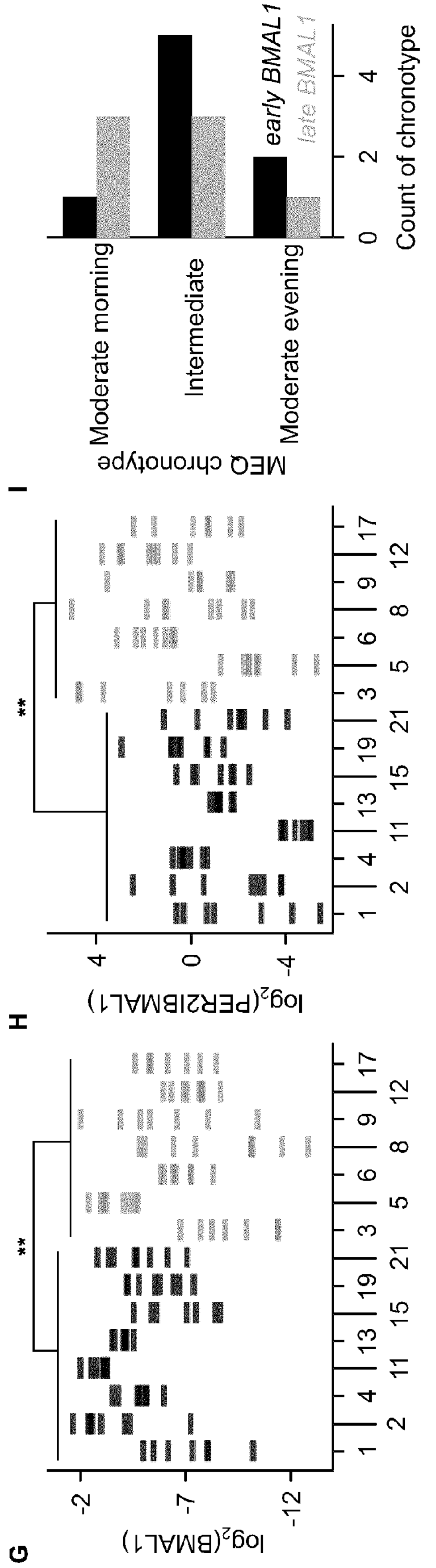
G
H
I
log$_2$(BMAL1)
-2
-7
-12
1
2
4
11
13
15
19
21
3
5
6
8
9
12
17
Participant Nr.
**
log$_2$(PER2/BMAL1)
4
0
-4
MEQ chronotype
Moderate morning
Intermediate
Moderate evening
0
2
4
Count of chronotype
early BMAL1
late BMAL1

Fig. 5

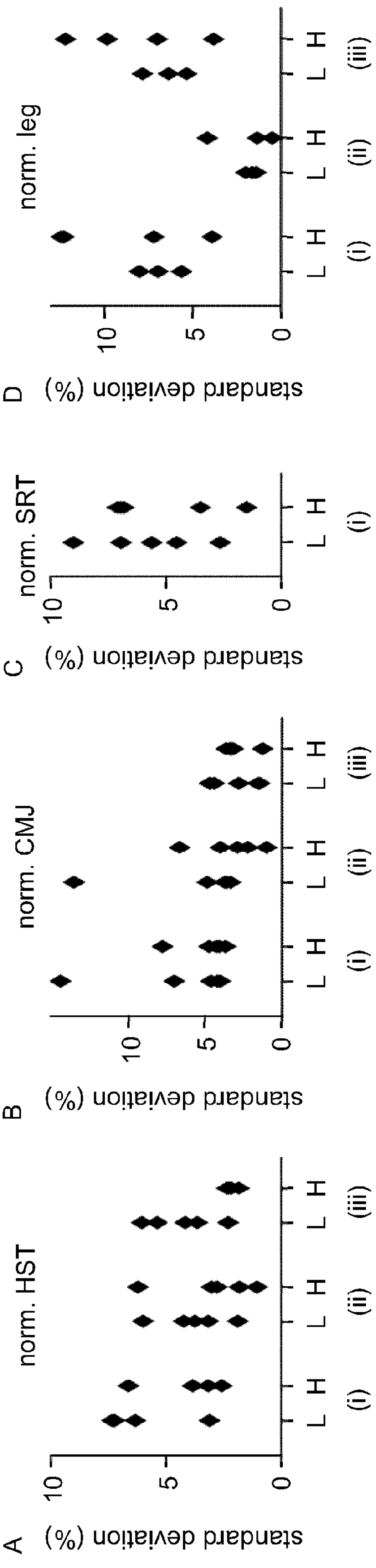
A
standard deviation (%)
norm. HST
0
5
10
L H
(i)
L H
(ii)
L H
(iii)
B
standard deviation (%)
norm. CMJ
0
5
10
L H
(i)
L H
(ii)
L H
(iii)
C
standard deviation (%)
norm. SRT
0
5
10
L H
(i)
D
standard deviation (%)
norm. leg
0
5
10
L H
(i)
L H
(ii)
L H
(iii)

Fig. 6

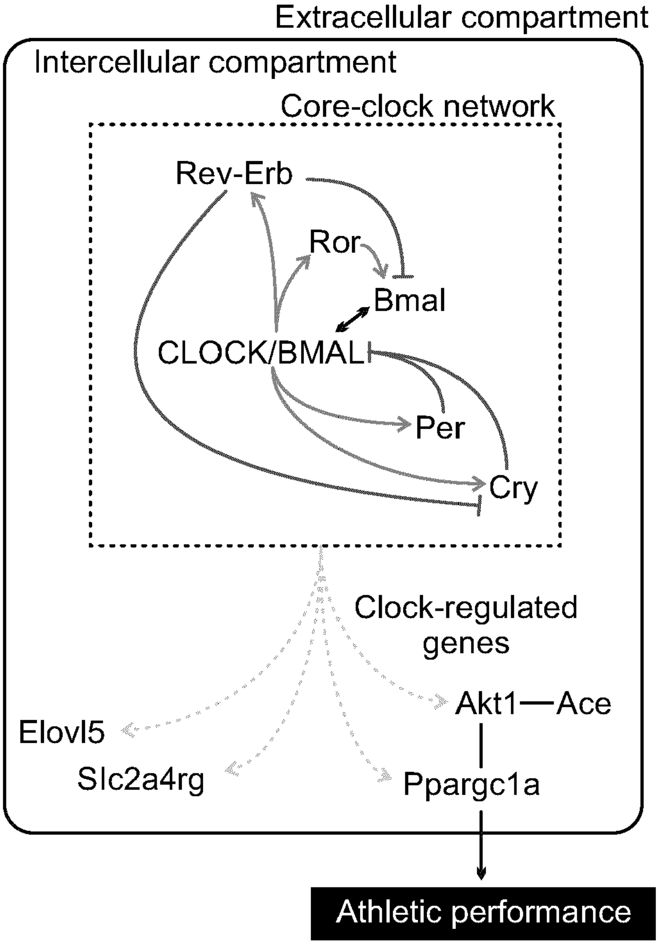
Extracellular compartment
Intercellular compartment
Core-clock network
Rev-Erb
Ror
Bmal
CLOCK/BMAL
Per
Cry
Clock-regulated genes
Akt1—Ace
Elovl5
Slc2a4rg
Ppargc1a
Athletic performance

Fig. 7

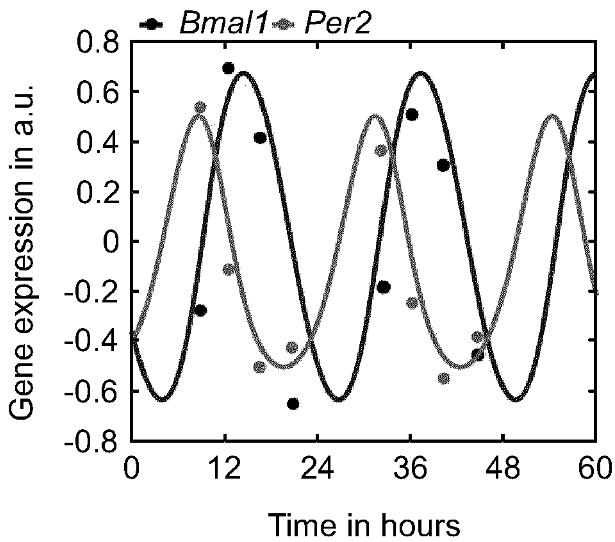
Bmal1
Per2
0.8
0.6
0.4
0.2
0
-0.2
-0.4
-0.6
-0.8
Gene expression in a.u.
0
12
24
36
48
60
Time in hours

Fig. 8a

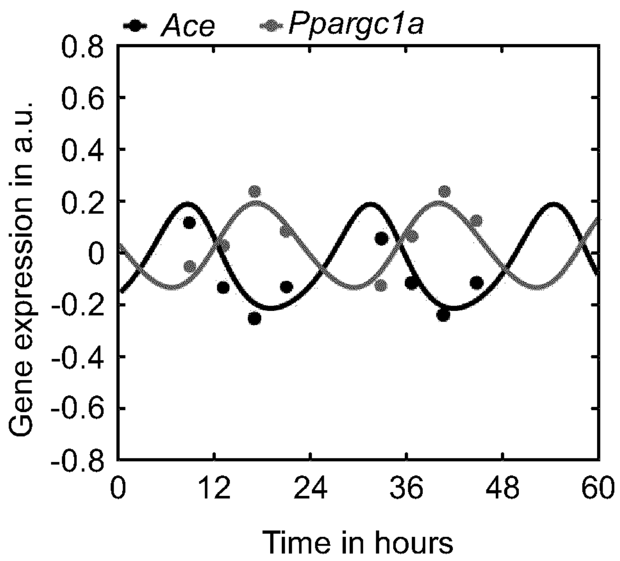


Fig. 8b

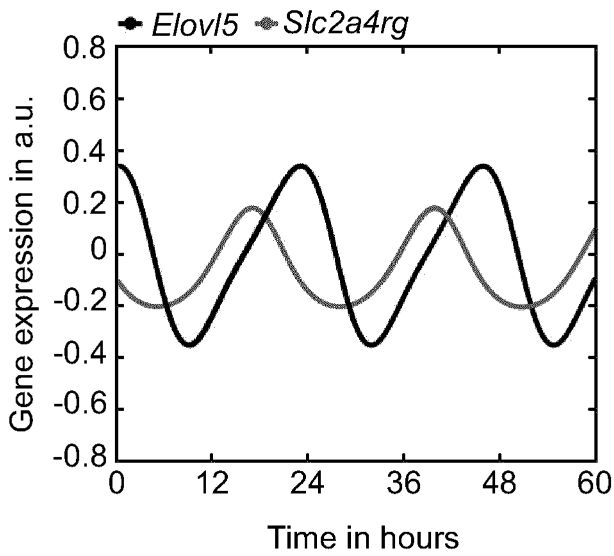


Fig. 8c

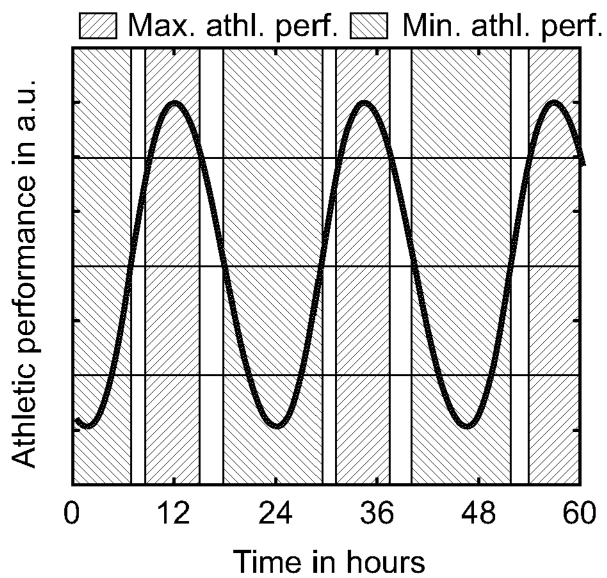


Fig. 9

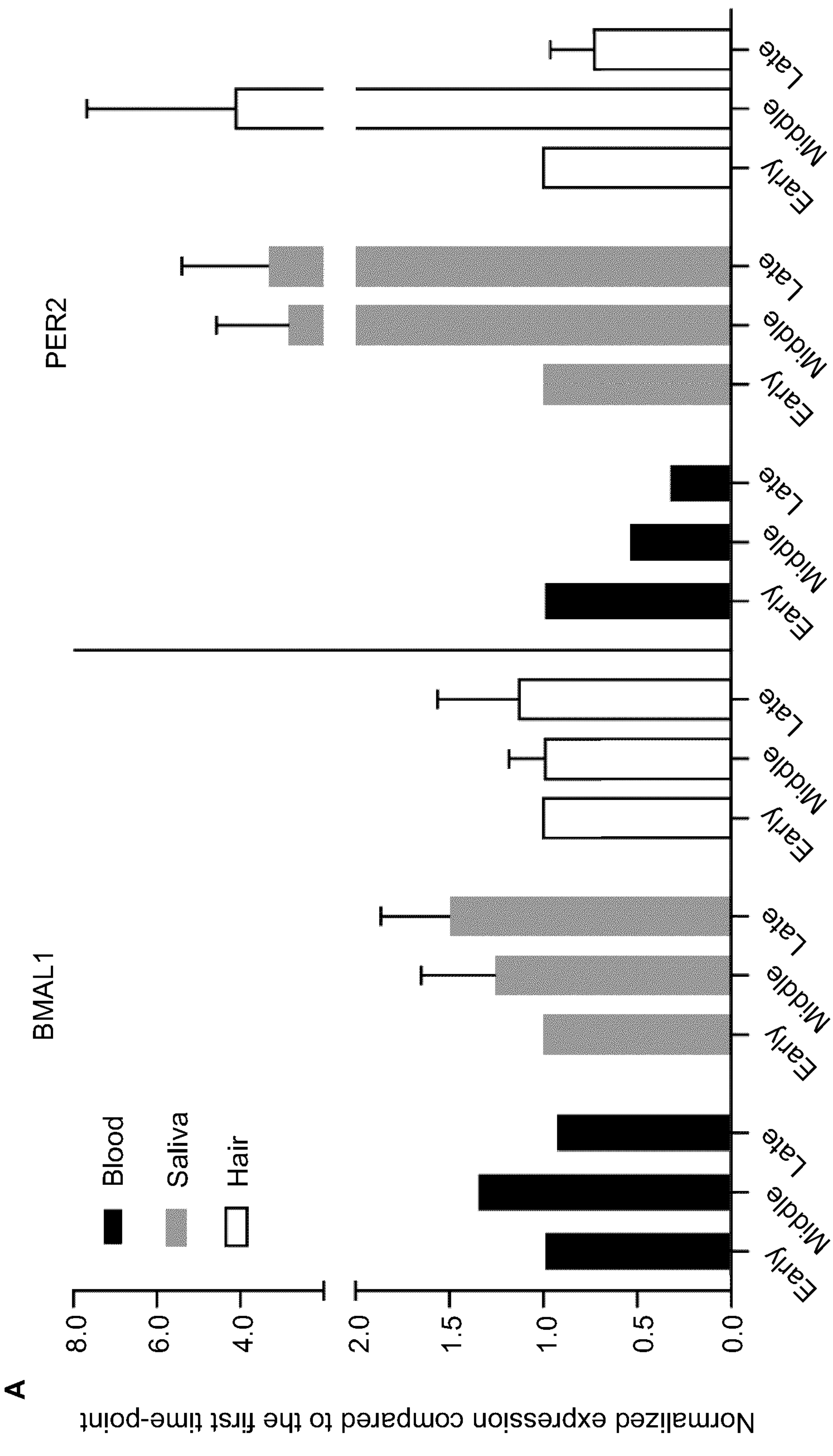
A
Normalized expression compared to the first time-point
0.0
0.5
1.0
1.5
2.0
4.0
6.0
8.0
Blood
Saliva
Hair
BMAL1
PER2
Early
Middle
Late

Fig. 10

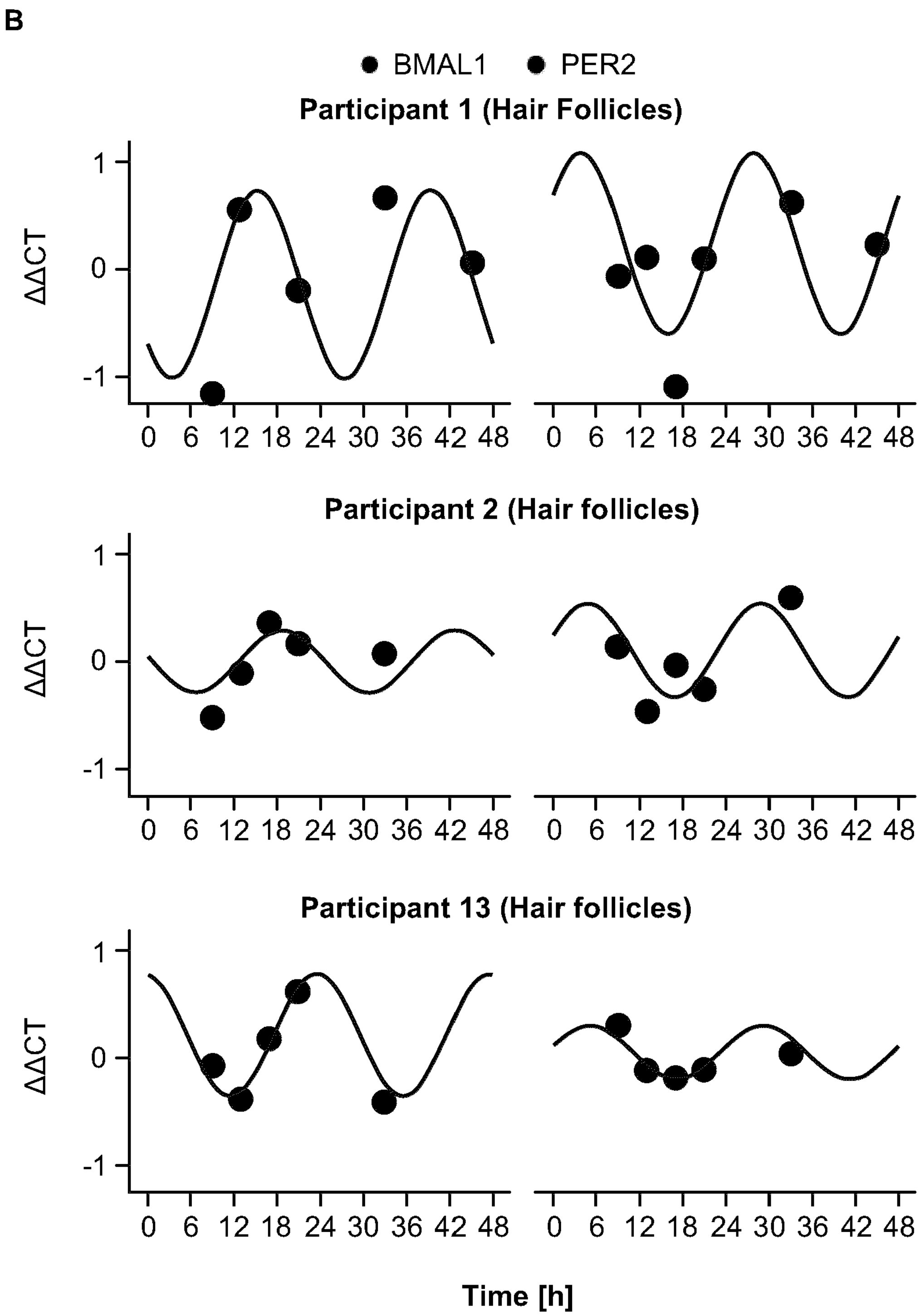
B
BMAL1
PER2
Participant 1 (Hair Follicles)
Participant 2 (Hair follicles)
Participant 13 (Hair follicles)
ΔΔCT
1
0
-1
0 6 12 18 24 30 36 42 48
Time [h]

Fig. 10

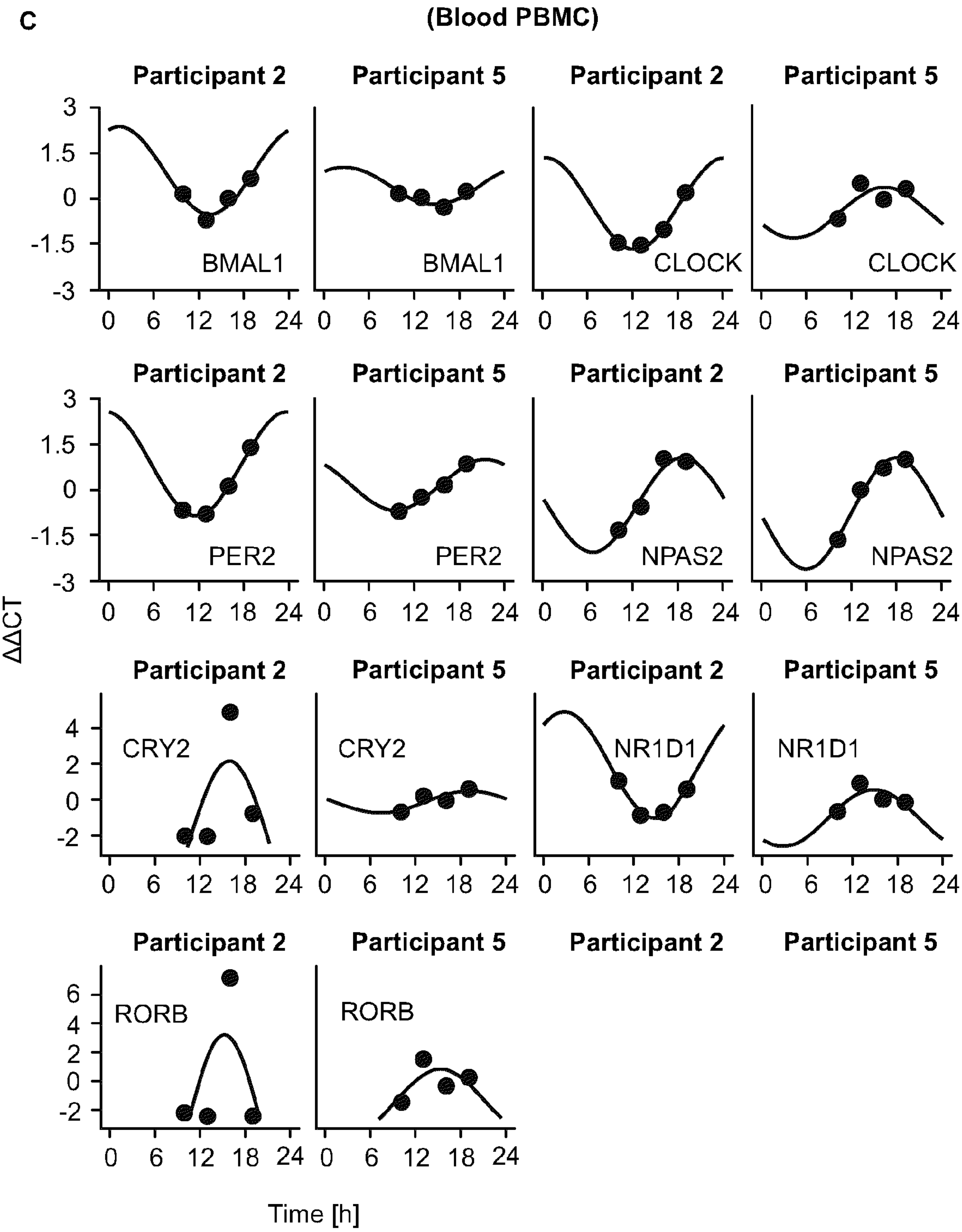
C
(Blood PBMC)
Participant 2
Participant 5
Participant 2
Participant 5
BMAL1
BMAL1
CLOCK
CLOCK
PER2
PER2
NPAS2
NPAS2
CRY2
CRY2
NR1D1
NR1D1
RORB
RORB
ΔΔCT
Time [h]

Fig. 10

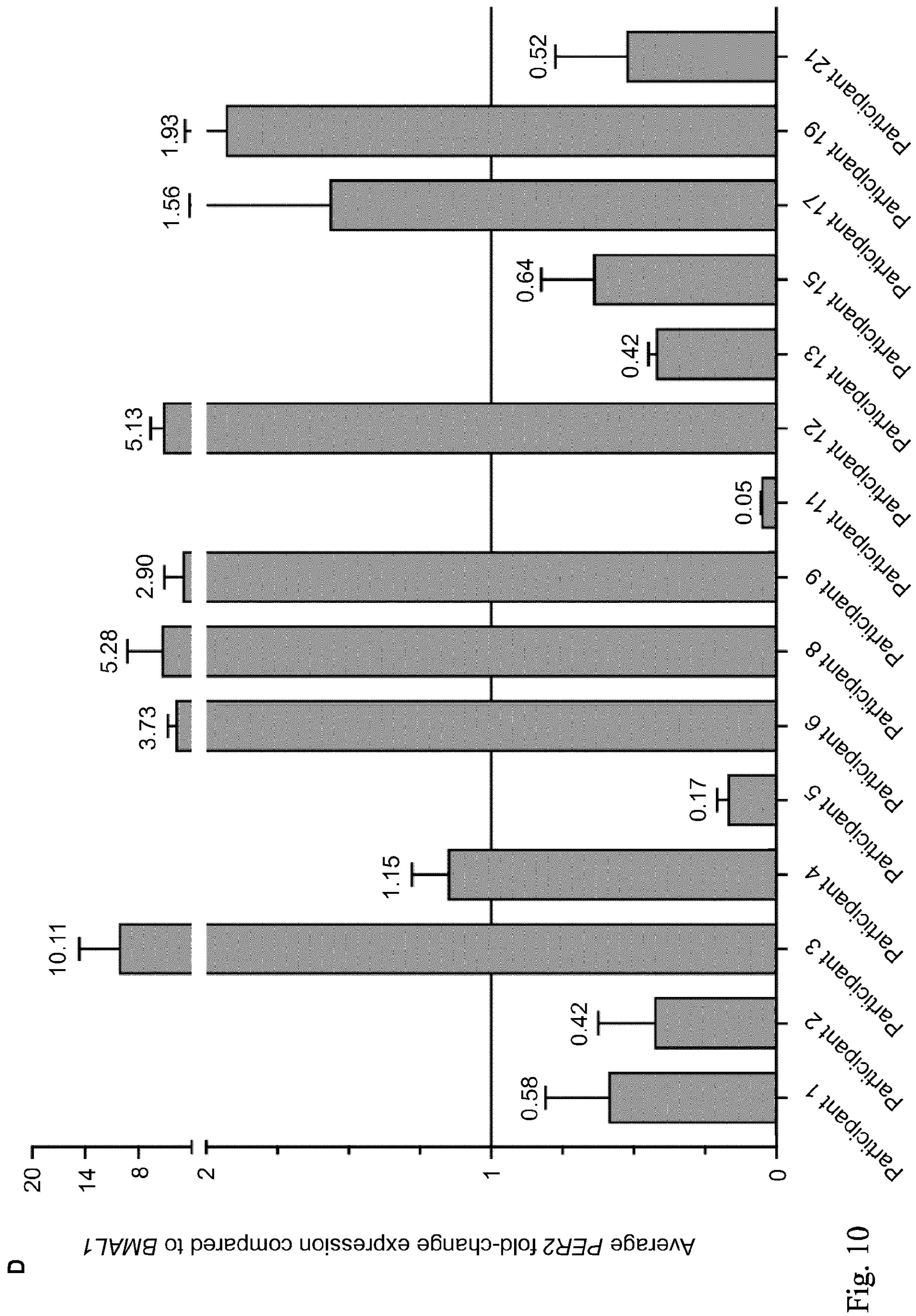
D
Average *PER2* fold-change expression compared to *BMAL1*
0
1
2
8
14
20
Participant 1
Participant 2
Participant 3
Participant 4
Participant 5
Participant 6
Participant 8
Participant 9
Participant 11
Participant 12
Participant 13
Participant 15
Participant 17
Participant 19
Participant 21
0.58
0.42
10.11
1.15
0.17
3.73
5.28
2.90
0.05
5.13
0.42
0.64
1.56
1.93
0.52

Fig. 10

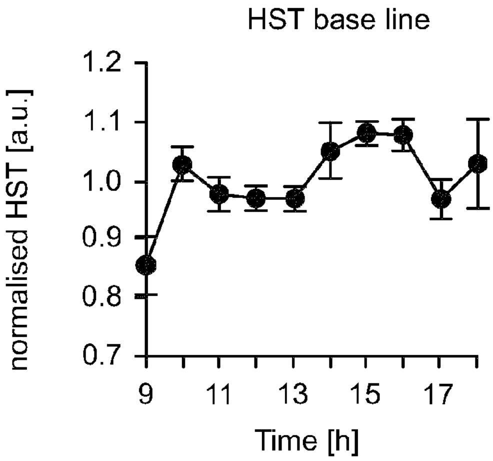
HST base line
normalised HST [a.u.]
1.2
1.1
1.0
0.9
0.8
0.7
9
11
13
15
17
Time [h]

Fig. 11

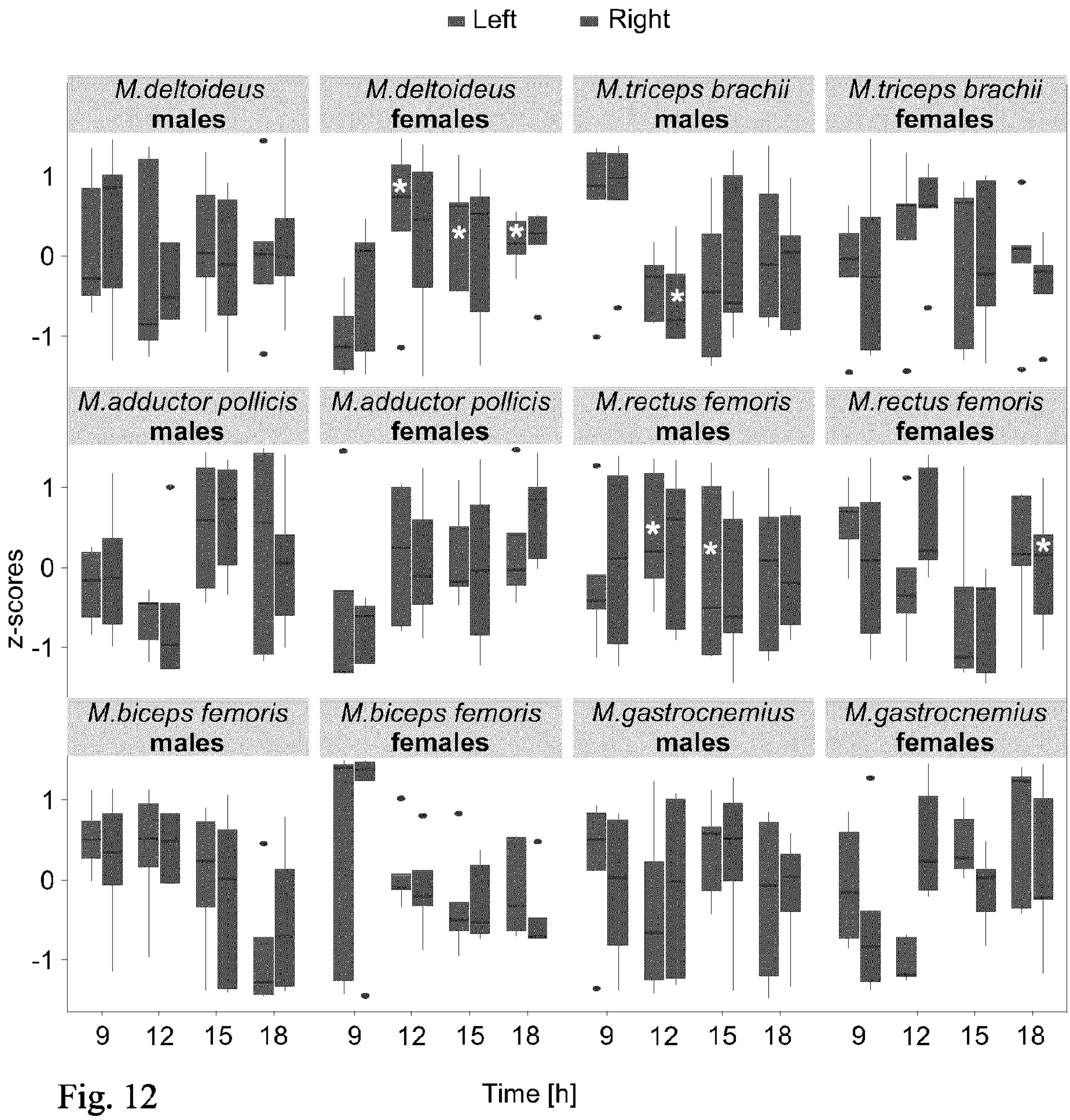
Left
Right
M.deltoideus males
M.deltoideus females
M.triceps brachii males
M.triceps brachii females
M.adductor pollicis males
M.adductor pollicis females
M.rectus femoris males
M.rectus femoris females
M.biceps femoris males
M.biceps femoris females
M.gastrocnemius males
M.gastrocnemius females
z-scores
1
0
-1
9
12
15
18
Time [h]

Fig. 12

Europäisches Patentamt
European Patent Office
Office européen des brevets

# EUROPEAN SEARCH REPORT

Application Number
EP 20 19 3227

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Oragene-RNA ¦ RE-100", DNA Genotek , 31 December 2016 (2016-12-31), XP055768952, Retrieved from the Internet: URL:https://www.dnagenotek.com/US/pdf/PD-PR-020-ML.pdf [retrieved on 2021-01-26] * p. 1, first two columns * | 11-15 | INV. C12Q1/6876 G16B40/00 |
| X | R M Iwasiow ET AL: "Stability and yield of RNA collected from saliva using Oragene-RNA for expression analysis", DNA Genotek , 30 June 2013 (2013-06-30), XP055768955, Retrieved from the Internet: URL:https://www.dnagenotek.com/ROW/pdf/PD-WP-008.pdf [retrieved on 2021-01-26] * p. 1, left-hand col., 2nd-4th para; p. 1, right-hand col., 2nd para. * | 11-15 | |
| X | WO 2011/114732 A1 (SHISEIDO CO LTD [JP]; GOZU YOKO [JP]; HAZE SHINICHIRO [JP]) 22 September 2011 (2011-09-22) | 1-3,11, 15 | |
| Y | * para. 24; examples 1-2; fig. 1-2; * | 7,9,10 | |
| X | KR 2017 0091047 A (UNIV KOREA RES & BUS FOUND [KR]) 8 August 2017 (2017-08-08) | 1-6,8 | |
| Y | * para. 8-15, 20, 41; fig. 1, 4, 5; claims 1-6 * | 7,9,10 | |
| | -/-- | | |

TECHNICAL FIELDS SEARCHED (IPC)
C12Q
G06F
G16B

1

The present search report has been drawn up for all claims

| Place of search | Date of completion of the search | Examiner |
|---|---|---|
| Munich | 28 January 2021 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 19 3227

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GERMAINE CORNELISSEN: "Cosinor-based rhythmometry", THEORETICAL BIOLOGY AND MEDICAL MODELLING, BIOMED CENTRAL LTD., LONDON, GB, vol. 11, no. 1, 11 April 2014 (2014-04-11), page 16, XP021182107, ISSN: 1742-4682, DOI: 10.1186/1742-4682-11-16 * the whole document * ----- | 1-10 | |
| Y | BILJANA VITOSEVIC: "The circadian clock and human athletic performance", THE UNIVERSITY THOUGHT - PUBLICATION IN NATURAL SCIENCES, vol. 7, no. 1, 31 December 2017 (2017-12-31), pages 1-7, XP055768416, ISSN: 1450-7226, DOI: 10.5937/univtho7-13650 * table 1; p. 4, left-hand col., 3rd para. - right-hand col., 1st para. * ----- | 7,9,10 | |
| | | | TECHNICAL FIELDS SEARCHED (IPC) |
| Y | EZAGOURI S. ET AL: "Physiological and Molecular Dissection of Daily Variance in Exercise Capacity", CELL METABOLISM, vol. 30, no. 1, 2 July 2019 (2019-07-02), pages 78-91, XP85726421, * abstract; p. 88, left-hand col., 3rd para. * ----- | 7,9,10 | |
| Y | FACER-CHILDS E. ET AL: "The Impact of Circadian Phenotype and Time since Awakening on Diurnal Performance in Athletes", CURRENT BIOLOGY, vol. 25, 16 February 2015 (2015-02-16), pages 518-522, XP55769500, * p. 519, left-hand col., last para. * ----- | 7,9,10 | |

1

The present search report has been drawn up for all claims

| Place of search | Date of completion of the search | Examiner |
|---|---|---|
| Munich | 28 January 2021 | Ripaud, Leslie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 19 3227

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2021

| Patent document cited in search report | Publication date | Patent family member(s) | Publication date |
|---|---|---|---|
| WO 2011114732 A1 | 22-09-2011 | JP 2011193779 A | 06-10-2011 |
| | | WO 2011114732 A1 | 22-09-2011 |
| KR 20170091047 A | 08-08-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KIM, JAE KYOUNG ; DANIEL B FORGER.** A Mechanism for Robust Circadian Timekeeping via Stoichiometric Balance. *Molecular Systems Biology,* December 2012, vol. 8, 630, https://doi.org/10.1038/msb.2012.62.
- **GEISS G et al.** Direct multiplexed measurement of gene expression with color-coded probe pairs. *Nature Biotechnology,* 08 February 2008, vol. 26, 317-25